# EUROPEAN PATENT APPLICATION

(11) **EP 2 949 344 A2**
(43) Date of publication of application: **02.12.2015**
(21) Application number: 15173103.1
(22) Date of filing: 16.03.2012
(51) Int. Cl.: A61K 47/48

(54) **USE OF INTRACELLULAR ENZYMES FOR THE RELEASE OF COVALENTLY LINKED BIOACTIVES**

(30) Priority: 18.03.2011 US 201161454314 P
(62) Divisional of application: 12761015.2
(71) Applicant: Catabasis Pharmaceuticals, Inc., Cambridge, Massachusetts 02142 (US)
(72) Inventor: JIROUSEK, Michael R., Cambridge, MA 02139 (US); MILNE, Jill C., Brookline, MA Massachusetts 02446 (US); CARNEY, David, Derry, NH New Hampshire 03038 (US); BEMIS, Jean E., Arlington, MA Massachusetts 02474 (US); VU, Chi B., Arlington, MA Massachusetts 02474 (US)
(74) Representative: Grund, Martin

(57) **Abstract**

The present invention relates to targeting an intracellular enzyme for release of covalently linked bioactives which results in a synergistic effect between the bioactives. The present invention relates to the use of bioactives that are directly connected covalently or through a covalent molecular linker which have increased therapeutic activity when released as the free bioactives by intracellular enzymes as compared to when the bioactives are administered individually (i.e. not covalendy linked). Further, methods are described of administering to patients in need thereof, bioactives as linked bioactives having increased therapeutic activity. Accordingly, this invention also relates to methods of treating patients for certain diseases.

## Description

### PRIORITY

This application claims the benefit of priority from U.S. Provisional Patent Application No. 61/454,314, filed March 18, 2011, the contents of which are herein incorporated by reference in its entirety.

### FIELD

The present invention relates to targeting an intracellular enzyme for release of covalently linked bioactives which results in a synergistic effect between the bioactives. The present invention also relates to the use of bioactives that are directly connected covalently or through a covalent molecular linker which have increased therapeutic activity when released as the free bioactives by intracellular enzymes as compared to when the bioactives are administered individually *(i.e.* not covalently linked). Further, methods are described of administering to patients in need thereof, bioactives as linked bioactives having increased therapeutic activity. Accordingly, this invention also relates to methods of treating patients for certain diseases.

### BACKGROUND

Combination therapy (or polytherapy) is the use of more than one drug or more than one therapy to treat a single disease state, while polypharmacy is the use of more than one drug to treat multiple, separate disease states (S Baron (ed.) Medical Microbiology. 4th edition., University of Texas Medical Branch at Galveston 1996.). For example, in certain antiviral approaches against HIV/AIDS, a combination therapy known as highly active antiretroviral therapy (HAART) is used. This treatment regime typically consists of combinations (or "cocktails") of at least three drugs belonging to at least two classes of antiretroviral agents. For example, a common approach is to combine two nucleoside analogue reverse transcriptase inhibitors (NARTIs) plus either a protease inhibitor or a non-nucleoside reverse transcriptase inhibitor (NNRTI). In situations in which multiple drugs are used, a highly desirable consequence is synergy, *i.e.* the super-additive effect of a combination of drugs that provides results that exceed what could be expected from the sum of the individual drugs administered in isolation (Tallarida, Drug Synergism and Dose-Effect Data Analysis, Chapman and Hall/CRC 2000). At the very least, that the administered drugs have an additive effect, *i.e.* the same effects as they would in isolation, is desired.

However, it is not uncommon for such approaches to fail. That is, some combination therapies are not productive, and therefore show effects that are sub-additive *(i.e.* lower than the additive effect) or even lower than what is observed with either drug in isolation. Furthermore, some drugs, despite having clear mechanistic links to particular disease states, have minimal or no effect when administered alone. A variety of reasons can be hypothesized for the failure of a drug or drugs in complex systems such as the physiological environment of patients. Etiology aside, there is a clear need for alternative drug delivery strategies that will maximize effects, potentially through synergy. Particularly, there is a need for methods of disease treatments that employ intracellular enzymes for the release of linked bioactives that produce an efficacy above what is observed when the bioactives are administered together but are not covalently linked.

### SUMMARY

The present invention relates to methods of targeting an intracellular enzyme for the release of bioactives which are covalently linked which produces a synergistic effect between the bioactives. The present invention is based in part on the discovery that certain bioactives when administered together are efficacious if they are covalently linked by a linker group ("linked bioactives") and subsequently released after administration to the free bioactives in targeted disease tissue by the action of an intracellular enzyme which acts upon the linker group. These linked bioactives have been found to be plasma stable and the bioactives are not freed in biologically effective amounts from the linker group until they reach the targeted disease tissue. More specifically, this efficacy is observed when the linker group of the linked bioactive is enzymatically cleaved through the activity of an intracellular enzyme or enzymes. This efficacy may be synergistic, *i.e.* the response is greater than the combination of the bioactives. In certain instances, when the two bioactives are covalently linked as a linked bioactive, the associated side effects of either bioactive is less than the side effects for the bioactive when administered alone. This is because the individual bioactives can only be released inside targeted cells through the activity of an intracellular enzyme or enzymes expressed inside the targeted cell and which acts on the linker group covalently binding the bioactives together.

Accordingly, in one aspect a method for increasing intracellular bioactivity or bioactivities of at least two bioactives is described, the method comprising: (a) selecting a first bioactive; (b) selecting a second bioactive that can be the same or different as compared to the bioactive of (a); (c) selecting a linker group that covalently binds the bioactive of (a) and the bioactive of (b) wherein the linker group comprises at least one linkage that acts as a functional substrate of an intracellular enzyme; and (d) linking the bioactives of (a) and (b) with the linker group of (c). In some embodiments, the intracellular enzyme is expressed to a greater level in a diseased cell type as compared to the non-diseased state of the same cell type.

In another aspect the use of an intracellular enzyme(s) to cleave a linkage comprised in a linker group that connects two or more bioactives in the treatment of a disease is described. In some embodiments, the linker group may be enzymatically cleaved or hydrolyzed by an intracellular enzyme and thus lead to the release of the bioactive(s) and a synergistic effect. In some embodiments, multiple enzymes are required to achieve release of the bioactives. Nonlimiting examples of enzymes useful in the present invention are amidases, proteases, thiol reductases, lipases, and amide hydrolases. In some embodiments, multiple cleavages by one or more enzymes to ultimately cleave or hydrolyze the linker between the bioactives or to free the bioactives from the linker occurs.

Another aspect of the present invention is the use of an intracellular enzyme(s) to cleave a linkage comprised in a linker group that connects two or more bioactives in the treatment of a disease wherein the enzyme is any enzyme capable of processing a fatty acid amide linked to a bioactive.

Another aspect of the present invention is the use of an intracellular enzyme(s) to cleave or hydrolyze a linkage comprised in a linker group that connects two or more bioactives in the treatment of a disease wherein the enzyme is a fatty acid amide hydrolase.

Another aspect of the present invention is the use of an intracellular enzyme(s) to cleave or hydrolyze a linkage comprised in a linker group that connects two or more bioactives in the treatment of a disease wherein the bioactive(s) may be distinct or identical chemical entities. Nonlimiting examples of linked bioactives are salicylate linked to omega 3 fatty acid, niacin linked to omega 3 fatty acid, fumarate linked to omega 3 fatty acid, omega 3 fatty acid linked to omega 3 fatty acid, and a cyclooxygenase inhibitor linked to an omega 3 fatty acid, among others with all these linked bioactives being stable in plasma.

Another aspect of the present invention is the use of an intracellular enzyme(s) to cleave or hydrolyze a linkage comprised in a linker group that connects two or more bioactives in the treatment of a disease wherein the intracellular enzyme(s) may be selected based on the state of the cell. For instance, one aspect of the present invention encompasses the selection of a linker group to be cleaved by an intracellular enzyme that has an expression pattern that correlates with the disease that is being targeted for therapy. In some embodiments, the linker group is selected based on tissue specific expression of an intracellular enzyme wherein the linker group comprises a linkage which is a substrate for the intracellular enzyme. In some embodiments, the functionality of certain enzymes in diseases or tissue environments is assayed for, if present, a linker group which comprises a substrate for the enzyme is selected.

The present application is based in part on the observation that certain therapeutics are efficacious when delivered as linked bioactives to specific environments expressing certain enzymes. Therefore, two or more bioactives for the treatment of a disease may have a lesser or no effect on the disease when they are administered separately or even when co-administered but not linked together as linked bioactives. In some embodiments, these effects are produced by an intracellular enzyme that hydrolyzes the linker group to make it efficacious. In different aspects of the invention, different diseases are treated. In some aspects, the diseases treated are inflammation diseases. In some aspects, the diseases are those of lipid homeostasis (including, but not limited to, dyslipidemia and hypertriglyceridemia), cardiovasular diseases, metabolic diseases (including, but not limited to, type 2 diabetes), kidney diseases, cancers, neurodegenerative disease, autoimmune diseases, septic shock, viral infection, and improper immune development disorders, among others.

Also, the pharmaceutically relevant activity of these bioactives may not be observed unless the linker group or linkage between the bioactives is cleaved by an intracellular enzyme. Therefore, another aspect of the present invention is the use of intracellular enzyme inhibitors to determine the dependency of linked bioactives on specific intracellular enzymes for efficacy in treating a condition. In one aspect, a linked bioactive is administered to a patient based on a disease and if a positive effect is observed, patient tissue is obtained and the patient tissue is tested for increased expression or functionality of certain intracellular enzymes suspected to be overexpressed or more functional. Afterwards, a linker group can be selected based on the enzyme and used between the bioactives to increase the therapeutic effect of the bioactives.

Yet another aspect of the invention is treatment of disease in which a bioactive combination and the linker groups connecting the respective bioactives are tailored to a patient based on the expression or functionality of specific enzymes that are associated with a disease or tissue type.

Another aspect of the present invention is a method of inhibiting, preventing, or treating diseases in individuals suffering from certain diseases comprising administration to the patient a linked bioactive that is selectively hydrolyzed by an intracellular enzyme. Further, the invention includes a method of administration of bioactives. Bioactives may be connected by a linker group comprising a linkage and be selected based on the linker group approximating the substrate of a particular enzyme such that the enzyme will cleave linker group.

Another aspect of the present invention is the selection of omega-3 fatty acid as one of the bioactive components. In some embodiments, omega 3 fatty acids are the bioactive component. In other embodiments, eicosapentaenoic acid (EPA), docosahexaenoic acid (DHA), or a combination of the two may be the bioactive component.

The details of the invention are set forth in the accompanying description below. Although methods and materials similar or equivalent to those described herein can be used in the practice or testing of the present invention, illustrative methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs. All patents and publications cited in this specification are incorporated herein by reference in their entireties.

### BRIEF DESCRIPTION OF THE DRAWINGS

**Figure 1** illustrates the *in vitro* hydrolysis of a linked bioactive.
**Figures 2-6** illustrate the synergistic effects of linked bioactives in *in vitro* assays.
**Figures 7A** and **7B** illustrate the release of cysteamine from a bis-fatty acid linked bioactive.
**Figure 8** illustrates the hydrolysis of compound I**I**-1 using recombinant MAGL.
**Figures 9A-D** illustrate the hydrolysis of compound **1-1** in COS-7 cells that have been overexpressed with either FAAH-1 or FAAH-2.

### DETAILED DESCRIPTION

The present invention relates to targeting an intracellular enzyme for release of linked bioactives which results in a synergistic effect between the bioactives. The present invention relates to the use of bioactives that are directly connected covalently or through a covalent molecular linker which have increased therapeutic activity when released as the free bioactives by intracellular enzymes as compared to when the bioactives are administered individually (*i.e*. not covalently linked). Further, methods are described of administering to patients in need thereof, bioactives as linked bioactives having increased therapeutic activity. Accordingly, this invention also relates to methods of treating patients for certain diseases.

Accordingly, in one aspect a method for increasing intracellular bioactivity or bioactivities of at least two bioactives is described, the method comprising: (a) selecting a first bioactive; (b) selecting a second bioactive that can be the same or different as compared to the bioactive of (a); (c) selecting a linkage comprised in a linker group between the bioactive of (a) and the bioactive of (b) wherein the linkage is a functional substrate of an intracellular enzyme; and (d) linking the bioactives of (a) and (b) with the linker group of (c). In some embodiments, the intracellular enzyme is expressed to a greater level in a diseased cell type as compared to the non-diseased state of the same cell type.

The present invention provides for the use of an appropriate intracellular enzyme(s) to cleave a linker group or linkage that connects two or more bioactive molecules in the treatment of a disease state.

The present invention also provides a method for treating individuals afflicted by certain diseases with a linked bioactive that is selectively hydrolyzed by an intracellular enzyme. Bioactives may be connected by a linker group comprising a linkage and be selected based on the observation that a particular enzyme will cleave such a linker group or linkage. One embodiment of the present invention encompasses the selection of a linker group to be cleaved by an intracellular enzyme that has an expression pattern that correlates with the disease state that is being targeted for therapy. Further, this selection of a linker group may be determined by tissue specific expression of an intracellular enzyme. Also, instead of assaying for expression levels, the invention encompasses assaying for the functionality of certain enzymes in disease states and tissue environments to select an appropriate linker group.

Further, the invention provides for a method of treatment using bioactives based on the expression or functional state of enzymes associated with disease states or tissue environments.

Further, the invention provides for a personalized medicine approach to disease treatment. One embodiment of this aspect of the invention is the use of intracellular enzyme inhibitors to determine the dependency of linked bioactives on specific intracellular enzymes for efficacy in treating a disease. Further, the present invention encompasses an approach to disease treatment in which a bioactive combination and the linker group connecting the respective bioactives are tailored to a patient based on the expression or functionality of specific enzymes that are associated with a disease state or tissue type.

### DEFINITIONS

The following definitions are used throughout the present application:

The articles "a" and "an" are used in this disclosure to refer to one or more than one *(i.e.,* to at least one) of the grammatical object of the article. By way of example, "an element" means one element or more than one element.

The term "and/or" is used in this disclosure to mean either "and" or "or" unless indicated otherwise.

The terms "bioactive" or "bioactives" are used in this disclosure to mean a chemical entity or entities, useful in the treatment of disease, in which at least one of the entities is selected from the following: an omega 3 fatty acid, a fatty acid that can be converted *in vivo* to an omega 3 fatty acid, and lipoic acid.

The term "linker group" is used in this disclosure to mean a molecular entity that covalently links a first bioactive and a second bioactive. Non-limiting representative examples of linker groups include the groups defined in the following formula 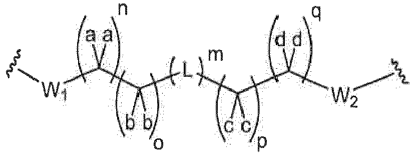 as described in e.g. Formula VI, herein.

The term "linkage" is used in this disclosure to mean a covalent bond that exists between the first bioactive and the "linker group" and any subsequent bioactive and the "linker group". A "linker group" will typically have at least two "linkages"; the first "linkage" is used to covalently join the first bioactive to the molecular entity that constitutes the "linker" and the second "linkage" is used to covalently join to the second bioactive. In some instances, the two bioactives can be covalently linked via a "linkage" without the use of the "linker group". A number of chemical bonds that are susceptible to enzymatic hydrolysis can be used in the "linkage". Non-limiting examples of chemical bonds that are susceptible to hydrolysis are: amides, esters, thio esters, phosphate esters, phosphoramidates, and disulfide.

The term "linked bioactive" is used in this disclosure to mean at least two bioactives that are covalently linked together through a linker group.

Unless otherwise specifically defined, the term "aryl" refers to cyclic, aromatic hydrocarbon groups that have one to two aromatic rings, including monocyclic or bicyclic groups such as phenyl, biphenyl or naphthyl. Where containing two aromatic rings (bicyclic, *etc*.)*,* the aromatic rings of the aryl group may be joined at a single point (*e.g.,* biphenyl), or fused (e.g., naphthyl). The aryl group may be optionally substituted by one or more substituents, *e.g*., one to five substituents, at any point of attachment. The substituents can themselves be optionally substituted.

"C₁-C₃ alkyl" refers to a straight or branched chain saturated hydrocarbon containing 1-3 carbon atoms. Examples of a C₁-C₃ alkyl group include, but are not limited to, methyl, ethyl, propyl and isopropyl.

"C₁-C₄ alkyl" refers to a straight or branched chain saturated hydrocarbon containing 1-4 carbon atoms. Examples of a C₁-C₄ alkyl group include, but are not limited to, methyl, ethyl, propyl, butyl, isopropyl, isobutyl, *sec*-butyl and *tert*-butyl.

"C₁-C₅ alkyl" refers to a straight or branched chain saturated hydrocarbon containing 1-5 carbon atoms. Examples of a C₁-C₅ alkyl group include, but are not limited to, methyl, ethyl, propyl, butyl, pentyl, isopropyl, isobutyl, sec-butyl and *tert*-butyl, isopentyl and neopentyl.

"C₁-C₆ alkyl" refers to a straight or branched chain saturated hydrocarbon containing 1-6 carbon atoms. Examples of a C₁-C₆ alkyl group include, but are not limited to, methyl, ethyl, propyl, butyl, pentyl, hexyl, isopropyl, isobutyl, *sec*-butyl, *tert*-butyl, isopentyl, and neopentyl.

The term "cycloalkyl" refers to a cyclic hydrocarbon containing 3-6 carbon atoms. Examples of a cycloalkyl group include, but are not limited to, cyclopropyl, cyclobutyl, cyclopentyl, and cyclohexyl. It is understood that any of the substitutable hydrogens on a cycloalkyl can be substituted with halogen, C₁-C₃ alkyl, hydroxyl, alkoxy and cyano groups.

The term "heterocycle" as used herein refers to a cyclic hydrocarbon containing 3-6 atoms wherein at least one of the atoms is an O, N, or S. Examples of heterocycles include, but are not limited to, aziridine, oxirane, thiirane, azetidine, oxetane, thietane, pyrrolidine, tetrahydrofuran, tetrahydrothiophene, piperidine, tetrahydropyran, thiane, imidazolidine, oxazolidine, thiazolidine, dioxolane, dithiolane, piperazine, oxazine, dithiane, and dioxane

The term "heteroaryl" as used herein refers to a monocyclic or bicyclic ring structure having 5 to 12 ring atoms wherein one or more of the ring atoms is a heteroatom, *e.g.* N, O or S and wherein one or more rings of the bicyclic ring structure is aromatic. Some nonlimiting examples of heteroaryl are pyridyl, furyl, pyrrolyl, thienyl, thiazolyl, oxazolyl, imidazolyl, indolyl, tetrazolyl, benzofuryl, xanthenes, and dihydroindole. It is understood that any of the substitutable hydrogens on a heteroaryl can be substituted with halogen, C₁-C₃ alkyl, hydroxyl, alkoxy, and cyano groups.

The term "any one of the side chains of the naturally occurring amino acids" as used herein means a side chain of any one of the following amino acids: isoleucine, alanine, leucine, asparagine, lysine, aspartate, methionine, cysteine, phenylalanine, glutamate, threonine, glutamine, tryptophan, glycine, valine, proline, arginine, serine, histidine, and tyrosine.

The term "fatty acid" as used herein means an omega-3 fatty acid and fatty acids that are metabolized *in vivo* to omega-3 fatty acids. Non-limiting examples of fatty acids are *all-cis*-7,10,13-hexadecatrienoic acid, α-linolenic acid (ALA or *all-cis*-9,12,15-*o*ctadecatrienoic acid), stearidonic acid (STD or *all-cis*-6,9,12,15-octadecatetraenoic acid), eicosatrienoic acid (ETE or *all-cis*-11,14,17-eicosatrienoic acid), eicosatetraenoic acid (ETA or *all-cis*-8,11,14,17-eicosatetraenoic acid), eicosapentaenoic acid (EPA or *all-cis-*5,8,11,14,17-eicosapentaenoic acid), docosapentaenoic acid (DPA, clupanodonic acid or *all-cis*-7,10,13,16,19-docosapentaenoic acid), docosahexaenoic acid (DHA or *all-cis-*4,7,10,13,16,19-docosahexaenoic acid), tetracosapentaenoic acid (*all-cis*-9,12,15,18,21-*d*ocosahexaenoic acid), or tetracosahexaenoic acid (nisinic acid or *all-cis*-6,9,12,15,18,21-tetracosenoic acid). In addition, the term "fatty acid" can also refer to medium chain fatty acids such as lipoic acid.

A "subject" is a mammal, *e.g*., a human, mouse, rat, guinea pig, dog, cat, horse, cow, pig, or non-human primate, such as a monkey, chimpanzee, baboon or rhesus, and the terms "subject" and "patient" are used interchangeably herein.

The invention also includes pharmaceutical compositions comprising an effective amount of a bis-fatty acid linked bioactive and a pharmaceutically acceptable carrier. The invention includes a bis-fatty acid linked bioactive provided as a pharmaceutically acceptable prodrug, hydrate, salt, such as a pharmaceutically acceptable salt, enantiomers, stereoisomers, or mixtures thereof.

Representative "pharmaceutically acceptable salts" include, *e.g*., water-soluble and water-insoluble salts, such as the acetate, amsonate (4,4-diaminostilbene-2, 2 - disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fiunarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, magnesium, malate, maleate, mandelate, mesylate, methylbromide, methylnitrate, methylsulfate, mucate, napsylate, nitrate, *N*-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "metabolic disease" as used herein refers to disorders, diseases and syndromes involving dyslipidemia, hypertriglyceridemia, insulin resistance, type 2 diabetes and the terms metabolic disorder, metabolic disease, and metabolic syndrome are used interchangeably herein.

An "effective amount" when used in connection with a linked bioactive is an amount effective for treating or preventing a disease.

The term "carrier," as used in this disclosure, encompasses carriers, excipients, and diluents and means a material, composition or vehicle, such as a liquid or solid filler, diluent, excipient, solvent or encapsulating material, involved in carrying or transporting a pharmaceutical agent from one organ, or portion of the body, to another organ, or portion of the body.

The term "treating," with regard to a subject, refers to improving at least one symptom of the subject's disorder. Treating can be curing, improving, or at least partially ameliorating the disorder.

The term "disorder" is used in this disclosure to mean, and is used interchangeably with, the terms disease, condition, or illness, unless otherwise indicated.

The term "administer," "administering," or "administration" as used in this disclosure refers to either directly administering a compound or pharmaceutically acceptable salt of the compound or a composition to a subject, or administering a prodrug derivative or analog of the compound or pharmaceutically acceptable salt of the compound or composition to the subject, which can form an equivalent amount of active compound within the subject's body.

The term "prodrug," as used in this disclosure, means a compound which is convertible *in vivo* by metabolic means (*e.g.,* by hydrolysis).

The following abbreviations are used herein and have the indicated definitions: Boc and BOC are *tert*-butoxycarbonyl, BoC₂O is di-*tert*-butyl dicarbonate, CDI is 1,1'-carbonyldiimidazole, DCC is *N*,*N'*-dicyclohexylcarbodiimide, DIEA is *N*,*N-*diisopropylethylamine, DMAP is 4-dimethylaminopyridine, DOSS is sodium dioctyl sulfosuccinate, EDC and EDCI are 1-ethyl-3-(3-dimethylaminopropyl)carbodiimide hydrochloride, EtOAc is ethyl acetate, h is hour, HATU is 2-(7-aza-1*H*-benzotriazole-1-yl)-1,1,3,3-tetramethyluronium hexafluorophosphate, HPMC is hydroxypropyl methylcellulose, oxone is potassium peroxymonosulfate, Pd/C is palladium on carbon, TFA is trifluoroacetic acid, TGPS is tocopherol propylene glycol succinate, THF is tetrahydrofuran, and TNF is tumor necrosis factor.

As described above the present invention relates to targeting an intracellular enzyme for release of linked bioactives which results in a synergistic effect between the bioactives. Thus in some embdiments, methods of treatment of a disease using bioactives that are covalently linked by amide bonds and cleaved intracellularly by fatty acid amide hydrolase to release the bioactives to produce a synergistic effect which is not observed when the bioactives are administered alone or in non-linked bioactive are provided. In other embodiments, methods of treatment of a disease using bioactives that are covalently linked by amide bonds and cleaved intracellularly by fatty acid amide hydrolase to release the bioactives to produce an efficacious response without the adverse effects or side effects of the individual components are provided. In even other embodiments, methods of treatment of a disease using bioactives that are covalently linked by amide bonds and cleaved intracellularly by fatty acid amide hydrolase to release the bioactives to produce an efficacious response that is better tolerated than that of the individual components are provided. In other embodimentds, methods of treatment of a disease using bioactives that are covalently linked by amide and disulfide bonds and cleaved intracellularly by thiol reductase and fatty acid amide hydrolase to release the bioactives to produce an efficacious response greater than that of the individual components are provided. In some embodiments, methods of treatment of a disease using bioactives that are covalently linked by amide and disulfide bonds and cleaved intracellularly by thiol reductase and fatty acid amide hydrolase to release the bioactives to produce an efficacious response without the adverse effects or side effects of the individual components are provided. In further embodiments, methods of treatment of a disease using bioactives that are covalently linked by amide and disulfide bonds and cleaved intracellularly by thiol reductase and fatty acid amide hydrolase to release the bioactives to produce an efficacious response that is better tolerated than that of the individual components are provided.

The present invention encompasses the use of any number of intracellular enzymes. In some embodiments, the enzyme is a hydrolase. Hydrolases include a class of enzymes that catalyze the cleavage of various covalent bonds in a substrate by the introduction of a molecule of water. The reaction involves a nucleophilic attack by the water molecule's oxygen atom on a target bond in the substrate. The water molecule is split across the target bond, breaking the bond and generating two product molecules. Hydrolases participate in reactions essential to such functions as synthesis and degradation of cell components and regulation of cell functions including cell signaling, cell proliferation, inflammation, apoptosis, secretion, and excretion. Hydrolases are involved in key steps in disease processes involving these functions. Hydrolases may be grouped by substrate specificity into classes including, but not limited to phosphatases, peptidases, lysophospholipases, phosphodiesterases, glycosidases, and glyoxalases. Hydrolases also include amidases.

Phosphatases hydrolytically remove phosphate groups from proteins, an energy-providing step that regulates many cellular processes, including intracellular signaling pathways that in turn control cell growth and differentiation, cell-cell contact, the cell cycle, and oncogenesis.

Peptidases, also called proteases, cleave peptide bonds that form the backbone of peptide or protein chains. Proteolytic processing is essential to cell growth, differentiation, remodeling, and homeostasis as well as inflammation and the immune response. Since typical protein half-lives range from hours to a few days, peptidases are continually cleaving precursor proteins to their active form, removing signal sequences from targeted proteins, and degrading aged or defective proteins. Peptidases function in bacterial, parasitic, and viral invasion and replication within a host. Nonlimiting examples of peptidases include trypsin and chymotrypsin, components of the complement cascade and the blood-clotting cascade, lysosomal cathepsins, calpains, pepsin, renin, and chymosin (Beynon & Bond (1994) Proteolytic Enzymes: A Practical Approach, Oxford University Press, New York, 1-5).

Lysophospholipases (LPLs) regulate intracellular lipids by catalyzing the hydrolysis of ester bonds to remove an acyl group, a key step in lipid degradation. Small LPL isoforms, approximately 15-30 kilodaltons, can function as hydrolases; larger isoforms can function as both hydrolases and transacylases. A particular substrate for LPLs, lysophosphatidylcholine, causes lysis of cell membranes. LPL activity is regulated by signaling molecules important in numerous pathways, including the inflammatory response.

Phosphodiesterases catalyze the hydrolysis of one of the two ester bonds in a phosphodiester compound. Phosphodiesterases are therefore crucial to a variety of cellular processes. Phosphodiesterases include DNA and RNA endo- and exo-nucleases, which are essential to cell growth and replication as well as protein synthesis. Another phosphodiesterase is acid sphingomyelinase, which hydrolyzes the membrane phospholipid sphingomyelin to ceramide and phosphorylcholine and therefore is involved in numerous intracellular signaling pathways.

Glycosidases catalyze the cleavage of hemiacetal bonds of glycosides, which are compounds that contain one or more sugar.

Other hydrolases act on ether bonds, including the thioether hydrolases.

Another group of hydrolases includes those enzymes which act on carbon-nitrogen (C-N) bonds other than peptide bonds. To this group belong those enzymes hydrolyzing amides *(e.g.* amidases), amidines (*e.g.* amidases), and other C-N bonds. This group can be further subdivided on the basis of the substrate specificity such as linear amides, cyclic amides, linear amidines, cyclic amidines, nitrites, and other compounds.

In other embodiments, the enzyme is a member of the cysteine hydrolase family. Nonlimiting exemplary members of this family include N-acylsphingosine amidohydrolase (acid ceramidase) and N-acylethanolamine-hydrolyzing acid amidase (NAAA).

In other embodiments, the enzyme is a member of the serine hydrolase superfamily of enzymes. Serine hydrolases include a functional class of hydrolytic enzymes that contain a serine residue in their active site. This class of enzymes contains proteinases, esterases, and lipases which hydrolyze a variety of substrates and, therefore, have different biological roles. Proteins in this superfamily can be further grouped into subfamilies based on substrate specificity or amino acid similarities (Puente & Lopez-Ont (1995) Cloning and Expression Analysis of a Novel Human Serine Hydrolase with Sequence Similarity to Prokaryotic Enzymes Involved in the Degradation of Aromatic Compounds J. Biol. Chem. 270(21): 12926-32).

Nonlimiting examples of the serine hydrolase superfamily which relate to the present invention, are as follows: serine proteases (nonlimiting examples include trypsin, chymotrypsin, and subtilisin); extracellular lipases (nonlimiting examples include pancreatic lipase, hepatic lipase, gastric lipase, endothelial lipase, and lipoprotein lipase); intracellular lipases (nonlimiting examples include hormone sensitive lipase, monoacylglycerol lipase, adipose triglyceride lipase, and diacylglycerol lipase); cholinesterases (nonlimiting examples include acetylcholinesterase and butyrylcholinesterase); small molecule thioesterases (nonlimiting examples include fatty acid synthase and the acyl-CoA thioesterases; monoacylglycerol lipases (MAGL); various phospholipases (nonlimiting examples include phospholipase A2 and platelet activating factor acetylhydrolase); protein and glycan hydrolases (nonlimiting examples include protein phosphate methylesterase 1, acyloxyacyl hydrolase and sialic acid acetylesterase); various peptidases (nonlimiting examples include dipeptiyl peptidase 4, fibroblast activation protein, and prolylendopeptidase); and various amidases (nonlimiting examples include, FAAH), among others.

Other enzymes which may hydrolyze the linkage or linker group to yield the free bioactives are butyrylcholinesterase, aminoacylase 1 (acyl) and L-asparagine amidohydrolase.

In one embodiment, the serine hydrolase is fatty acid amide hydrolase (FAAH). In another embodiment, the hydrolase is N-acylethanolamine-hydrolyzing acid amidase (NAAA).

In another embodiment, the hydrolase is N-acylsphingosine amidohydrolase (acid ceramidase).

In another embodiment, the hydrolase is arylformamidase (Afmid).

In another embodiment, the intracellular reduction of the disulfide bond to the corresponding thiol species takes place via the action of certain thiol reductases. The characterization of a gamma-interferon-inducible lysosomal thiol reductase has been described in Arunachalam et al, PNAS, 2000, 97, p. 645-750.

The enzyme to be used is intracellular. Accordingly, the enzyme may be localized to the nuclear compartment, the lysosome, the endosome, the intercisternal space, any organelle, and the cytosol. Also, there may instances in which some copies of an enzyme are in a different area than others.

Disease states that can be treated using the present invention include, but are not limited to diseases associated with inflammation. The inflammation can be associated with an inflammatory disease or a disease where inflammation contributes to the disease. Inflammatory diseases can arise where there is an inflammation of the body tissue. These include local inflammatory responses and systemic inflammation. Examples of such diseases include, but are not limited to: organ transplant rejection; reoxygenation injury resulting from organ transplantation (Grupp et al. Protection Against Hypoxia-reoxygenation in the Absence of Poly (ADP-ribose) Synthetase in Isolated Working Hearts J. Mol. Cell. Cardiol. 1999, 31, 297-03) including, but not limited to, transplantation of the following organs: heart, lung, liver and kidney; chronic inflammatory diseases of the joints, including, but not limited to, arthritis, rheumatoid arthritis, osteoarthritis and bone diseases associated with increased bone resorption; inflammatory bowel diseases, nonlimiting examples include ileitis, ulcerative colitis, Barrett's syndrome, and Crohn's disease; inflammatory lung diseases, nonlimiting examples include asthma, adult respiratory distress syndrome, chronic obstructive airway disease, and cystic fibrosis; inflammatory diseases of the eye, nonlimiting examples include corneal dystrophy, trachoma, onchocerciasis, uveitis, sympathetic ophthalmitis and endophthalmitis; chronic inflammatory diseases of the gum, , nonlimiting examples include gingivitis and periodontitis; inflammatory diseases of the kidney, nonlimiting examples include uremic complications, glomerulonephritis and nephrosis; inflammatory diseases of the skin , nonlimiting examples include sclerodermatitis, psoriasis and eczema; inflammatory diseases of the central nervous system, nonlimiting examples include chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; metabolic disease, nonlimiting examples include type II diabetes mellitus; the prevention of type I diabetes; dyslipidemia; hypertriglyceridemia; diabetic complications, including, but not limited to, glaucoma, retinopathy, macula edema, nephropathy, such as microalbuminuria and progressive diabetic nephropathy, polyneuropathy, diabetic neuropathy, atherosclerotic coronary arterial disease, peripheral arterial disease, nonketotic hyperglycemichyperosmolar coma, mononeuropathies, autonomic neuropathy, joint problems, and a skin or mucous membrane complication, nonlimiting examples include an infection, a shin spot, a candidal infection or necrobiosis lipoidica diabeticorum; immune-complex vasculitis, systemic lupus erythematosus; and inflammatory diseases of the heart such as cardiomyopathy, ischemic heart disease hypercholesterolemia, and atherosclerosis.

Also included are various other diseases that can have significant inflammatory components, nonlimiting examples include preeclampsia; chronic liver failure, brain and spinal cord trauma, and cancer. The inflammatory disease can also be a systemic inflammation of the body, exemplified by gram-positive or gram negative shock, hemorrhagic or anaphylactic shock, or shock induced by cancer chemotherapy in response to proinflammatory cytokines, a nonlimiting example being shock associated with proinflammatory cytokines. Such shock can be induced, by way of nonlimiting example, by a chemotherapeutic agent that is administered as a treatment for cancer. Other disorders include depression, obesity, allergic diseases, acute cardiovascular events, arrhythmia, prevention of sudden death, inflammatory myopathies, nonlimiting examples include dermatomositis, inclusion body myositis, and olymyositis, and cancer cachexia.

The invention also pertains to muscle wasting diseases and muscular dystrophies, including but not limited to, Duchenne Muscular Dystrophy, Becker Muscular Dystrophy, Emery-Dreifuss Muscular Dystrophy, Facioscapulohumeral Muscular Dystrophy, Limb-girdle Muscular Dystrophy, Myotonia Congenita, and Myotonic Dystrophy.

The invention also pertains to diseases afflicting particular organs. As a nonlimiting example, the organ may be the liver. Therefore, this invention may be used with various types of hepatitis, including but not limited to, Nonalcoholic steatohepatitis (NASH). Further this invention pertains to the varieties of Fatty Liver Disease. Also, this invention relates to Cirrhosis of the liver, including but not limited to Primary Biliary Cirrhosis (PBC). As another nonlimiting example, the organ may be the kidney and this invention may be used to treat or prevent various kidney diseases such as chronic kidney disease (CKD), IgA nephropathy and nephropathic cystinosis.

Also, inflammation that results from surgery and trauma can be treated with the compositions and methods of the invention.

One embodiment of the invention is the design of a linker group or groups based on the correlation to an intracellular enzyme or enzymes action on a linkage. Thus, by way of nonlimiting examples, linkages comprising chemical bonds that covalently join the bioactive consisting of one or more of phosphate, peptide bonds, amides, thioamides, esters, phosphodiester, hemiacetal, ethers, thioethers, disulfides, C-N bonds other than amides, among others, are encompassed in this invention.

In some embodiments, the invention encompasses treating diseases in a subject by designing a linked bioactive comprising a linker group which comprises linkages which linker group and/or linkage can be acted upon by intracellular enzymes expressed in target diseased tissue. When the intracellular enzyme is expressed in said target diseased tissue and the linked bioactive is delivered to this tissue the enzyme acts upon the linker group thereby releasing the free bioactives which accumulate in the target tissue thereby treating the disease. Some disease states upregulate expression of intracellular enzymes, while in other disease states the expression of certain enzymes are upregulated while the expression of other enzymes is downregulated. Such simultaneous upregulation and downregulation of different enzymes in certain tisues allows for selective use of linkages and/or enzyme subtrates together in linker groups to deliver linked bioactives that are released in just those tissues.

Non-limiting examples of correlation of FAAH with certain disease states are provided below. There are currently two known human FAAH enzymes, which share 20% sequence identity and are referred to as FAAH-1 and FAAH-2 (Cravatt et al, J. Biological Chemistry 2006, 281, p. 36569-36578).

For instance, the expression of FAAH has been correlated with neurodegenerative diseases such as Alzheimer's Disease and Huntington's Disease. For example, in the case of Alzheimer's Disease, selective enhancement of FAAH expression and activity has been observed in glial cells that are linked to the inflammatory processes that that accompany this disease. (Quinn & Wang (eds.), Lipids in Health and Disease, Volume 49, Springer 2008). Also, in Alzheimer's Disease, FAAH is overexpressed in astrocytes surrounding neuritic plaques (*see,* for example, C. Benito, et al. (Dec. 2003) J. Neuroscience 23(35): 11136-41).

Also, FAAH expression has been linked to forms of arthritis. For example, in one report, FAAH expression was identified in the knee synovia of patients with osteoarthritis and rheumatoid arthritis (Richardson, et al. Characterisation of the Cannabinoid Receptor System in Synovial Tissue and Fluid in Patients with Osteoarthritis and Rheumatoid Arthritis Arthritis Research & Therapy 2008, 10(2): R43).

Also, expression levels of FAAH have been correlated with certain cancers. For example, elevated FAAH protein expression has been detected in prostate cancer tissue as compared to normal prostate tissue samples (Endsley et al. Expression and Function of Fatty Acid Amide Hydrolase in Prostate Cancer Int. J. Cancer: 123, 1318-1326 (2008)). By way of further example, inhibition of FAAH increased endocannabinoid concentrations and reduced the earliest identifiable neoplastic lesions through increased apoptosis in a mouse colon carcinogenic model, indicating that FAAH expression and activity may contribute to tumorigenesis (Izzo et al. Increased Endocannabinoid Levels Reduce the Development of Precancerous Lesions in the Mouse Colon. J Mol Med 2007;86:89-98.).

Expression levels of FAAH have been correlated with inflammation. The expression of FAAH is upregulated in T-lymphocytes by leptin (leptin increases fat mass and inflammation in adipose increased). (Maccarrone, M. et al., Leptin Activates the Anandamide Hydrolase Promoter in Human T Lymphocytes through STAT3, Journal of Biological Chemistry, 2003, 278 (11):13318-324.

In another example, plasma FAAH levels are upregulated during sepsis in humans (Tanaka M, et al., The mRNA Expression of Fatty Acid Amide Hydrolase in Human Whole Blood Correlates with Sepsis. J Endotoxin Res 2007, 13: 35-8), and FAAH expresion increased in the intestines of mice when they were treated with LPS (De Filippis, D., et al., Effect of Cannabidiol on Sepsis-induced Motility Disturbances in Mice: Involvement of CB1 Receptors and Fatty Acid Amide Hydrolase, Neurogastroenterol Motil (2008) 20, 919-927.

In another example, in rats suffering from spinal cord injury, FAAH expression is down regulated but the expression of monoglyceride lipase (MAGL) is upregulated which is the main contributor to 2-arachidonoylglycerol hydrolysis in the brain (Garcia-Ovejero, D. et al., The Endocannabinoid System is Modulated in Response to Spinal Cord Injury in Rats, Neurobiology of Disease 2009, 33:57-71.

In some embodiments, the expression of intracellular enzymes in tissue types determines the selection of certain linker groups and/or linkages which act as substrates of the enzyme. A tissue may be a mass of connected cells and/or extracellular matrix material. Nonlimiting examples of tissue are skin tissue, nasal passage tissue, CNS tissue, neural tissue, eye tissue, liver tissue, kidney tissue, placental tissue, mammary gland tissue, gastrointestinal tissue, musculoskeletal tissue, genitourinary tissue, bone marrow, and the like, derived from, as nonlimiting examples, a human or other mammal and includes the connecting material and the liquid material in association with the cells and/or tissues.

Those of skill in the art are familiar with techniques that may be used to detect instances of gene expression or protein expression correlation with a disease state or a tissue type. In some embodiments, the gene expression or protein expression is indicative of a particluar enzyme expresed in target diseased tissue. As a nonlimiting example, immunoassays may be used. Immunoassays can include any analysis or examination of materials that generates a measurable result and involve the association of an antibody with an antigen. Immunoassays rely on the generation of a signal to yield a measurable result (Lefkovits Immunology Methods Manual: The Comprehensive Sourcebook of Techniques, Vol 1-4, Academic Press, Inc., New York, 1996). This signal may be, as nonlimiting examples, radioactive, colorimetric, fluorescent, luminescent, etc. The signal may be, as nonlimiting examples, associated with the antibody, antigen, a secondary antibody, or any other material in the assay.

Immunoassays can be classified as either competitive or noncompetitive. In one-step competitive immunoassays, unlabeled antigen is measured for its ability to compete with labeled antigen for binding to the antibody's binding site (which is present in a limited amount). Therefore, the signal is inversely related to the amount of unlabeled antigen in the assay. Two-step competitive assays involve incubating an unlabeled antigen of interest with an excess of antibody and subsequently introducing labeled competing antigen. As in one step competitive assays, the amount of signal is inversely related to the amount of unlabeled antigen. Noncompetitive assays directly measure the binding of antibody to antigen, thus providing a direct proportionality of amount of signal and amount of measured antigen. These immunoassays can be one-step or two-step and include sandwich assays. Sandwich assays typically involve two antibodies binding to an antigen such that the antigen is "sandwiched" between the two antibodies. Frequently, one of the antibodies is immobilized to a solid support.

Immunoassays can also be homogenous and heterogeneous assays. In the former, measurement is taken directly on the antibody-antigen complex and therefore there is no need to separate the antibody-antigen complex from the remainder of the assay components. The latter requires isolation of the antibody-antigen complex from the assay components before measurement.

A nonlimiting exemplary immunoassay is a radioimmunoassay in which a radioactive isotope is used to detect an analyte using either a competitive or non-competitive experiment. Another nonlimiting exemplary immunoassay is an enzymatic immunoassay in which an enzyme is used to generate the detection label. Useful enzymes include alkaline phosphatase, horseradish peroxidase, and beta-galactosidase and the signal may be a color change or an emission of light. Another nonlimiting exemplary immunoassay is a fluorescent immunoassay in which a fluorescent label is used as a signal; the label could be on, for example, but not limited to, an antigen or antibody. An nonlimiting example of a fluorescent immunoassay is a the fluorescence polarization immunoassay which takes advantage of the slow rotation of larger molecules (such as a complex of antibody and antigen) and uses polarization of light to differentiate the slower antibody-antigen complexes from the smaller antibody or antigen molecules.

Nonlimiting exemplary immunoassays include: turbidimetry; western blot immunoprecipitation; chromatin immunoprecipitation; immunodiffusion; Ouchterlony double immunodiffusion; radial immunodiffusion; immunoelectrophoresis; counterimmunoelectrophoresis; ELISA; enzyme multiplied immunoassay technique; RAST test; agglutination; hemagglutination/hemagglutinin (Coombs test); latex fixation test; nephelometry; complement fixation test; immunohistochemistry; epitope mapping; skin allergy test; and patch test. Protocols for such methods are known to those in the art.

Other protein expression assays may also be used in this invention. Nonlimiting examples include proteomics methods, mass spectrometry, and other methods known to those in the art.

One skilled in the art would appreciate, based on the disclosure provided herein, that the level of expression and/or activity of an intracellular enzyme or enzymes can also be measured by determining the level of expression and/or activity of DNA or RNA encoding the intracellular enzyme or enzymes. Further, nucleic acid-based detection methods, such as Northern blot, nuclease protection assays, in situ hybridization, and polymerase chain reaction (PCR) assays and the like, can be used. For instance, real time polymerase chain reaction (RT-PCR) may be used for detection of expression levels of an intracellular enzyme or enzymes. In addition, the level of intracellular enzyme or enzymes activity in a cell can also be assessed by determining the level of various parameters which can be affected by the activity of intracellular enzyme or enzymes such as, by way of nonlimiting example, the level of substrate. Thus, one skilled in the art would appreciate, based upon the extensive disclosure provided herein, that there are a multitude of methods which can be used to assess the expression levels of an intracellular enzyme or enzymes.

Another embodiment of the present invention pertains to assaying for the functional state of certain enzymes in disease states and tissue environments in an effort to select an appropriate linkage based on the enzyme and bioactives based on disease to be treated. In light of the fact that many proteins are regulated by a variety of post-translational mechanisms, protein abundance assaying may not always correlate with protein activity. For example, some proteins, such as enzymes, are subject to modification. For instance, the active site of an enzyme represents only a small portion of the entire surface of the protein. The chemical nature and reactivity of this active site is governed by the local environment of the site, which is conferred by its amino acid compositions and its three dimensional structure. The shape and/or exposure of the active site of an enzyme can be modulated by any number of biological events. In many cases, the active site of an enzyme can be masked by natural inhibitors. Alternatively, the shape of the active site can be made more favorable for activity by the action of allosteric cofactors. Therefore, measuring protein abundance alone may not account for the status of an enzyme's active site and therefore the functional state of the enzyme.

Accordingly, an activity-based protein profiling (ABPP) system, in which active site-directed probes are used to record variations in the activity of proteins in whole proteomes, may be employed in this invention (U.S. Patent Application No. 2002/0182652 and Liu, et al. Activity-Based Protein Profiling: The Serine Hydrolases Proc. Nat'l Acad. Sci 1999 96(26): 14694-99). ABPP probes label active enzymes, but not their inactive precursor or inhibitor-bound forms, and thus report on the major post-translational events that regulate enzyme *function in vivo* (Jessani & Cravatt The Development and Application of Methods for Activity-Based Protein Profiling. Curr. Opin. Chem. Biol. 2004, 8, 54-59). ABPP probes typically include three moieties: a binding group that promotes interactions with the active sites of specific classes of enzymes, a reactive group that covalently labels these active sites, and a reporter group (e.g., fluorophore, biotin) for the visualization and affinity purification of probe-labeled enzymes.

Another embodiment of this aspect of the invention is the use of intracellular enzyme inhibitors to determine the dependency of linked bioactives on specific intracellular enzymes for efficacy in treating a condition.

In some embodiments, FAAH is the targeted intracellular enzyme and nonlimiting exemplary FAAH inhibitors that are useful include phenylmethylsulfonylfluoride (PMSF), methoxy arachidonyl fluorophosphonate (MAFP), arachidonoyltrifluoromethylketone (ATMK), URB532, URB597, PF-622, PF-3845, and PF-750 (see, for example, Ahn K, et al. (Nov. 2007) Novel Mechanistic Class of Fatty Acid Amide Hydrolase Inhibitors with Remarkable Selectivity Biochemistry 46(45): 13019-30). Celecoxib (CELEBREX®; 4-[[5-(p-tolyl)-3-(trifluoromethyl)-1H-pyrazol-1-yl]]benzene sulfonamide) (U.S. Pat. No. 5,466,823) and valdecoxib (BEXTRA®; 4-(5-methyl-3-phenyl-isoxazol-4-yl)benzenesulfonamide) (U.S. Pat. No. 5,633,272) are also effective in inhibiting FAAH.

Both reversible and irreversible inhibitors are encompassed by this invention. Therefore, electrophiles that irreversibly inhibit FAAH by covalently linking to the catalytic nucleophile, Ser-241, at the active site are included here (See Kathuria, et al., Modulation of Anxiety Through Blockade of Anandamide Hydrolysis, Nature Medicine, 2003, 9(1): 76-81; Patricelli, et al. Chemical and Mutagenic Investigations of Fatty Acid Amide Hydrolase: Evidence for a Family of Serine Hydrolases with Distinct Catalytic Properties, Biochemistry, 1999, 38(31): 9804-12; Boger, et al., Exceptionally Potent Inhibitors of Fatty Acid Amide Hydrolase: the Enzyme Responsible for Degradation of Endogenous Oleamide and Anandamide, Proc Nat'l Acad Sci 2000, 97(10): 5044-49). Also, reversible FAAH inhibitors have been identified and found to be efficacious in animal models of pain (See Boger, et al., Discovery of a Potent, Selective, and Efficacious Class of Reversible - Ketoheterocycle Inhibitors of Fatty Acid Amide Hydrolase Effective as Analgesics , J. Med. Chem., 2005, 48: 1849-1856).

In some embodiments, thiol reductase is involved in the enzymatic release of the bioactives and a number of thiol reductase inhibitors can be used to examine the hydrolysis process. A number of thioredoxin reductase inhibitors have been reported in S. Urig and K. Becker, Seminars in Cancer Biology 2006, 16, p. 452-465 and Klossowski et al, J. Med. Chem. 2012, 55, p. 55-67, the contents of which are hereby incorporated by reference in their entirety. Additional disulfide-reductase inhibitors, such as clomipramine and mepacrine, have also been described in Schirmer et al, Angew. Chem. Int. Ed. Engl. 1995, 34, p. 141-154, the contents of which are hereby incorporated by reference in their entirety.

Another embodiment is the use of an appropriate intracellular enzyme(s) to cleave a linker group that connects two or more bioactive molecules in the treatment of a disease state.

In certain embodiments, two of the same bioactives are linked together. In some embodiments, more than two of the same bioactives are linked together. In some embodiments, more than three of the same bioactives are linked together. In some embodiments, more than four of the same bioactives are linked together. In certain embodiments, two or more distinct bioactives are linked together. In certain embodiments, three or more distinct bioactives are linked together. In certain embodiments, four or more distinct bioactives can be linked in a single linked bioactive.

In certain embodiments the bioactives to be used are selected based on the use of the bioactives against a specific disease. For instance, in the treatment of a specific disease in the manner described herein, establishment of the identity of the bioactives for use could be made by reference to the scientific literature, FDA Orange Book, United States Pharmacopeia, or some other source known in the art.

In certain embodiments, the bioactives to be used are from a family of cholesterol-lowering agents. Non limiting examples of cholesterol-lowering agents are atorvastatin, cerivastatin, fluvastatin, lovastatin, pitavastatin, pravastatin, rosuvastatin, simvastatin, ezetimibe, and the combination of ezetimibe/simvastatin (VYTORIN®).

In certain embodiments, the bioactive to be used is a fibrate or hypolipidemic agent. Non-limiting examples of fibrates or hypolipidemic agents are acifran, acipimox, beclobrate, bezafibrate, binifibrate, ciprofibrate, clofibrate, colesevelam, gemfibrozil, fenofibrate, melinamide, niacin, and ronafibrate.

In certain embodiments, the bioactive to be used is an anti-diabetic agent. Non-limiting examples of anti-diabetic agents are metformin and pioglitazone.

In certain embodiments, the bioactive to be used is an antiepileptic agent. Non-limiting examples of antiepileptic agents include gabapentin and pregabalin.

In certain embodiments, the bioactive to be used is an antiglaucoma agent. Non-limiting examples of antiglaucoma agents include, bimatroprost, latanoprost, tafluprost, and travoprost.

In certain embodiments, the bioactive to be used is an antihypertensive agent. Non-limiting examples of antihypertensive agents include alacepril, alfuzosin, aliskiren, amlodipine besylate, amosulalol, aranidipine, arotinolol HCl, azelnidipine, barnidipine hydrochloride, benazepril hydrochloride, benidipine hydrochloride, betaxolol HCl, bevantolol HCl, bisoprolol fumarate, bopindolol, bosentan, budralazine, bunazosin HCl, candesartan cilexetil, captopril, carvedilol, celiprolol HCl, cicletanine, cilazapril, cinildipine, clevidipine, delapril, dilevalol, doxazosin mesylate, efonidipine, enalapril maleate, enalaprilat, eplerenone, eprosartan, felodipine, fenoldopam mesylate, fosinopril sodium, guanadrel sulfate, imidapril HCl, irbesartan, isradipine, ketanserin, lacidipine, lercanidipine, lisinopril, losartan, manidipine hydrochloride, mebefradil hydrochloride, moxonidine, nebivolol, nilvadipine, nipradilol, nisoldipine, olmesartan medoxomil, perindopril, pinacidil, quinapril, ramipril, rilmedidine, spirapril HCl, telmisartan, temocarpil, terazosin HCl, tertatolol HCl, tiamenidine HCl, tilisolol hydrochloride, trandolapril, treprostinil sodium, trimazosin HCl, valsartan, and zofenopril calcium.

In certain embodiments, the bioactive to used is an anti-inflammatory agent. Non-limiting examples of anti-inflammatory agents include ibuprofen, naproxen, indomethacin, salicylic acid (SA), salsalate, 5-aminosalicylic acid (5-ASA), dimethylfumarate, monomethyl fumarate (MMF), methotrexate, prednisone, and fluticasone propionate.

In certain embodiments, the bioactive to be used is an anti-depressant agent. Non-limiting examples of anti-depressant agents include bupropion HCl, citalopram, desvenlafaxine, fluoxetine HCl, fluvoxamine maleate, metapramine, milnacipran, mirtazapine, moclobemide, nefazodone, paroxetine, pivagabine, reboxetine, setiptiline, sertraline HCl, tianeptine sodium, toloxatone, and venlafaxine.

In certain embodiments, the bioactive to be used is an anti-cancer agent. Non-limiting examples of anti-cancer agents include lonidamine, docetaxel, vorinostat, and mitoxantrone HCl.

In certain embodiments, the bioactive agent to be used is an immunosuppressant agent. Non-limiting examples of immunosuppressant agents include mycophenolic acid, mycophenolate sodium, and mycophenolate mofetil.

In some embodiments, the bioactive agent to be used is an agent to treat osteoporosis. Non-limiting examples of agents to treat osteoporosis include raloxifene HCl, lasofoxifene, and bazedoxifene.

In some embodiments, the bioactive agent to be used is an agent to treat multiple sclerosis. Non-limiting examples of agents to treat multiple sclerosis include dimethyl fumarate, mono methyl fumarate, fingolimod, teriflunomide, laquinimod, cladribine, and mitoxantrone HCl.

In some embodiments, the bioactive agent to be used is an antiviral agent. Non-limiting examples of antiviral agents include atazanavir, amprenavir, indinavir, imiquimod, lopinavir, nelfinavir, oseltamivir, ritonavir, saquinavir, rimantadine, darunavir, boceprevir, telaprevir, zanamivir, laninamivir, peramivir, PSI-7977, abacavir, adefovir dipivoxil, cidoflovir, didanosine, emtricitabine, entecavir, lamivudine, famciclovir, ganciclovir, penciclovir, sorivudine, zalcitabine, stavudine, zidovudine (AZT), clevudine, and telbivudine.

In certain embodiments, the identity of the linker group in the linked bioactive is subject to design. The selection of chemical groups in this linker group may be driven by the identity of an intracellular enzyme or enzymes associated with a disease state or tissue type.

In certain embodiments, the linker comprises at least one amide group. In certain embodiments, the linker comprises at least one ester group. In certain embodiments, the linker comprises at least one disulphide group. In certain embodiments, the linker comprises at least one carbamate.

In certain embodiments, the linker comprises at least two amides. In certain embodiments, the linker comprises at least two ester groups. In certain embodiments, the linker comprises at least two ether groups. In certain embodiments, the linker comprises at least one amide and at least one ester. In certain embodiments, the linker comprises at least one amide and at least one ether. In certain embodiments, the linker comprises at least one ester and at least one ether.

Nonlimiting examples of linked bioactives are disclosed in US 2010/0041748; US 2010/0184730; US 2011/0053990; USSN 12/986,713 filed Jan. 7, 2011; and PCT/US2011/026305 and described below.

Compounds of the **Formula I:** 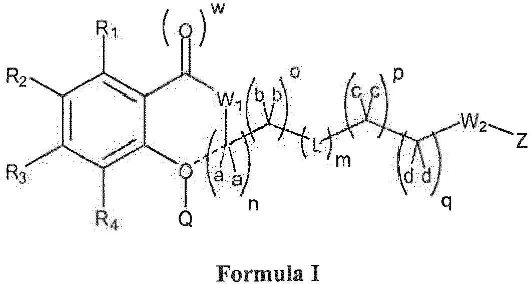 and pharmaceutically acceptable salts, hydrates, solvates, prodrugs, enantiomers, and stereoisomers thereof;
wherein
R₁, R₂, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
W₁ and W₂ are each independently null, O, or NH, or when W₁ and W₂ are both NH, then both W₁ and W₂ can be taken together to form a piperidine moiety;
each symbol - - - - - represents an optional bond, which when present between the phenolic oxygen and the methylene containing substituent a, requires that Q is null, or when present between substituent a and the carbon of the methylene containing substituent a, requires that Q not be null;
each a and c is independently H, CH₃, -OCH₃, -OCH₂CH₃, C(O)OH, C(O)OR or benzyl;
each b is independently H, CH₃, C(O)OH, O-Z, C(O)OR or benzyl;
each d is independently H, C(O)OH, C(O)OR or benzyl;
each n, o, p, and q is independently 0 or 1;
each L is independently -O-, -S-, -S(O)-, -S(O)₂-, -S-S-, 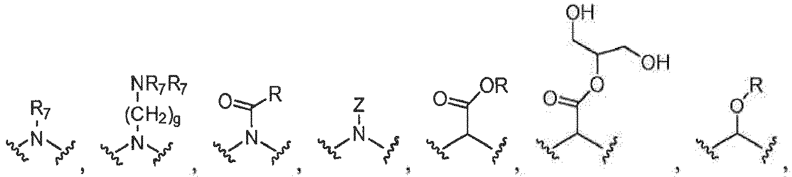 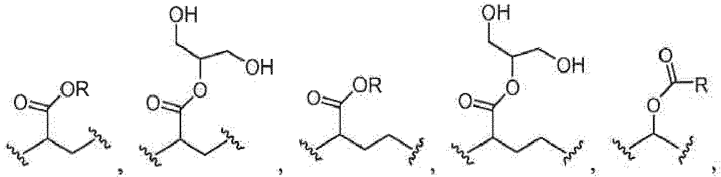 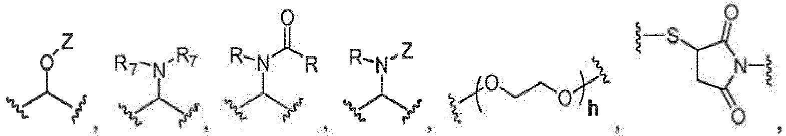 or 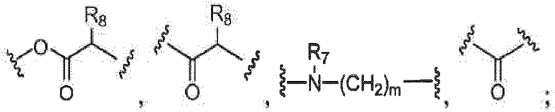 each g is independently 2, 3 or 4;
each h is independently 1, 2, 3 or 4;
m is 0, 1, 2, 3, 4 or 5;
each R₇ is independently H or C₁-C₆ alkyl, or both R₇ groups, when taken together with the nitrogen to which they are attached, can form a heterocycle;
each R₈ is independently e, H or straight or branched C₁-C₁₀ which can be optionally substituted with OH, NH₂, CO₂R, CONH₂, phenyl, C₆H₄OH, imidazole or arginine;
each R is independently H, -C₁-C₃ alkyl, or straight or branched C₁-C₄ alkyl optionally substituted with OH or halogen;
each Z is independently H, or 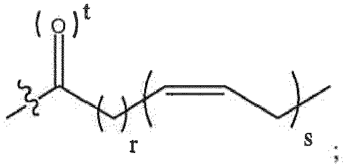 with the proviso that there is at least one 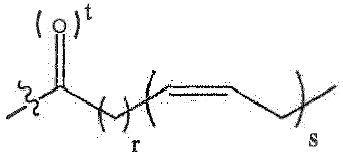 in the compound;
each r is independently 2 or 3;
each s is independently 5 or 6;
w is 0 or 1;
each t is independently 0 or 1;
Q is null, C(O)CH₃, Z, 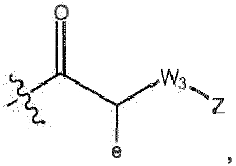 or 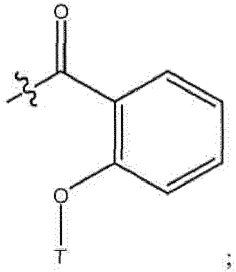 each e is independently H or any one of the side chains of the naturally occurring amino acids;
W₃ is null, -O-, or -N(R)-; and
T is H, C(O)CH₃, or Z;
provided that
when m, n, o, p, and q are each 0, W₁ and W₂ are each null, w is 1, and Z is 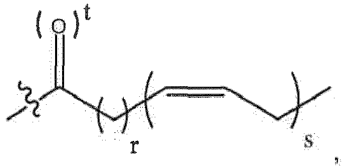 then t must be 0; and
when w is 0, then W₁ is O or NH.

### Compounds of the Formula II:

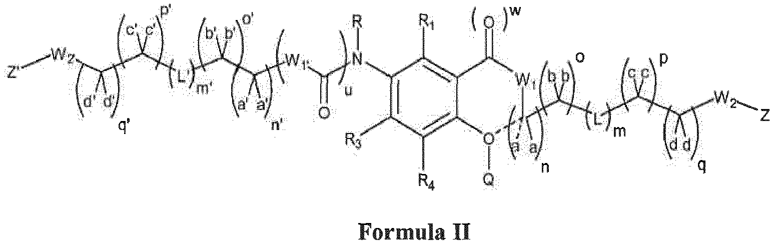

and pharmaceutically acceptable salts, hydrates, solvates, prodrugs, enantiomers, and stereoisomers thereof;
wherein
R₁, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
each W₁, W₁, W₂ and W₂' is independently null, O, or NH, or when W₁ and W₂ or W_{1'} and W_{2'} are both NH, then both W₁ and W₂ or W_{1'} and W_{2'} can be taken together to form a piperidine moiety;
each symbol - - - - - represents an optional bond, which when present between the phenolic oxygen and the methylene containing substituent a, requires that Q is null, or when present between substituent a and the carbon of the methylene containing substituent a, requires that Q not be null;
each a, a', c, and c' is independently H, CH₃, -OCH₃, -OCH₂CH₃, C(O)OH, C(O)OR or benzyl;
each b and b' is independently H, CH₃, C(O)OH, O-Z, C(O)OR or benzyl;
each d and d' is independently H, C(O)OH, C(O)OR or benzyl;
each n, n', o, o', p, p', q, and q' is independently 0 or 1;
each L and L' is independently -O-, -S-, -S(O)-, -S(O)₂-, -S-S-, 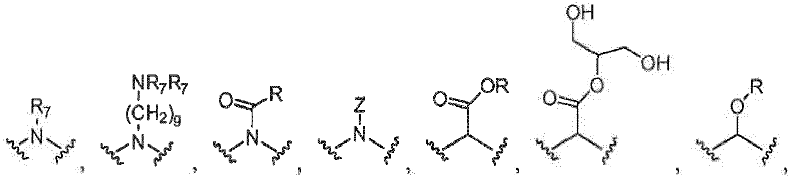 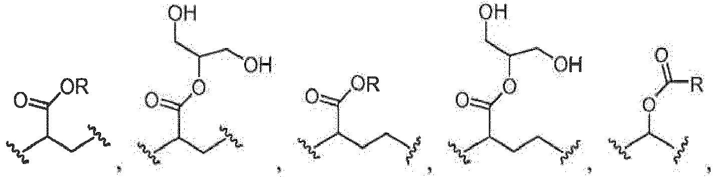 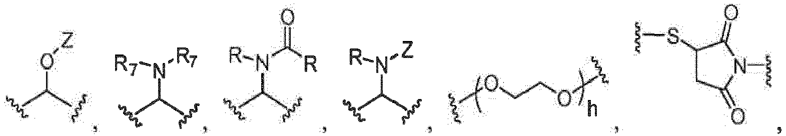 or 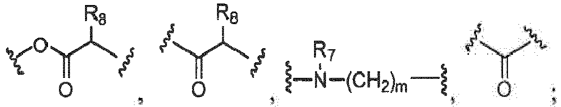 each g is independently 2, 3 or 4;
each h is independently 1, 2, 3 or 4;
each m and m' is independently 0, 1, 2, 3, 4 or 5;
each R₇ is independently H or C₁-C₆ alkyl, or both R₇ groups, when taken together with the nitrogen to which they are attached, can form a heterocycle;
each R₈ is independently e, H or straight or branched C₁-C₁₀ which can be optionally substituted with OH, NH₂, CO₂R, CONH₂, phenyl, C₆H₄OH, imidazole or arginine;
each R is independently H, -C₁-C₃ alkyl, or straight or branched C₁-C₄ alkyl optionally substituted with OH or halogen;
each Z and Z' is independently H, or 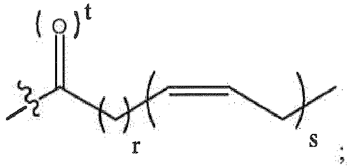 with the proviso that there is at least one 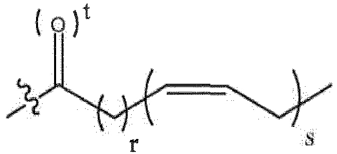 in the compound;
each r is independently 2, 3, or 7;
each s is independently 3, 5, or 6;
each t is independently 0 or 1;
w is 0 or 1;
u is 0 or 1;
Q is null, C(O)CH₃, Z, 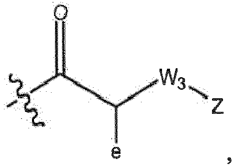 or 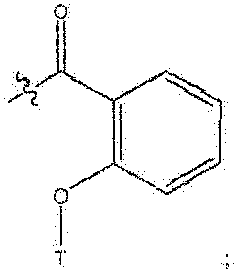 each e is independently H or any one of the side chains of the naturally occurring amino acids;
W₃ is null, -O-, or -N(R)-; and
T is H, C(O)CH₃, or Z;
provided that
when m, n, o, p, and q are each 0, W₁ and W₂ are each null, w is 1, and Z is 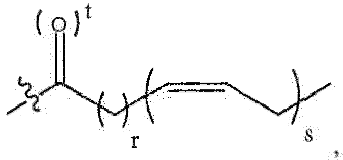 then t must be 0;
when m', n', o', p', and q' are each 0, u is 1, W_{1'} and W_{2'} are each null, and Z' is 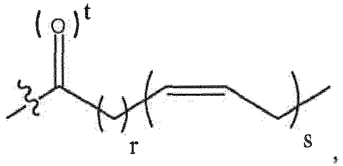 then t must be 0;
when w is 0, then W₁ is O or NH; and
when w is 1, Z is 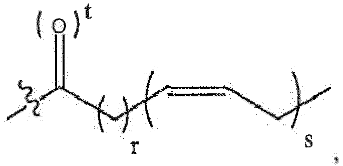 and r is 7, then t is 1.

Compounds of the Formula III: 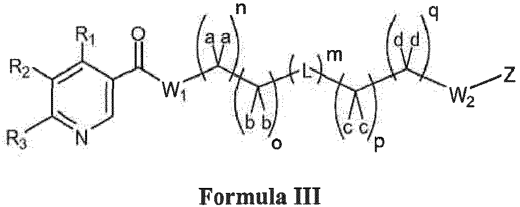 and pharmaceutically acceptable salts, hydrates, solvates, prodrugs, enantiomers and stereoisomers thereof;
wherein
R₁, R₂, and R₃ are each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
W₁ and W₂ are each independently null, O, S, NH, NR, or W₁ and W₂ can be taken together can form an imidazolidine or piperazine group, with the proviso that W₁ and W₂ can not be O simultaneously;
each a, b, c, and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0 or 1;
each L is independently-O-, -S-, -S(O)-, -S(O)₂-, -S-S-, 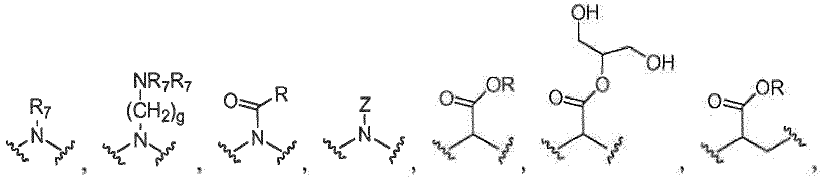 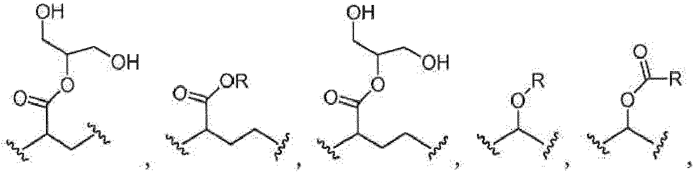 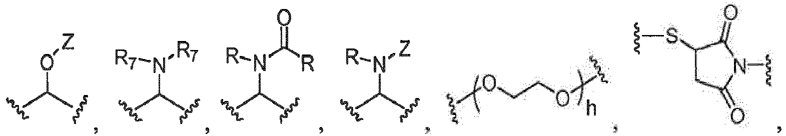 or 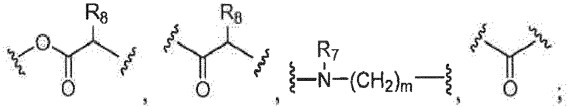 each g is independently 2, 3 or 4;
each h is independently 1, 2, 3 or 4;
m is 0, 1, 2, 3, 4 or 5;
each R₇ is independently H or C₁-C₆ alkyl, or both R₆ groups, when taken together with the nitrogen to which they are attached, can form a heterocycle;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 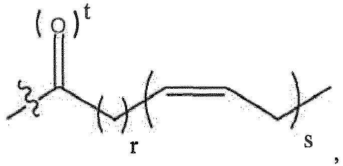 or 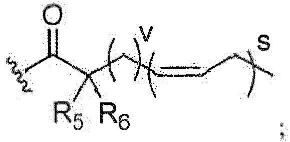 with the proviso that there is at least one 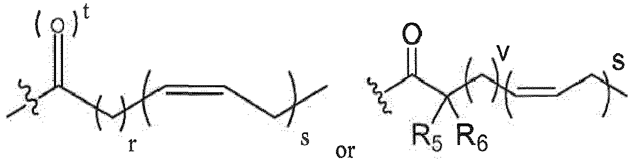 in the compound;
each r is independently 2, 3, or 7;
each s is independently 3, 5, or 6;
each t is independently 0 or 1;
each v is independently 1, 2, or 6;
R₅ and R₆ are each independently hydrogen, deuterium, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently H, -C₁-C₃ alkyl, or straight or branched C₁-C₄ alkyl optionally substituted with OH or halogen;
provided that
when m, n, o, p, and q are each 0, W₁ and W₂ are each null, and Z is 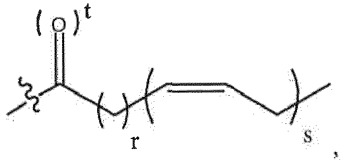 then t must be 0; and
when each of m, n, o, p, and q is 0, and W₁ and W₂ are each null, then Z must not be

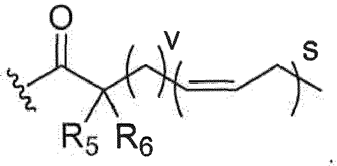

Compounds of the **Formula IV** and **Formula V:** 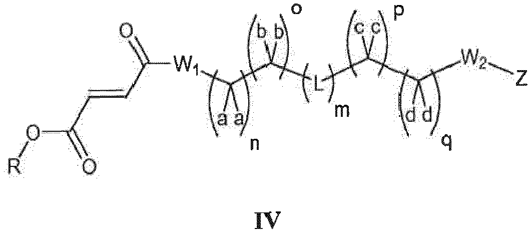 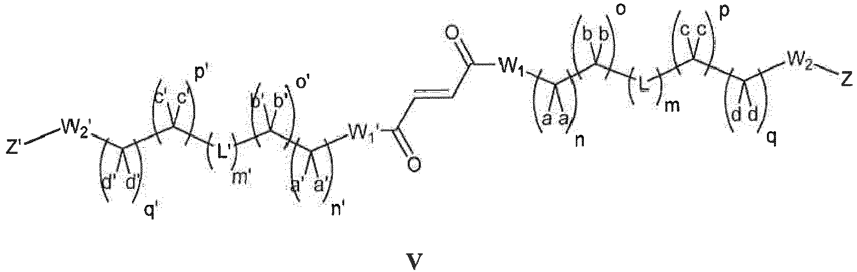 and pharmaceutically acceptable salts, hydrates, solvates, prodrugs, enantiomers, and stereoisomers thereof;
wherein
each W₁, W₂, W₁', and W₂' is independently null, O, S, NH, or NR, or W₁ and W₂, or W₁' and W₂' can be taken together to form an optionally substituted imidazolidine or piperazine group;
each a, b, c, d, a', b', c', and d' is independently -H, -D, -CH₃, -OCH₃, -OCH₂CH₃, -C(O)OR, -O-Z, or benzyl, or two of a, b, c, and d or any two of a', b', c', and d' can be taken together, along with the single carbon to which they are bound, to form a cycloalkyl or heterocycle;
each n, o, p, q, n', o', p', and q' is independently 0, 1, or 2;
each L and L' is independently null, -O-, -C(O)-, -S-, -S(O)-, -S(O)₂-, -S-S-, -(C₁-C₆alkyl)-, -(C₃-C₆cycloalkyl)-, a heterocycle, a heteroaryl, 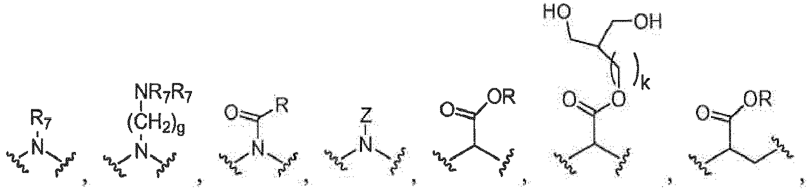 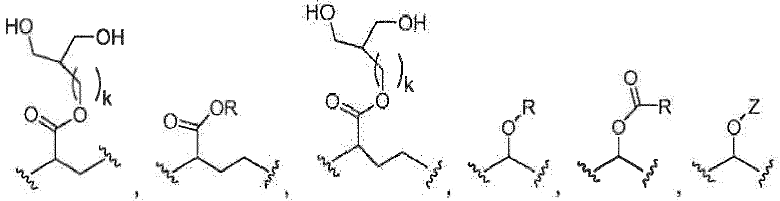 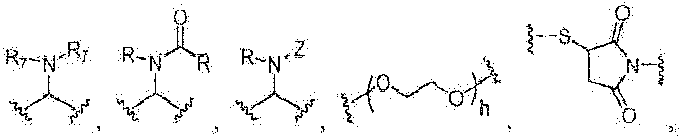 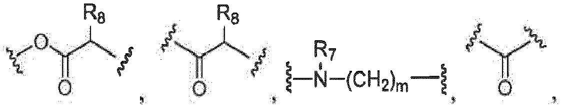 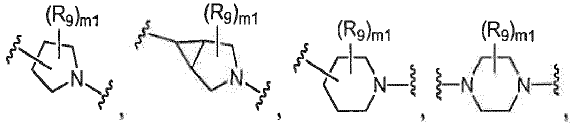 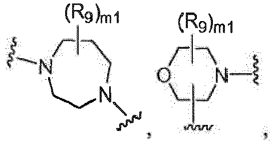 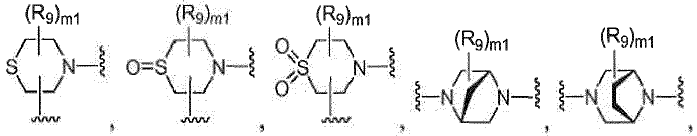 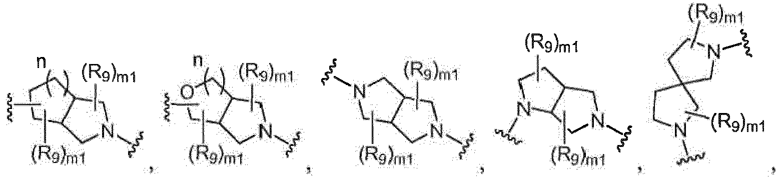 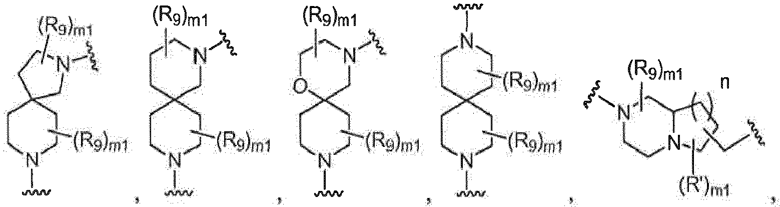 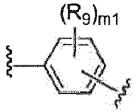 wherein the representation of L and L' is not limited directionally left to right as is depicted, rather either the left side or the right side of L and L' can be bound to the W₁ or W₁' side of the compound of **Formula IV or Formula V,** respectively;
each R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3, or 4;
each h is independently 1, 2, 3, or 4;
each m and m' is independently 0, 1, 2, or 3; if m or m' is more than 1, then L or L' can be the same or different;
each m1 is independently 0, 1, 2, or 3;
k is 0, 1, 2, or 3;
z is 1, 2, or 3;
each R₇ is independently H or optionally substituted C₁-C₆ alkyl, wherein a methylene unit of the C₁-C₆ alkyl can be optionally substituted for either O or NR, and in NR₇R₇, both R₇ when taken together with the nitrogen to which they are attached can form a heterocyclic ring such as a pyrrolidine, piperidine, morpholine, piperazine or pyrrole;
each Z and Z' is independently H, 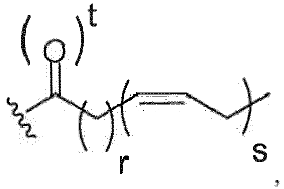 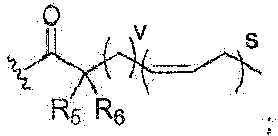 or 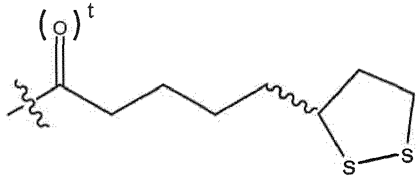 provided that
there is at least one 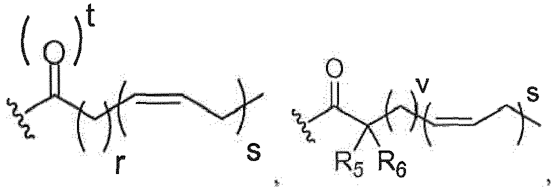 or 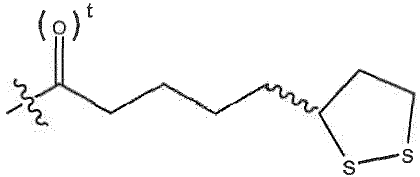 in the compound;
each t is independently 0 or 1;
each r is independently 2, 3, or 7;
each s is independently 3, 5, or 6;
each v is independently 1, 2, or 6;
each R₁ and R₂ is independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each R is independently -H or straight or branched C₁-C₄ alkyl optionally substituted with OH or halogen;
provided that
when each of m, n, o, p, and q, is 0, W₁ and W₂ are each null, and Z is 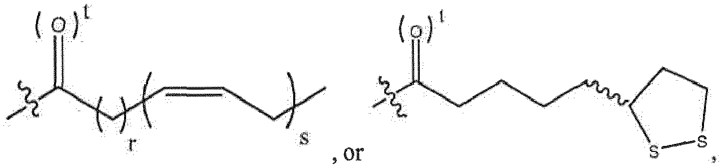 then t must be 0;
when each of m', n', o', p', and q', is 0, W₁' and W₂' are each null, and Z' is 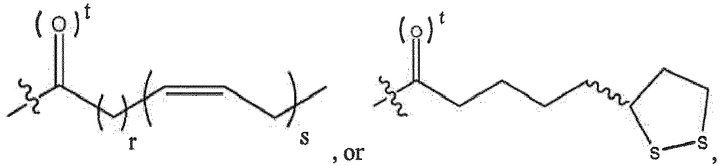 then t must be 0; and
when each of m, n, o, p, and q is 0, and W₁ and W₂ are each null, or when each of m', n', o', p', and q', is 0, W₁' and W₂' are each null, then Z or Z' must not be

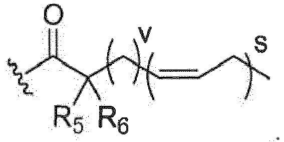

Compounds of the Formula VI: 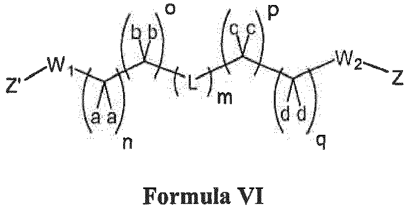 and pharmaceutically acceptable salts, hydrates, solvates, prodrugs, enantiomers and stereoisomers thereof;
wherein
W₁ and W₂ are each independently O, S, NH, NR, or W₁ and W₂ can be taken together can form an imidazolidine or piperazine group;
with the proviso that W₁ and W₂ can not simultaneously be O and one of W1 and W2 is NH or NR;
each a, b, c, and d is independently -H, -D, -CH₃, -OCH₃, -OCH₂CH₃, -C(O)OR, -O-Z, or benzyl, or two of a, b, c, and d can be taken together, along with the single carbon to which they are bound, to form a cycloalkyl or heterocycle;
each n, o, p, and q is independently 0, 1 or 2;
L is independently null, -O-, -S-, -S(O)-, -S(O)₂-, -S-S-, -(C₁-C₆alkyl)-, -(C₃-C₆cycloalkyl)-, a heterocycle, a heteroaryl, 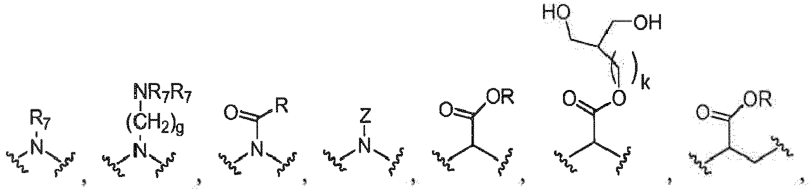 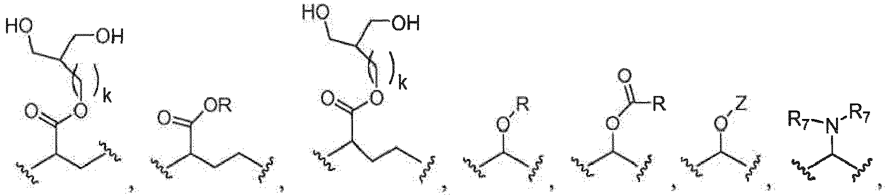 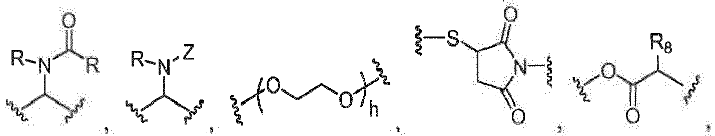 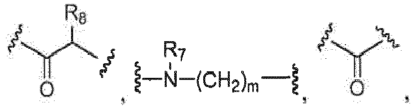 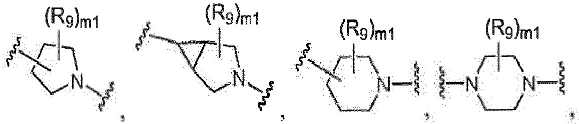 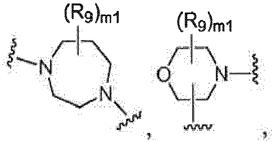 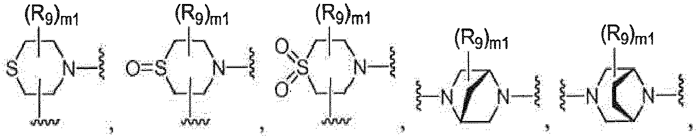 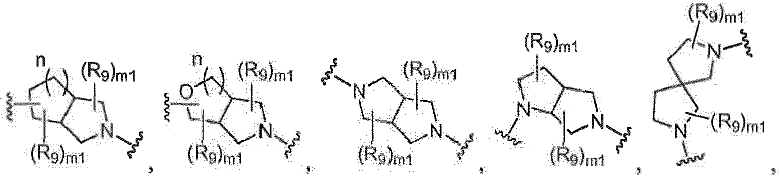 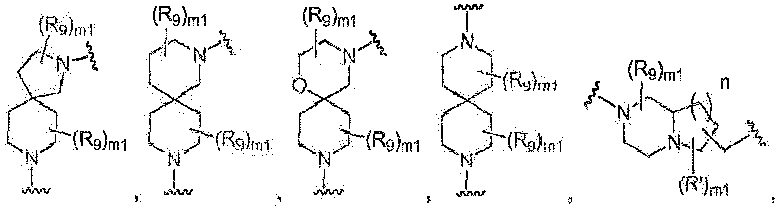 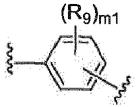 wherein the representation of L is not limited directionally left to right as is depicted, rather either the left side or the right side of L can be bound to the W₁ side of the compound of **Formula VI;**
R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3 or 4;
each h is independently 1, 2, 3 or 4;
m is 0, 1, 2, 3, 4 or 5; if m is more than 1, then L can be the same or different;
m1 is 0, 1, 2 or 3;
k is 0, 1, 2, or 3;
z is 1, 2, or 3;
each R₇ is independently H or C₁-C₆ alkyl that can be optionally substituted with either O or N and in NR₇R₇, both R₇ when taken together with the nitrogen to which they are attached can form a heterocyclic ring such as a pyrrolidine, piperidine, morpholine, piperazine or pyrrole;
each R₈ independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z and Z' is independently -H, or 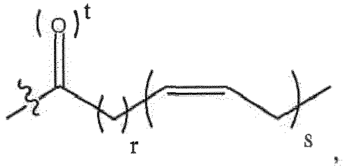 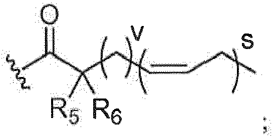 or 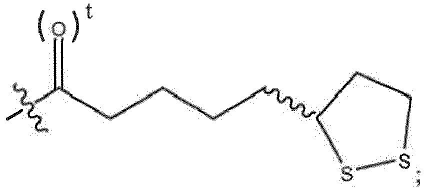 with the proviso that there is at least two of 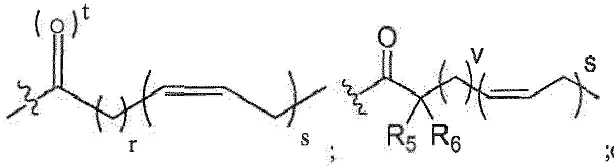 ; or 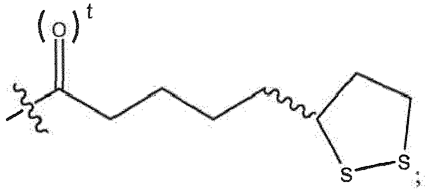 in the compound;
each r is independently 2, 3, or 7;
each s is independently 3, 5, or 6;
each t is independently 0 or 1;
each v is independently 1, 2, or 6;
R₅ and R₆ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, or straight or branched C₁-C₄ alkyl optionally substituted with OH, or halogen;
with the further proviso that the compound is not (4Z,7Z,10Z,13Z,16Z,19Z)-docosa-4,7,10,13,16,19-hexaenoic acid {2-[2-((4Z,7Z,10Z,13Z,16Z,19Z)-docosa-4,7,10,13,16,19-hexaenoylamino)-ethylamino]-ethyl}-amide (**A**)**;** (5Z,8Z,11Z,14Z,17Z)-icosa-5,8,11,14,17-pentaenoic acid {2-[2-((5Z,8Z,11Z,14Z,17Z)-icosa-5,8,11,14,17-pentaenoylamino)-ethylamino]-ethyl}-amide **(**B); (4Z,7Z,10Z,13Z,16Z,19Z)-docosa-4,7,10,13,16,19-hexaenoic acid {2-[2-((4Z,7Z,10Z,13Z,16Z,19Z)-docosa-4,7,10,13,16,19-hexaenoylamino)-ethoxy]-ethyl}-amide (C); (5Z,8Z,11Z,14Z,17Z)-icosa-5,8,11,14,17-pentaenoic acid {2-[2-((5Z,8Z,11Z,14Z,17Z)-icosa-5,8,11,14,17-pentaenoylamino)-ethoxy]-ethyl}-amide **(D);** (S)-2,6-Bis-((4Z,7Z,10Z,13Z,16Z,19Z)-docosa-4,7,10,13,16,19-hexaenoylamino)-hexanoic acid **(E).**

Compounds of the **Formula VII:** 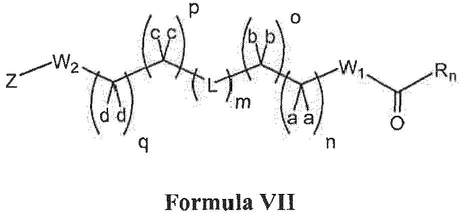 and pharmaceutically acceptable salts, hydrates, solvates, prodrugs, enantiomers, and stereoisomers thereof;
wherein

Rₙ is 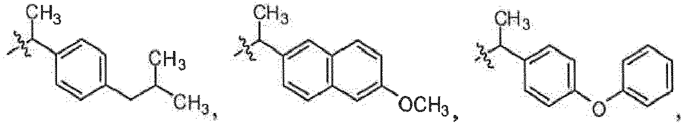 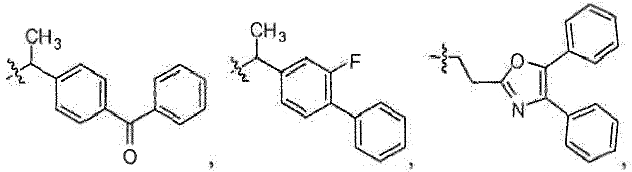 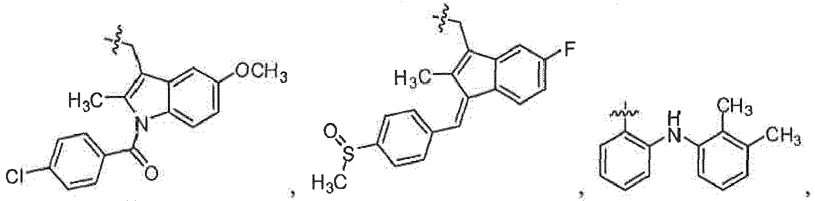 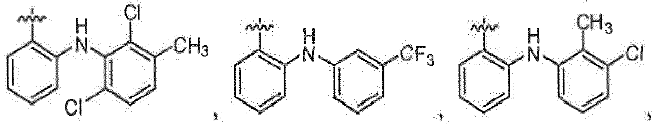 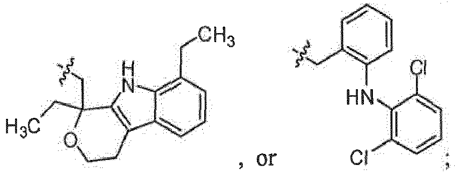 W₁ and W₂ are each independently null, O, S, NH, NR, or W₁ and W₂ can be taken together can form an imidazolidine or piperazine group, with the proviso that W₁ and W₂ can not be 0 simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a heteroaryl, 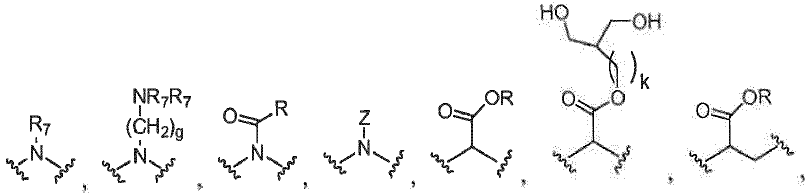 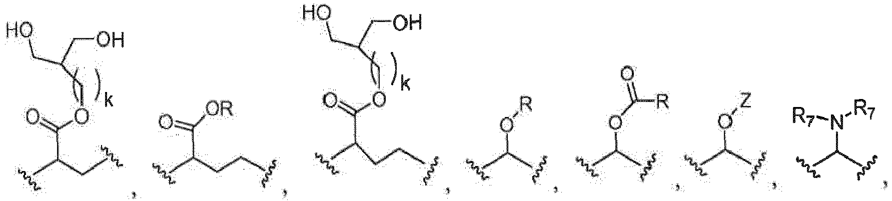 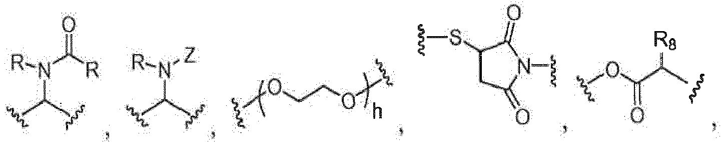 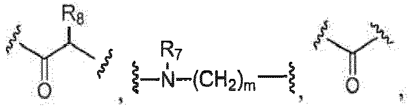 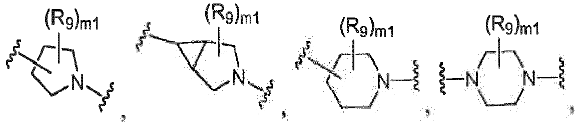 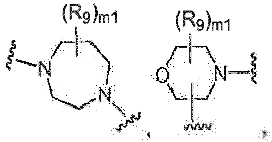 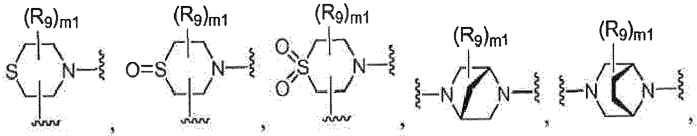 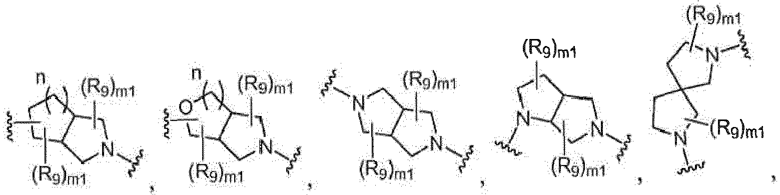 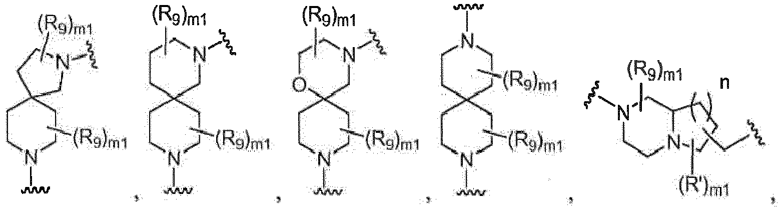 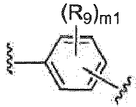 wherein the representation of L is not limited directionally left to right as is depicted, rather either the left side or the right side of L can be bound to the W₁ side of the compound of Formula I;
R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3 or 4;
each h is independently 1, 2, 3 or 4;
m is 0, 1, 2, 3, 4 or 5; if m is more than 1, then L can be the same or different;
m1 is 0, 1, 2 or 3;
k is 0, 1, 2, or 3;
z is 1, 2, or 3;
each R₇ is independently H or C₁-C₆ alkyl that can be optionally substituted with either O or N and in NR₇R₇, both R₇ when taken together with the nitrogen to which they are attached can form a heterocyclic ring such as a pyrrolidine, piperidine, morpholine, piperazine or pyrrole;
each R₈ independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 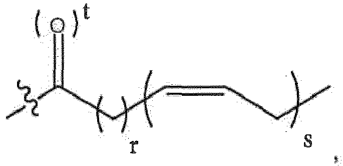 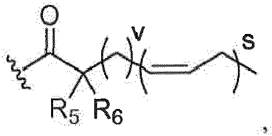 or 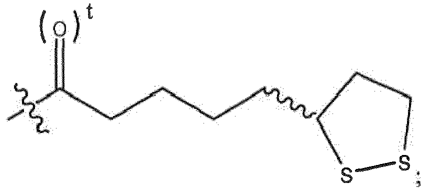 with the proviso that there is at least one 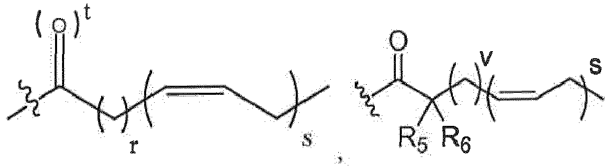 or 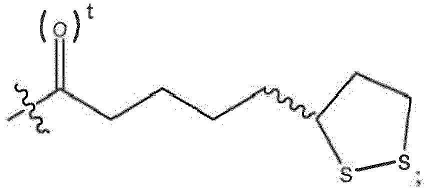 in the compound;
each r is independently 2, 3, or 7;
each s is independently 3, 5, or 6;
each t is independently 0 or 1;
each v is independently 1, 2, or 6;
R₅ and R₆ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, or straight or branched C₁-C₄ alkyl optionally substituted with OH, or halogen;
provided that
when m, n, o, p, and q are each 0, W₁ and W₂ are each null, and Z is 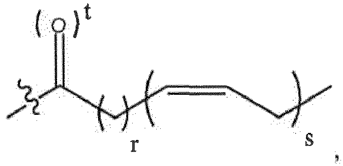 then t must be 0; and
when m, n, o, p, and q are each 0, and W₁ and W₂ are each null, then Z must not be 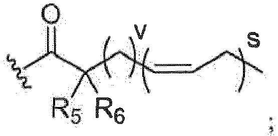 with the further proviso that the compound is not 5Z,8Z,11Z,14Z,17Z)-1-(2-(1-(4-chlorobenzoyl)-5-methoxy-2-methyl-1*H*-indol-3-yl)acetoxy)ethyl icosa-5,8,11,14,17-pentaenoate or 5-((S)-1,2-dithiolan-3-yl)-N-(2-(2-(4-isobutylphenyl)propanamido)ethyl)pentanamide.

Compounds of the **Formula Ia:** 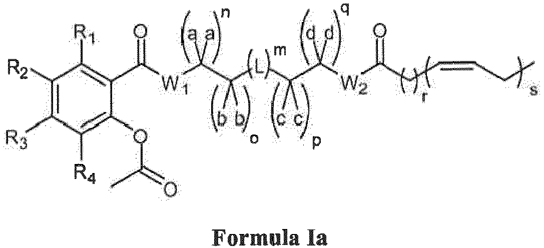 and pharmaceutically acceptable salts, hydrates, solvates, enantiomers, and stereoisomers thereof,
wherein
R₁, R₂, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
W₁ and W₂ are each independently null, O, or NH, or when W₁ and W₂ are both NH, then both W₁ and W₂ can be taken together to form a piperidine moiety;
each a and c is independently H, CH₃, -OCH₃, -OCH₂CH₃, C(O)OH, C(O)OR or benzyl;
each b is independently H, CH₃, C(O)OH, O-Z, C(O)OR or benzyl;
each d is independently H, C(O)OH, C(O)OR or benzyl;
each n, o, p, and q is independently 0 or 1;
each L is independently -O-, -S-, -S(O)-, -S(O)₂-, -S-S-, 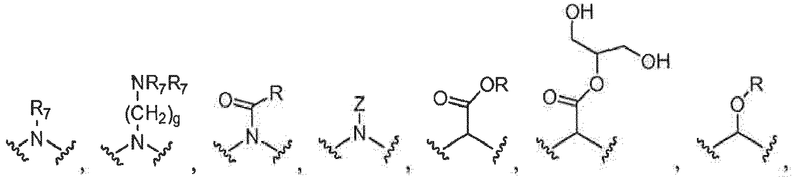 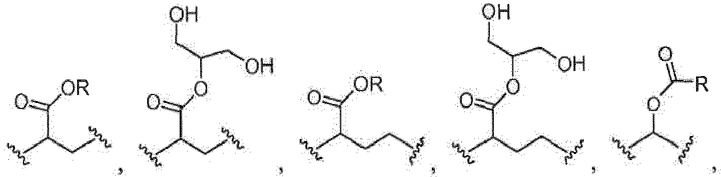 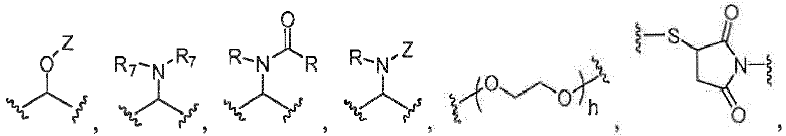 or 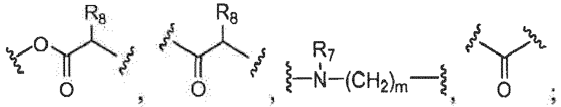 each g is independently 2, 3 or 4;
each h is independently 1, 2, 3 or 4;
m is 0, 1, 2, 3, 4 or 5;
each R₇ is independently H or C₁-C₆ alkyl, or both R₇ groups, when taken together with the nitrogen to which they are attached, can form a heterocycle;
each R₈ is independently e, H or straight or branched C₁-C₁₀ which can be optionally substituted with OH, NH₂, CO₂R, CONH₂, phenyl, C₆H₄OH, imidazole or arginine;
each R is independently H, -C₁-C₃ alkyl, or straight or branched C₁-C₄ alkyl optionally substituted with OH or halogen;
each Z is independently H, or 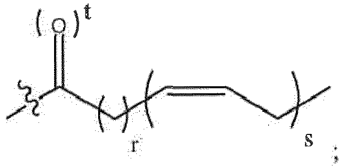 each r is independently 2 or 3;
each s is independently 5 or 6;
each t is independently 0 or 1, and
each e is independently H or any one of the side chains of the naturally occurring amino acids;
provided that
when m, n, o, p, and q are each 0, W₁ and W₂ must not both be null; and when W₁ and W₂ are each null, one of m, n, o, p, and q must be at least 1.

Compounds of the **Formula Ib:** 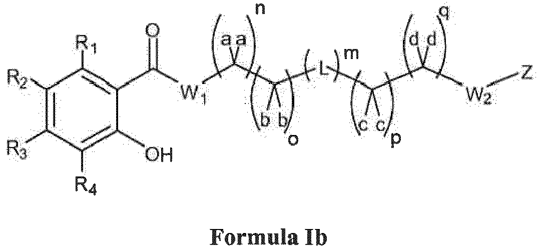 and pharmaceutically acceptable salts, hydrates, solvates, prodrugs, enantiomers, and stereoisomers thereof;
wherein
R₁, R₂, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
W₁ and W₂ are each independently null, O, or NH, or when W₁ and W₂ are both NH, then both W₁ and W₂ can be taken together to form a piperidine moiety;
each a and c is independently H, CH₃, -OCH₃, -OCH₂CH₃, C(O)OH, C(O)OR or benzyl;
each b is independently H, CH₃, C(O)OH, O-Z, C(O)OR or benzyl;
each d is independently H, C(O)OH, C(O)OR or benzyl;
each n, o, p, and q is independently 0 or 1;
each L is independently -O-, -S-, -S(O)-, -S(O)₂-, -S-S-, 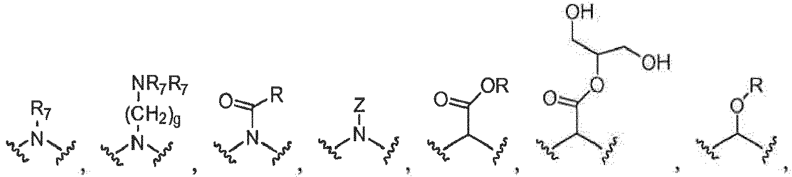 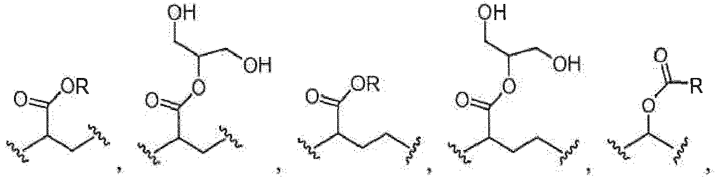 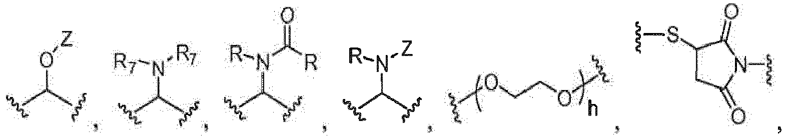 or 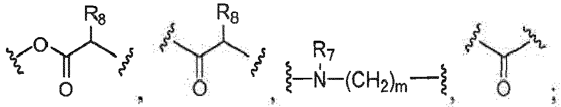 each g is independently 2, 3 or 4;
each h is independently 1, 2, 3 or 4;
m is 0, 1, 2, 3, 4 or 5;
each R₇ is independently H or C₁-C₆ alkyl, or both R₇ groups, when taken together with the nitrogen to which they are attached, can form a heterocycle;
each R₈ is independently e, H or straight or branched C₁-C₁₀ which can be optionally substituted with OH, NH₂, CO₂R, CONH₂, phenyl, C₆H₄OH, imidazole or arginine;
each R is independently H, -C(O)-C₁-C₃ alkyl, or straight or branched C₁-C₄ alkyl optionally substituted with OR, NR₂, or halogen;
each Z is independently H, or 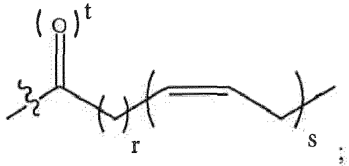 with the proviso that there is at least one 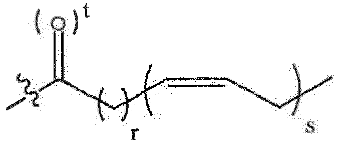 in the compound;
each r is independently 2 or 3;
each s is independently 5 or 6;
each t is independently 0 or 1; and
each e is independently H or any one of the side chains of the naturally occurring amino acids;
provided that
when m, n, o, p, and q are each 0, W₁ and W₂ are each null, and Z is 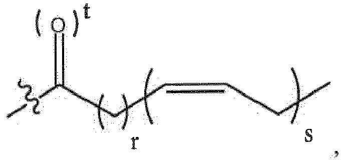 then t must be 0.

In another aspect, compounds of the **Formula Ic:** 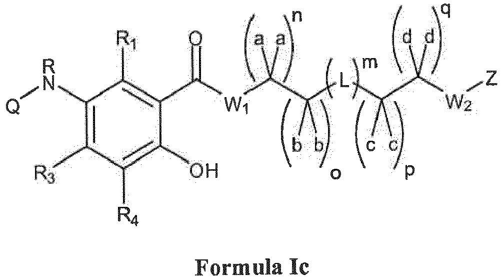 and pharmaceutically acceptable salts, hydrates, solvates, prodrugs, enantiomers, and stereoisomers thereof;
wherein
R₁, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
W₁ and W₂ are each independently null, O, or NH, or when W₁ and W₂ are both NH, then both W₁ and W₂ can be taken together to form a piperidine moiety;
each a and c is independently H, CH₃, -OCH₃, -OCH₂CH₃or C(O)OH, C(O)OR or benzyl;
each b is independently H, CH₃, C(O)OH, O-Z, C(O)OR or benzyl;
each d is independently H, C(O)OH, C(O)OR or benzyl;
each n, o, p, and q is independently 0 or 1;
each L is independently -O-, -S-, -S(O)-, -S(O)₂-, -S-S-, 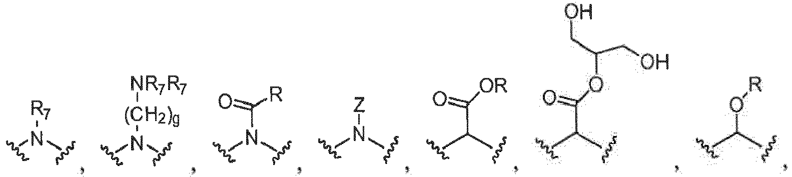 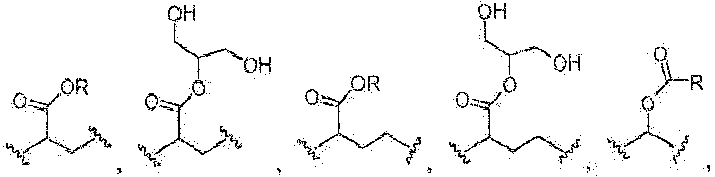 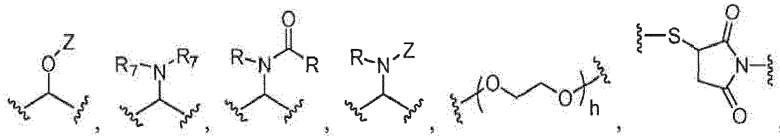 or 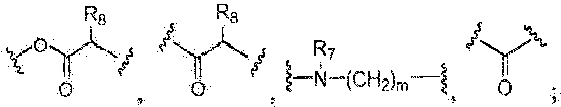 each g is independently 2, 3 or 4;
each h is independently 1, 2, 3 or 4;
m is 0, 1, 2, 3, 4 or 5;
each R₇ is independently H or C₁-C₆ alkyl, or both R₇ groups, when taken together with the nitrogen to which they are attached, can form a heterocycle;
each R₈ is independently e, H or straight or branched C₁-C₁₀ which can be optionally substituted with OH, NH₂, CO₂R, CONH₂, phenyl, C₆H₄OH, imidazole or arginine;
each R is independently H, --C₁-C₃ alkyl, or straight or branched C₁-C₄ alkyl optionally substituted with OH or halogen;
each Z is independently H, or 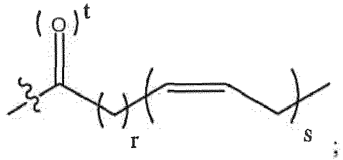 with the proviso that there is at least one 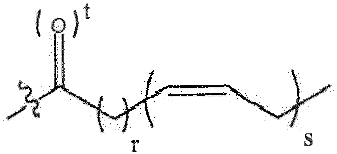 in the compound;
each r is independently 2 or 3;
each s is independently 5 or 6;
each t is independently 0 or 1;
Q is null, H, C(O)CH₃, Z, or 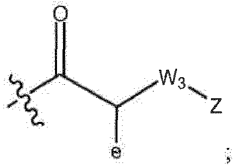 each e is independently H or any one of the side chains of the naturally occurring amino acids;
W₃ is null, -O-, or N(R)-; and
T is H, C(O)CH₃, or Z;
provided that
when m, n, o, p, and q are each 0, W₁ and W₂ are each null, and Z is 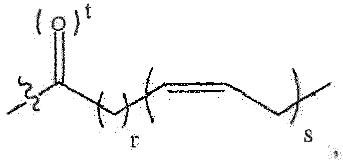 then t must be 0.

Compounds of the **Formula IVa:** 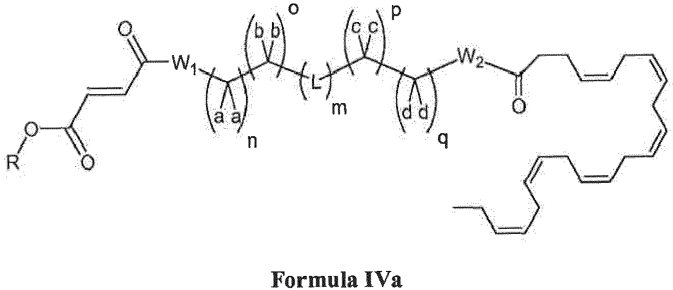 and pharmaceutically acceptable salts, hydrates, solvates, prodrugs, enantiomers, and stereoisomers thereof;
wherein
each W₁ and W₂ is independently null, O, S, NH, or NR, or W₁ and W₂ can be taken together to form an optionally substituted imidazolidine or piperazine group;
each a, b, c, and d, is independently -H, -D, -CH₃, -OCH₃, -OCH₂CH₃, -C(O)OR, or benzyl, or two of a, b, c, and d can be taken together, along with the single carbon to which they are bound, to form a cycloalkyl or heterocycle;
each n, o, p, q, is independently 0, 1 or 2;
each L is independently null, -O-, -C(O)-,-S-, -S(O)-, -S(O)₂-, -S-S-, -(C₁-C₆alkyl)-, -(C₃-C₆cycloalkyl)-, a heterocycle, a heteroaryl, 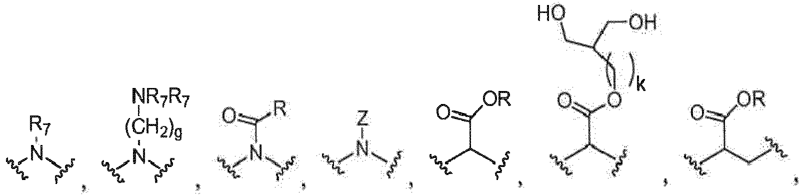 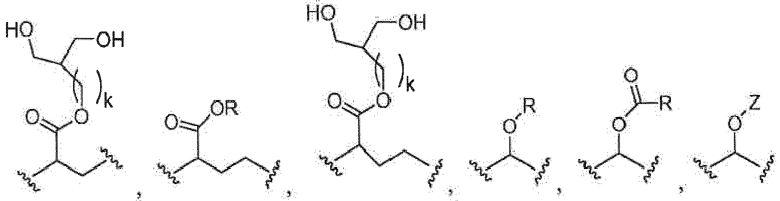 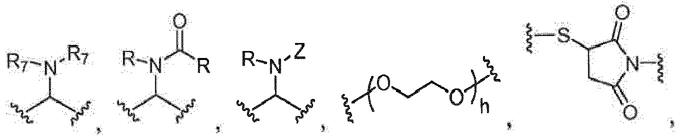 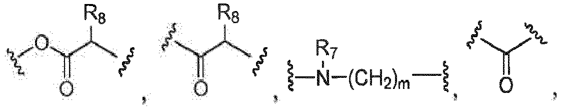 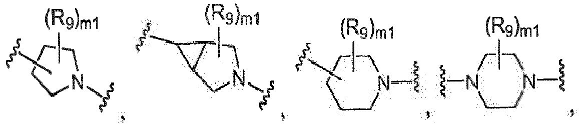 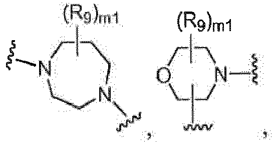 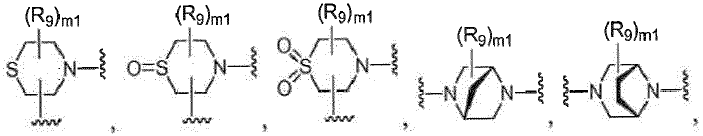 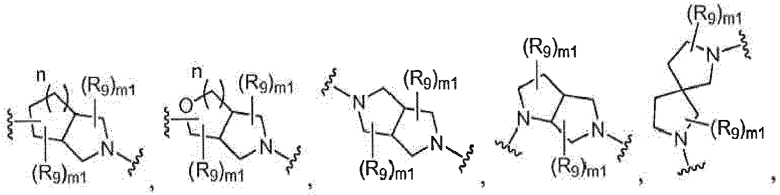 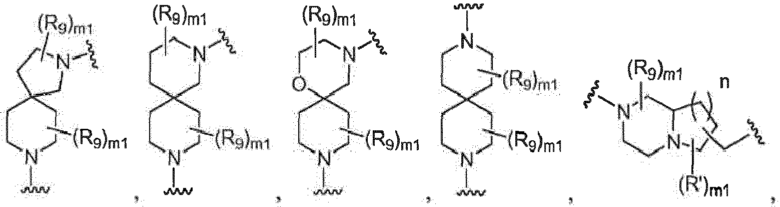 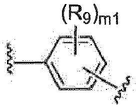 wherein the representation of L is not limited directionally left to right as is depicted, rather either the left side or the right side of L can be bound to the W₁ side of the compound of **Formula IVa;**
each R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3, or 4;
each h is independently 1, 2, 3, or 4;
each m is independently 0, 1, 2, 3, 4 or 5; if m is more than 1, then L can be the same or different;
each m1 is independently 0, 1, 2, or 3;
k is 0, 1, 2, or 3;
z is 1, 2, or 3;
each R₇ is independently H or optionally substituted C₁-C₆ alkyl, wherein a methylene unit of the C₁-C₆ alkyl can be optionally substituted for either O or NR, and in NR₇R₇, both R₇ when taken together with the nitrogen to which they are attached can form a heterocyclic ring such as a pyrrolidine, piperidine, morpholine, piperazine or pyrrole;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each R is independently -H, or straight or branched C₁-C₄ alkyl optionally substituted with OH, or halogen.

Compounds of **Formula IVb:** 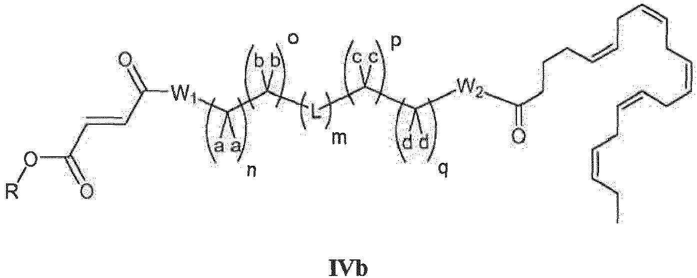 and pharmaceutically acceptable salts, hydrates, solvates, prodrugs, enantiomers, and stereoisomers thereof;
wherein
each W₁ and W₂ is independently null, O, S, NH, or NR, or W₁ and W₂ can be taken together can form an optionally substituted imidazolidine or piperazine group;
each a, b, c, and d, is independently -H, -D, -CH₃, -OCH₃, -OCH₂CH₃, -C(O)OR, or benzyl, or two of a, b, c, and d can be taken together, along with the single carbon to which they are bound, to form a cycloalkyl or heterocycle;
each n, o, p, q, is independently 0, 1, or 2;
each L is independently null, -O-, -C(O)-,-S-, -S(O)-, -S(O)₂-, -S-S-, -(C₁-C₆alkyl)-, -(C₃-C₆cycloalkyl)-, a heterocycle, a heteroaryl, 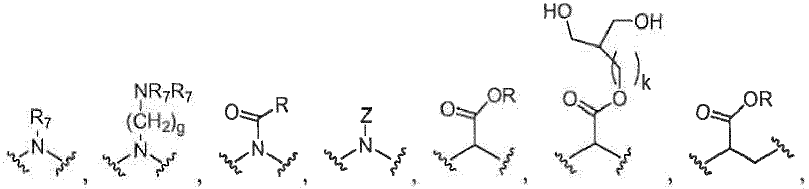 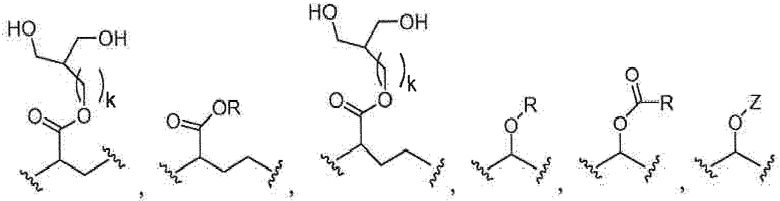 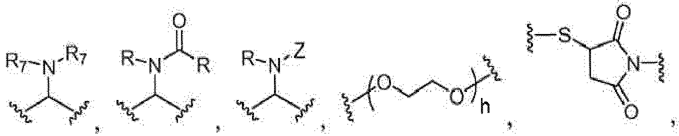 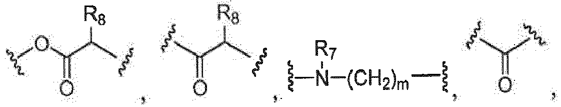 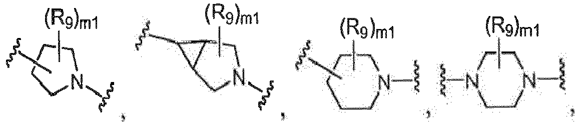 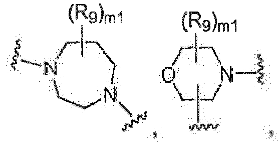 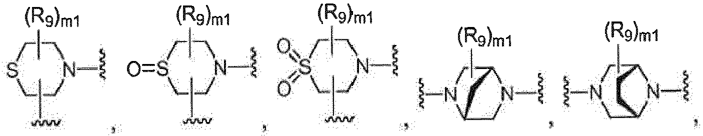 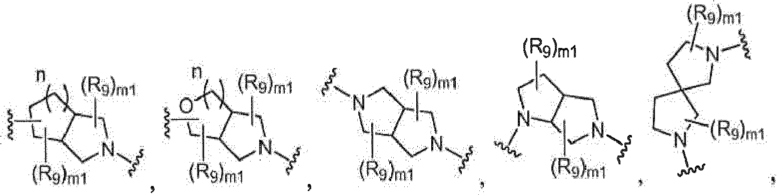 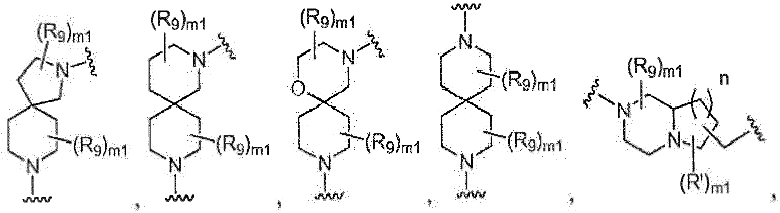 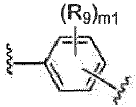 wherein the representation of L is not limited directionally left to right as is depicted, rather either the left side or the right side of L can be bound to the W₁ side of the compound of **Formula IVb;**
each R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3, or 4;
each h is independently 1, 2, 3, or 4;
each m is independently 0, 1,2, or 3; if m is more than 1, then L can be the same or different;
each m1 is independently 0, 1, 2 or 3;
k is 0, 1, 2, or 3;
z is 1, 2, or 3;
each R₇ is independently H or optionally substituted C₁-C₆ alkyl, wherein a methylene unit of the C₁-C₆ alkyl can be optionally substituted for either O or NR, and in NR₇R₇, both R₇ when taken together with the nitrogen to which they are attached can form a heterocyclic ring such as a pyrrolidine, piperidine, morpholine, piperazine or pyrrole;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each R is independently -H, or straight or branched C₁-C₄ alkyl optionally substituted with OH, or halogen.

Compounds of **Formula IVc:** 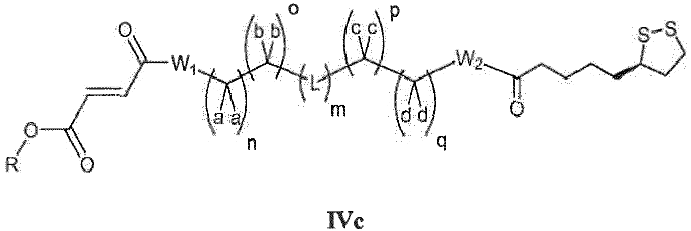 and pharmaceutically acceptable salts, hydrates, solvates, prodrugs, enantiomers, and stereoisomers thereof;
wherein
each W₁ and W₂ is independently null, O, S, NH, or NR, or W₁ and W₂ can be taken together can form an optionally substituted imidazolidine or piperazine group;
each a, b, c, and d, is independently -H, -D, -CH₃, -OCH₃, -OCH₂CH₃, -C(O)OR, or benzyl, or two of a, b, c, and d can be taken together, along with the single carbon to which they are bound, to form a cycloalkyl or heterocycle;
each n, o, p, q, is independently 0, 1, or 2;
each L is independently null, -O-, -C(O)-,-S-, -S(O)-, -S(O)₂-, -S-S-, -(C₁-C₆alkyl)-, -(C₃-C₆cycloalkyl)-, a heterocycle, a heteroaryl, 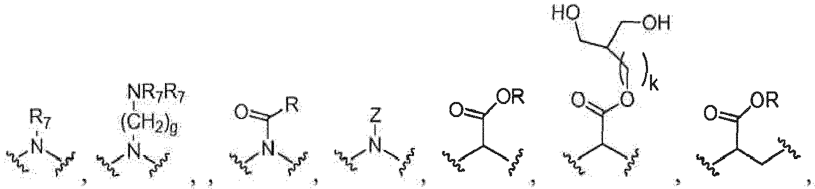 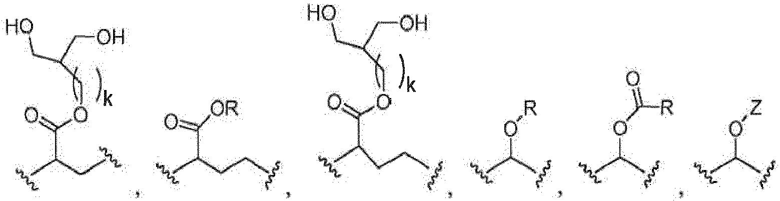 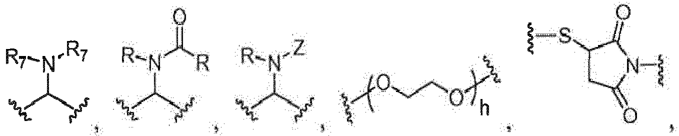 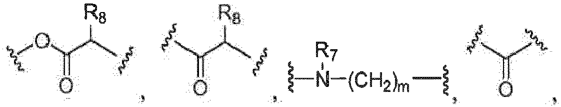 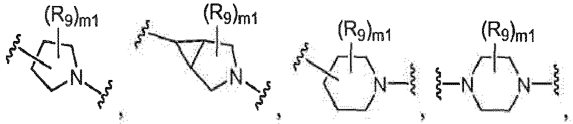 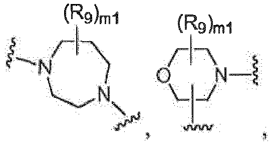 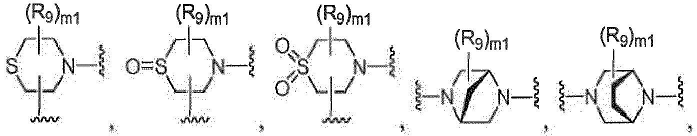 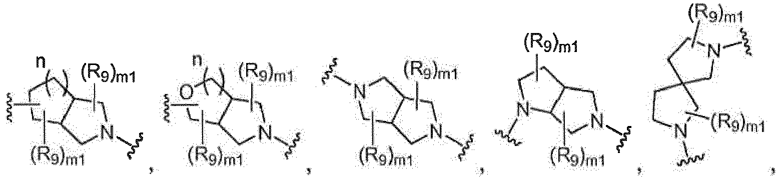 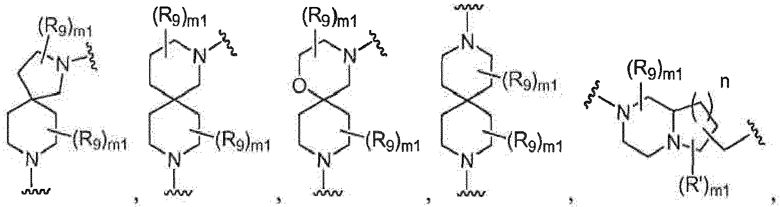 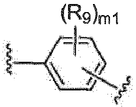 wherein the representation of L is not limited directionally left to right as is depicted, rather either the left side or the right side of L can be bound to the W₁ side of the compound of **Formula IVc;**
each R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3, or 4;
each h is independently 1, 2, 3, or 4;
each m is independently 0, 1, 2, 3, 4 or 5; if m is more than 1, then L can be the same or different;
each m1 is independently 0, 1, 2, or 3;
k is 0, 1, 2, or 3;
z is 1, 2, or 3;
each R₇ is independently H or optionally substituted C₁-C₆ alkyl, wherein a methylene unit of the C₁-C₆ alkyl can be optionally substituted for either O or NR, and in NR₇T₇, both R₄ when taken together with the nitrogen to which they are attached can form a heterocyclic ring such as a pyrrolidine, piperidine, morpholine, piperazine or pyrrole;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each R is independently -H, or straight or branched C₁-C₄ alkyl optionally substituted with OH or halogen.

Compounds of the **Formula VIIa:** 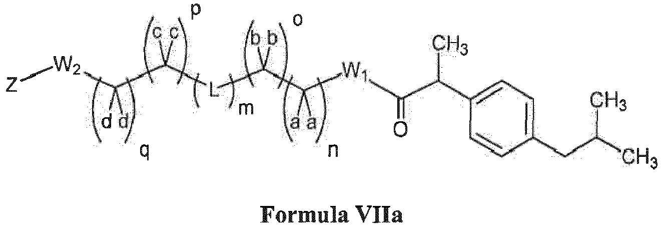 and pharmaceutically acceptable salts, hydrates, solvates, prodrugs, enantiomers, and stereoisomers thereof;
wherein
W₁ and W₂ are each independently null, O, S, NH, NR, or W₁ and W₂ can be taken together can form an imidazolidine or piperazine group, with the proviso that W₁ and W₂ can not be O simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently null, -O-, -S-, -S(O)-, -S(O)₂-, -S-S-, -(C₁-C₆alkyl)-, -(C₃-C₆cycloalkyl)-, a heterocycle, a heteroaryl, 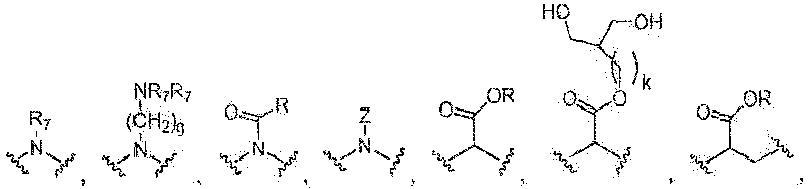 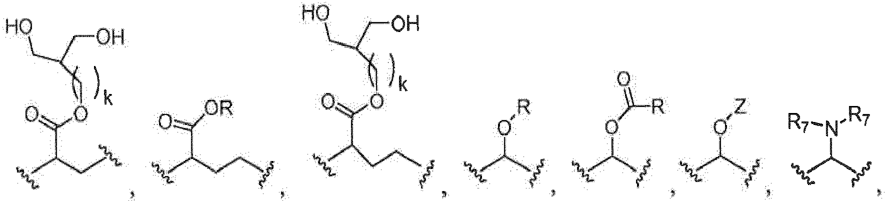 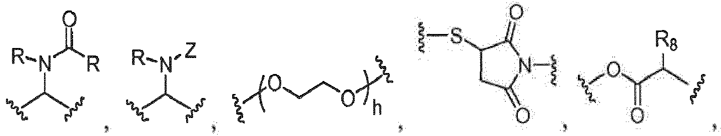 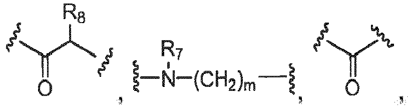 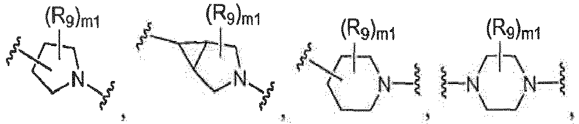 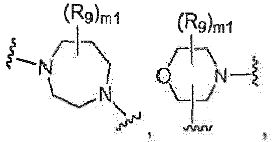 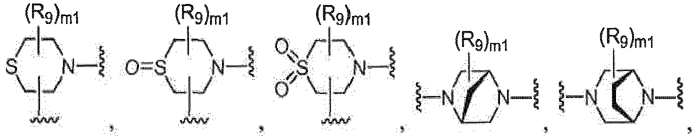 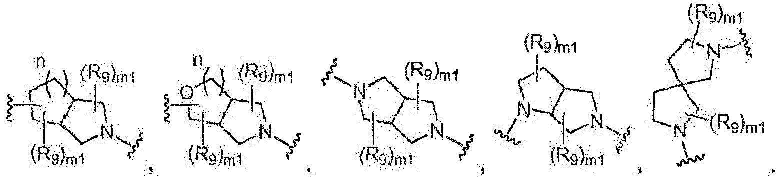 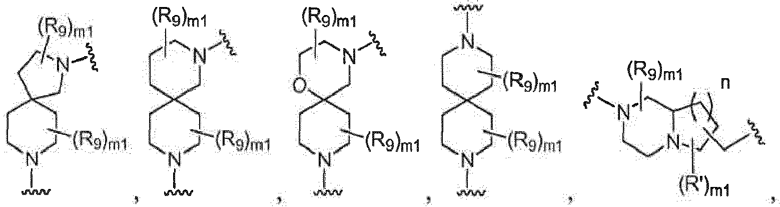 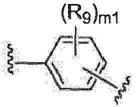 wherein the representation of L is not limited directionally left to right as is depicted, rather either the left side or the right side of L can be bound to the W₁ side of the compound of Formula I;
R₉ is independently -H, -D, -C₁-C₄ alkyl, -halogen, cyano, oxo, thiooxo, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl;
each g is independently 2, 3 or 4;
each h is independently 1, 2, 3 or 4;
m is 0, 1, 2, 3, 4 or 5; if m is more than 1, then L can be the same or different;
m1 is 0, 1, 2 or 3;
k is 0, 1, 2, or 3;
z is 1, 2, or 3;
each R₇ is independently H or C₁-C₆ alkyl that can be optionally substituted with either O or N and in NR₇R₇, both R₇ when taken together with the nitrogen to which they are attached can form a heterocyclic ring such as a pyrrolidine, piperidine, morpholine, piperazine or pyrrole;
each R₈ independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 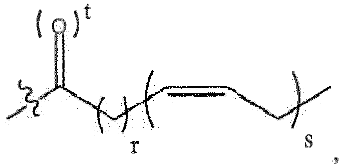 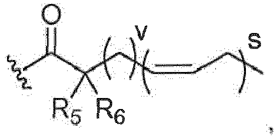 or 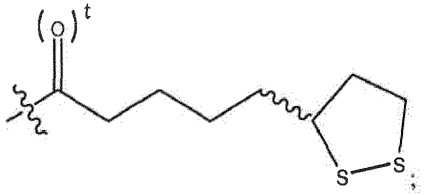 with the proviso that there is at least one 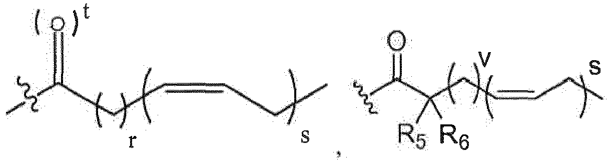 or 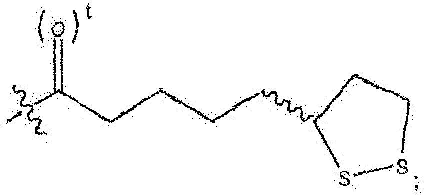 in the compound;
each r is independently 2, 3, or 7;
each s is independently 3, 5, or 6;
each t is independently 0 or 1;
each v is independently 1, 2, or 6;
R₅ and R₆ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, or straight or branched C₁-C₄ alkyl optionally substituted with OH, or halogen;
provided that
when m, n, o, p, and q are each 0, W₁ and W₂ are each null, and Z is 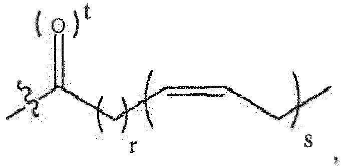 then t must be 0; and
when m, n, o, p, and q are each 0, and W₁ and W₂ are each null, then Z must not be 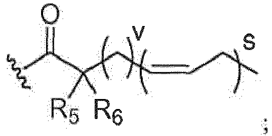 with the further proviso that the compound is not 5Z,8Z,11Z,14Z,17Z)-1-(2-(1-(4-chlorobenzoyl)-5-methoxy-2-methyl-1*H*-indol-3-yl)acetoxy)ethyl icosa-5,8,11,14,17-pentaenoate or 5-((S)-1,2-dithiolan-3-yl)-N-(2-(2-(4-isobutylphenyl)propanamido)ethyl)pentanamide.

Compounds of the **Formula Id:** 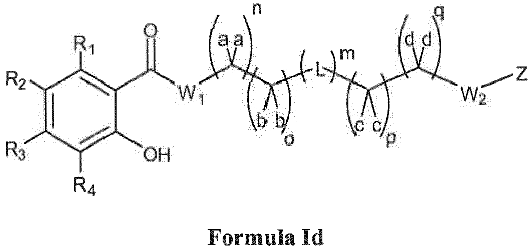 and pharmaceutically acceptable salts, hydrates, solvates, prodrugs, enantiomers, and stereoisomers thereof;
wherein
R₁, R₂, R₃, and R₄ are each independently selected from the group consisting of H, Cl, F, CN, NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl, -S(O)₂C₁-C₃ alkyl, difluorophenyl, and trifluoromethyl;
W₁ and W₂ are each independently null, O, or NH, or when W₁ and W₂ are both NH, then both W₁ and W₂ can be taken together to form a piperidine moiety;
each a and c is independently H, CH₃, -OCH₃, -OCH₂CH₃, C(O)OH, C(O)OR or benzyl;
each b is independently H, CH₃, C(O)OH, O-Z, C(O)OR or benzyl;
each d is independently H, C(O)OH, C(O)OR or benzyl;
each n, o, p, and q is independently 0 or 1;
each L is independently 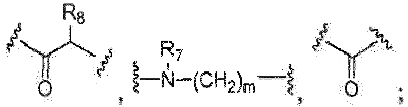 m is 0, 1, 2, 3, 4 or 5;
each R₇ is independently H or C₁-C₆ alkyl, or both R₇ groups, when taken together with the nitrogen to which they are attached, can form a heterocycle;
each Rg is independently e, H or straight or branched C₁-C₁₀ which can be optionally substituted with OH, NH₂, CO₂R, CONH₂, phenyl, C₆H₄OH, imidazole or arginine;
each Z is independently H, or 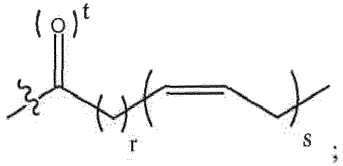 with the proviso that there is at least one 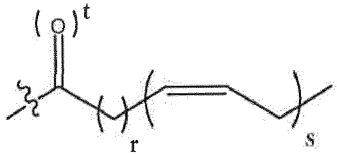 in the compound;
each r is independently 2 or 3;
each s is independently 5 or 6;
each t is independently 0 or 1; and
each e is independently H or any one of the side chains of the naturally occurring amino acids;
provided that
when m, n, o, p, and q are each 0, W₁ and W₂ are each null, and Z is 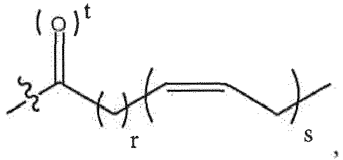 then t must be 0.

Compounds of the **Formula III-a:** 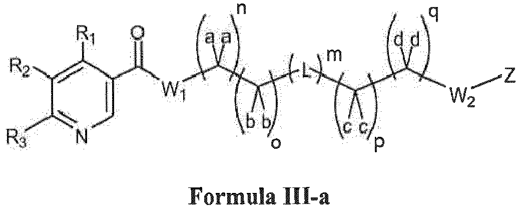 and pharmaceutically acceptable salts, hydrates, solvates, prodrugs, enantiomers and stereoisomers thereof;
wherein
R₁, R₂, and R₃ are each independently selected from the group consisting of -H, -D, -Cl, -F, -CN, -NH₂, -NH(C₁-C₃ alkyl), -N(C₁-C₃ alkyl)₂, -NH(C(O)C₁-C₃ alkyl), -N(C(O)C₁-C₃ alkyl)₂,-C(O)H, -C(O)C₁-C₃ alkyl, -C(O)OC₁-C₃ alkyl, -C(O)NH₂, -C(O)NH(C₁-C₃ alkyl), -C(O)N(C₁-C₃ alkyl)₂, -C₁-C₃ alkyl, -O-C₁-C₃ alkyl, -S(O)C₁-C₃ alkyl and -S(O)₂C₁-C₃ alkyl;
W₁ and W₂ are each independently null, O, S, NH, NR, or W₁ and W₂ can be taken together can form an imidazolidine or piperazine group, with the proviso that W₁ and W₂ can not be 0 simultaneously;
each a, b, c, and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0 or 1;
each L is independently 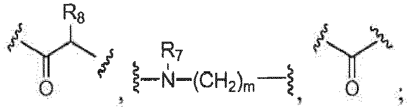 m is 0, 1, 2, 3, 4 or 5;
each R₇ is independently H or C₁-C₆ alkyl, or both R₆ groups, when taken together with the nitrogen to which they are attached, can form a heterocycle;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 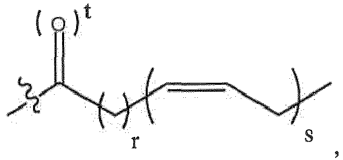 or 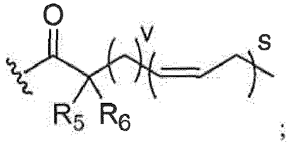 with the proviso that there is at least one 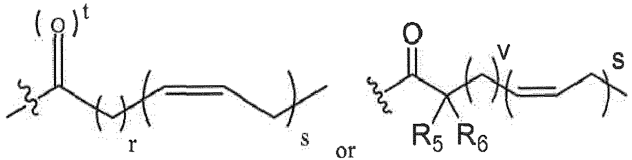 in the compound;
each r is independently 2, 3, or 7;
each s is independently 3, 5, or 6;
each t is independently 0 or 1;
each v is independently 1, 2, or 6;
R₅ and R₆ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
provided that
when m, n, o, p, and q are each 0, W₁ and W₂ are each null, and Z is 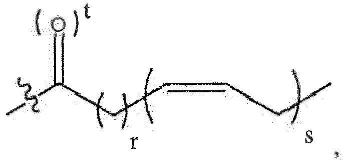 then t must be 0; and
when each of m, n, o, p, and q is 0, and W₁ and W₂ are each null, then Z must not be

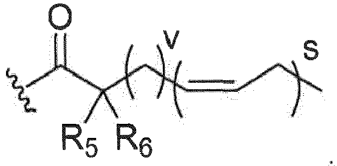

Compounds of the Formula IV-d: 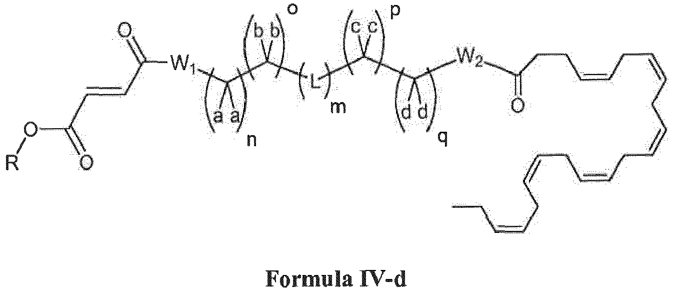 and pharmaceutically acceptable salts, hydrates, solvates, prodrugs, enantiomers, and stereoisomers thereof;
wherein
each W₁ and W₂ is independently null, O, S, NH, or NR, or W₁ and W₂ can be taken together to form an optionally substituted imidazolidine or piperazine group;
each a, b, c, and d, is independently -H, -D, -CH₃, -OCH₃, -OCH₂CH₃, -C(O)OR, or benzyl, or two of a, b, c, and d can be taken together, along with the single carbon to which they are bound, to form a cycloalkyl or heterocycle;
each n, o, p, q, is independently 0, 1 or 2;
each L is independently 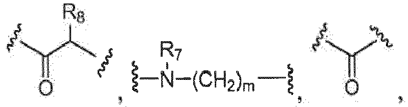 wherein the representation of L is not limited directionally left to right as is depicted, rather either the left side or the right side of L can be bound to the W₁ side of the compound of **Formula IV-d;**
each m is independently 0, 1, 2, 3, 4 or 5; if m is more than 1, then L can be the same or different;
each m1 is independently 0, 1,2, or 3;
each R₇ is independently H or optionally substituted C₁-C₆ alkyl, wherein a methylene unit of the C₁-C₆ alkyl can be optionally substituted for either O or NR, and in NR₇R₇, both R₇ when taken together with the nitrogen to which they are attached can form a heterocyclic ring such as a pyrrolidine, piperidine, morpholine, piperazine or pyrrole;
each R₈ is independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each R is independently -H, or straight or branched C₁-C₄ alkyl optionally substituted with OH, or halogen.

Compounds of the **Formula VII-b:** 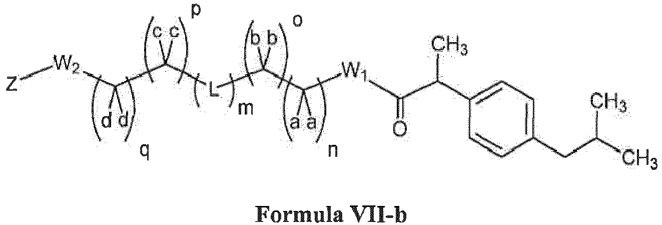 and pharmaceutically acceptable salts, hydrates, solvates, prodrugs, enantiomers, and stereoisomers thereof;
wherein
W₁ and W₂ are each independently null, O, S, NH, NR, or W₁ and W₂ can be taken together can form an imidazolidine or piperazine group, with the proviso that W₁ and W₂ can not be O simultaneously;
each a, b, c and d is independently -H, -D, -CH₃, -OCH₃, -OCH₂CH₃, -C(O)OR, or -O-Z, or benzyl, or two of a, b, c, and d can be taken together, along with the single carbon to which they are bound, to form a cycloalkyl or heterocycle;
each n, o, p, and q is independently 0, 1 or 2;
each L is independently 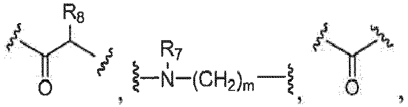 wherein the representation of L is not limited directionally left to right as is depicted, rather either the left side or the right side of L can be bound to the W₁ side of the compound of Formula I;
m is 0, 1, 2, 3, 4 or 5; if m is more than 1, then L can be the same or different;
m1 is 0, 1, 2 or 3;
each R₇ is independently H or C₁-C₆ alkyl that can be optionally substituted with either O or N and in NR₇R₇, both R₇ when taken together with the nitrogen to which they are attached can form a heterocyclic ring such as a pyrrolidine, piperidine, morpholine, piperazine or pyrrole;
each R₈ independently e, H or straight or branched C₁-C₁₀ alkyl which can be optionally substituted with OH, NH₂, CO₂R, CONH₂, phenyl, C₆H₄OH, imidazole or arginine;
each e is independently H or any one of the side chains of the naturally occurring amino acids;
each Z is independently -H, or 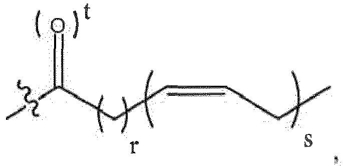 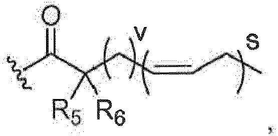 or 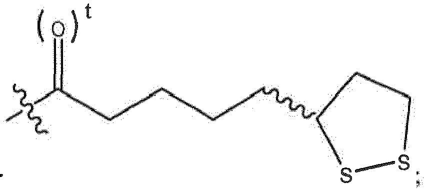 with the proviso that there is at least one 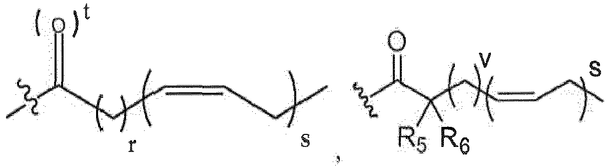 or 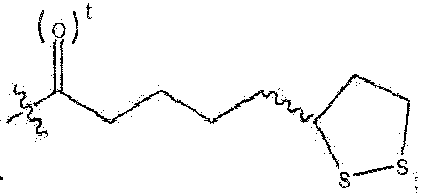 in the compound;
each r is independently 2, 3, or 7;
each s is independently 3, 5, or 6;
each t is independently 0 or 1;
each v is independently 1, 2, or 6;
R₅ and R₆ are independently -H, -D, -C₁-C₄ alkyl, -halogen, -OH, -C(O)C₁-C₄ alkyl, -O-aryl, -O-benzyl, -OC(O)C₁-C₄ alkyl, -C₁-C₃ alkene, -C₁-C₃ alkyne, -C(O)C₁-C₄ alkyl, -NH₂, -NH(C₁-C₃ alkyl), -N(C₁-C₃ alkyl)₂, -NH(C(O)C₁-C₃ alkyl), -N(C(O)C₁-C₃ alkyl)₂, -SH, -S(C₁-C₃ alkyl), -S(O)C₁-C₃ alkyl, -S(O)₂C₁-C₃ alkyl; and
each R is independently -H, -C₁-C₃ alkyl, or straight or branched C₁-C₄ alkyl optionally substituted with OH, or halogen;
provided that
when m, n, o, p, and q are each 0, W₁ and W₂ are each null, and Z is 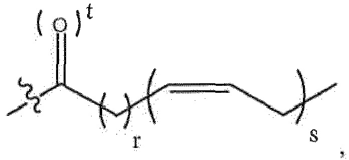 then t must be 0; and
when m, n, o, p, and q are each 0, and W₁ and W₂ are each null, then Z must not be 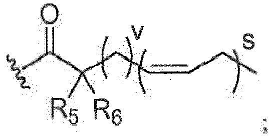 with the further proviso that the compound is not 5Z,8Z,11Z,14Z,17Z)-1-(2-(1-(4-chlorobenzoyl)-5-methoxy-2-methyl-1*H*-indol-3-yl)acetoxy)ethyl icosa-5,8,11,14,17-pentaenoate or 5-((S)-1,2-dithiolan-3-yl)-N-(2-(2-(4-isobutylphenyl)propanamido)ethyl)pentanamide.

Specific illustrative embodiments of **Formulae I, II, III, IV, V, IV, Ia, Ib, Ic, IVa, IVb, IVc** and **VIIa** are described below:

In some embodiments, W₁ and W₂ are each NH, m is 0, n, and o are each 1, and p and q are each 0.

In some embodiments, W₁ and W₂ are each NH, m is 1, n, o, p, and q are each 1, and L is O.

In some embodiments, W₁ and W₂ are each NH, m is 1, n, o, p, and q are each 1, and L is

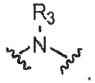

In some embodiments, W₁ and W₂ are each NH, m is 1, n, o, p, and q are each 1, and L is -S-S-.

In some embodiments, W₁ and W₂ are each NH, m is 1, n and o are each 0, p and q are each 1, and L is

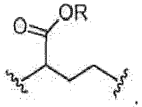

In some embodiments, W₁ and W₂ are each NH, m is 1, k is O, n and o are each 0, p and q are each 1, and L is

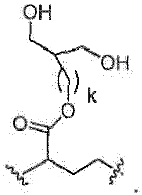

In some embodiments, W₁ and W₂ are each NH, m is 1, n and o are each 1, p and q are each 0, and L is

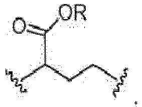

In some embodiments, W₁ and W₂ are each NH, m is 1, k is 0, n is 1, o, p and q are each 0, and L is

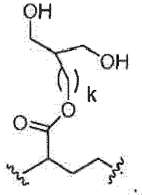

In some embodiments, W₁ and W₂ are each NH, m is 1, n, o, and p are each 0, and q is 1, and L is

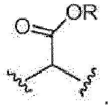

In some embodiments, W₁ and W₂ are each NH, m is 1, k is 1, n, o, and p are each 0, and q is 1, and L is

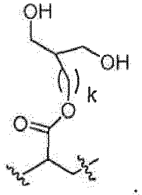

In some embodiments, W₁ and W₂ are each NH, m is 1, n is 1, and o, p, and q are each 0, and L is

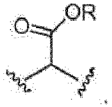

In some embodiments, W₁ and W₂ are each NH, m is 1, k is l, o, p, and q are each 0, and L is

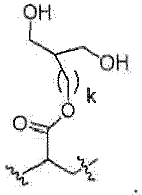

In some embodiments, W₁ and W₂ are each NH, m is 1, n, o, p, and q are each 1, and L is

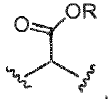

In some embodiments, W₁ and W₂ are each NH, m is 1, n, o, p, and q are each 1, and L is

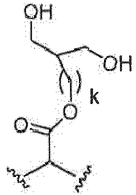

In some embodiments, W₁ and W₂ are each NH, m is 0, k is 1, o and p are each 1, and q is 0.

In some embodiments, W₁ and W₂ are each NH, m is 0, n, o, p, and q are each 1.

In some embodiments, W₁ and W₂ are each NH, m is 0, n and o are each 1, p and q are each 0, and each a is CH₃.

In some embodiments, W₁ and W₂ are each NH, m is 0, n and o are each 1, p and q are each 0, and each b is CH₃.

In some embodiments, W₁ and W₂ are each NH, m is 1, n, o, p, and q are each 1, R₃ is H, and L is

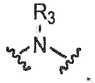

In some embodiments, W₁ and W₂ are each NH, m is 1, n, p and q are each 1, and o is 2, R₄ is H, and L is

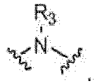

In some embodiments, W₁ and W₂ are each NH, m is 1, n, o, p are each 1, and q is 2, and L is

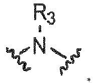

In some embodiments, W₁ and W₂ are each NH, m is 1, n, o, p, and q are each 1, and L is

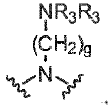

In some embodiments, W₁ and W₂ are each NH, m is 1, n and p are each 1, and o and q are each 0, and L is -C(O)-.

In some embodiments, W₁ and W₂ are each NH, m is 1, n and p are each 1, and o, and q are each 0, and L is

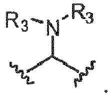

In some embodiments, W₁ and W₂ are each NH, m is 1, n, o, p, q are each 1, and L is

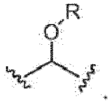

In some embodiments, W₁ and W₂ are each NH, m is 1, n, o, p , and q are each 1, his 1, and L is

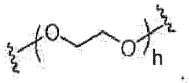

In some embodiments, W₁ and W₂ are each NH, m is 1, n, o, p , and q are each 1, and L is-S-.

In some embodiments, W₁ and W₂ are each NH, m is 1, n, o, p are each 0, q is 1, one d is -CH₃, and L is

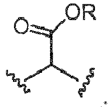

In some embodiments, W₁ and W₂ are each NH, m is 2, n, o, p, and q are each 0, one L is 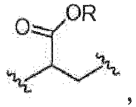 and
one L is

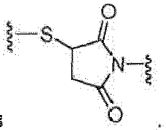

In some embodiments, m is 0, n, o, p, and q are each 0, and W₁ and W₂ are taken together to form an optionally substituted piperazine group.

In some embodiments, m is 1, n, o, p, and q are each 0, W₁ and W₂ are each null, and L is

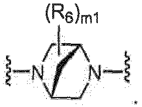

In some embodiments, m is 1, n and p are each 1, o and q are each 0, W₁ and W₂ are each NH, and L is C₃-C₆ cycloalkyl.

In some embodiments, m is 1, n is l, o, p, and q are each 0, W₁ and W₂ are each NH, and L is C₃-C₆ cycloalkyl.

In some embodiments, m is 1, n, o, p, are each 0, q is 1, W₁ and W₂ are each NH, and L is C₃-C₆ cycloalkyl.

In some embodiments, m is 1, n, o, p, and q are each 0, W₁ is NH, W₂ is null, and L is

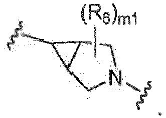

In some embodiments, m is 1, n o, p, and q are each 0, W₁ is null, W₂ is NH, and L is

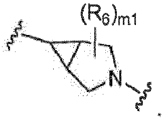

In some embodiments, m is 1, n o, p, and q are each 0, W₁ is NH, W₂ is null, and L is

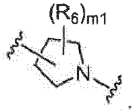

In some embodiments, m is 1, n o, p, and q are each 0, W₁ is null, W₂ is NH, and L is

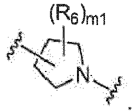

In some embodiments, m is 1, n is l, o, p, and q are each 0, W₁ is NH, W₂ is null, and L is

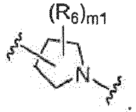

In some embodiments, m is 1, n, o, p, are each 0, q is 1, W₁ is null, W₂ is NH, and L is

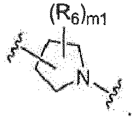

In some embodiments, m is 1, n, o, p, and q are each 0, W₁ is NH, W₂ is null, and L is

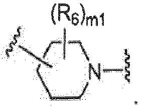

In some embodiments, m is 1, n, o, p, and q are each 0, W₁ is null, W₂ is NH, and L is

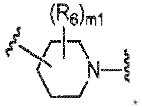

In some embodiments, m is 1, n is 1, o, p, and q are each 0, W₁ is NH, W₂ is null, and L is

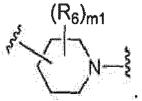

In some embodiments, m is 1, n, o, p, are each 0, q is 1, W₁ is null, W₂ is NH, and L is

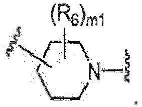

In some embodiments, m is 1, n is l, o, p, and q are each 0, W₁ is NH, W₂ is null, and L is

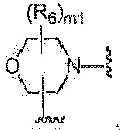

In some embodiments, m is 1, n, o, p, are each 0, q is 1, W₁ is null, W₂ is NH, and L is

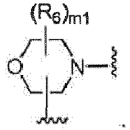

In some embodiments, m is 1, n, o, p, q are each 0, W₁ and W₂ is null, and L is

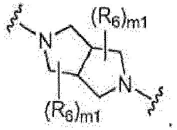

In some embodiments, m is 1, n, o, p, q are each 0, W₁ and W₂ is null, and L is

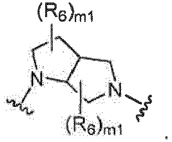

In some embodiments, m is 1, n, o, p, q are each 0, W₁ is NH, W₂ is null, and L is

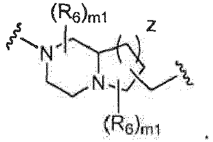

In some embodiments, m is 1, n, o, p, q are each 0, W₁ is null, W₂ is NH, and L is

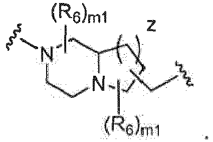

In some embodiments, m is 1, n, o, p, are each 0, q is 1, W₁ and W₂ are each and NH, is null, L is

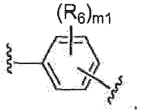

In some embodiments, when W1 and W2 is each NH, and n, o, p, q, are each 0 and m is 3, then at least two of the L groups have to be

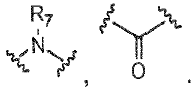

In some embodiments, when W1 and W2 is each NH, and n, o, p, q, are each 0 and m is 4, then at least two of the L groups have to be

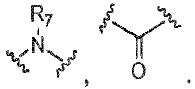

In some embodiments, when W1 and W2 is each NH, and n, o, p, q, are each 0 and m is 5, then at least two of the L groups have to be 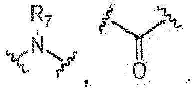 In some embodiments, when W1 and W2 is each NH, m is 2, and n, o, p, q are each 0, then L is 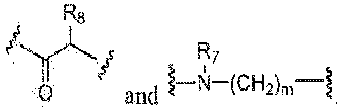 wherein R₇ is H.

In other illustrative embodiments, linked bioactives of **Formula I, II, III, IV, V, VI, Ia, Ib, Ic, Iva, IVb, IVc,** and **VIIa** are as set forth below:
N-(2-(4Z,7Z,10Z,13Z, 16Z, 19Z)-docosa-4,7,10,13,16,19-hexaenamidoethyl)-2-hydroxybenzamide **(I-1);**
2-hydroxy-N-(2-((4Z,7Z,10Z,13Z, 16Z, 19Z)-N-methyldocosa-4,7,10,13,16,19-hexaenamido)ethyl)benzamide **(I-2);** N-(1-(4Z,7Z,10Z,13Z, 16Z,19Z)-docosa-4,7,10,13,16,19-hexaenamidopropan-2-yl)-2-hydroxybenzamide **(I-3);**
N-(2-(4Z,7Z,10Z,13Z,16Z,19Z)-docosa-4,7,10,13,16,19-hexaenamidopropyl)-2-hydroxybenzamide **(1-4);**
N-(3-(4Z,7Z,10Z,13Z,16Z,19Z)-docosa-4,7,10,13,16,19-hexaenamidobutan-2-yl)-2-hydroxybenzamide **(I-5);**
ethyl 2-(2-(4Z,7Z, 10Z,13Z,16Z,19Z)-docosa-4,7,10,13,16,19-hexaenamido-3-phenylpropanoyloxy)benzoate **(I-6);**
2',4'-Difluoro-4-hydroxy-biphenyl-3-carboxylic acid [2-((4Z,7Z,10Z,13Z,16Z,19Z)-docosa-4,7,10,13,16,19-hexaenoylamino)-ethyl]-amide **(1-7);**
2',4'-Difluoro-4-hydroxy-biphenyl-3-carboxylic acid [2-((5Z,8Z,11Z,14Z,17Z)-icosa-5,8,11,14,17-pentaenoylamino)-ethyl]-amide **(I-8);**
N-(2-(2-(2-(4Z,7Z, 10Z,13Z,16Z,19Z)-docosa-4,7,10,13,16,19-hexaenamidoethyl)disulfanyl)ethyl)-2-hydroxybenzamide **(I-9);**
2-hydroxy-N-(2-(5Z,8Z,11Z,14Z,17Z)-icosa-5,8,11,14,17-pentaenamidoethyl)benzamide **(I-10);**
N-(1-(4Z,7Z,10Z,13Z, 16Z,19Z)-docosa-4,7,10,13,16,19-hexaenamido-2-methylpropan-2-yl)-2-hydroxybenzamide **(I-11);**
(R)-3-[(R)-2-((4Z,7Z,10Z,13Z,16Z,19Z)-Docosa-4,7,10,13,16,19-hexaenoylamino)-2-methoxycarbonyl-ethyldisulfanyl]-2-(2-hydroxy-benzoylamino)-propionic acid methyl ester **(I-12);**
(S)-6-((4Z,7Z,10Z,13Z,16Z,19Z)-docosa-4,7,10,13,16,19-hexaenamido)-2-(2-hydroxybenzamido)hexanoic acid **(1-13);**
N-(2-((2-(4Z,7Z, 10Z13Z, 16Z,19Z)-docosa-4,7,10,13,16,19-hexaenamidoethyl)(methyl)amino)ethyl)-2-hydroxybenzamide **(1-15);**
N-((S)-1-((2-((4Z,7Z,10Z,13Z,16Z,19Z)-docosa-4,7,10,13,16,19-hexaenamido)ethyl)amino)-1-oxopropan-2-yl)-2-hydroxybenzamide **(1-16);** N-((S)-1-((2-((4Z,7Z,10Z,13Z,16Z,19Z)-docosa-4,7,10,13,16,19-hexaenamido)ethyl)amino)-4-methyl-1-oxopentan-2-yl)-2-hydroxybenzamide **(I-17);**
N-(2-((S)-2-((4Z,7Z,10Z,13Z,16Z,19Z)-docosa-4,7,10,13,16,19-hexaenamido)propanamido)ethyl)-2-hydroxybenzamide **(I-18);**
N-(2-((S)-2-((4Z,7Z,10Z,13Z,16Z,19Z)-docosa-4,7,10,13,16,19-hexaenamido)-5-guanidinopentanamido)ethyl)-2-hydroxybenzamide **(I-19);**
N-(2-((S)-5-guanidino-2-((5Z,8Z,11Z,14Z,17Z)-icosa-5,8,11,14,17-pentaenamido)pentanamido)ethyl)-2-hydroxybenzamide **(I-20);**
N-((S)-1-((2-((4Z,7Z,10Z,13Z,16Z,19Z)-docosa-4,7,10,13,16,19-hexaenamido)ethyl)amino)-5-guanidino-1-oxopentan-2-yl)-2-hydroxybenzamide (I-21);
N-((S)-5-guanidino-1-((2-((5Z,8Z,11Z,14Z,17Z)-icosa-5,8,11,14,17-pentaenamido)ethyl)amino)-1-oxopentan-2-yl)-2-hydroxybenzamide **(I-22);**
5-(4Z,7Z,10Z,13Z,16Z,19Z)-docosa-4,7,10,13,16,19-hexaenamido-2-hydroxybenzoic acid **(II-1);**
5-(2-(4Z,7Z,10Z,13Z,16Z,19Z)-docosa-4,7,10,13,16,19-hexaenamido-4-methylpentanamido)-2-hydroxybenzoic acid **(II-2);**
methyl 5-(4Z,7Z,10Z,13Z,16Z,19Z)-docosa-4,7,10,13,16,19-hexaenamido-2-hydroxybenzoate **(II-4);**
methyl 5-((2-(4Z,7Z,10Z,13Z,16Z,19Z)-docosa-4,7,10,13,16,19-hexaenamidoethoxy)carbonyl)-2-hydroxybenzoate **(II-5);**
N-(2-(5Z,8Z,11Z,14Z,17Z)-icosa-5,8,11,14,17-pentacnamidoethyl)nicotinamide **(III-1);**
N-(2-(4Z,7Z,10Z,13Z,16Z,19Z)-docosa-4,7,10,13,16,19-hexaenamidoethyl)nicotinamide **(III-2);**
(S)-6-((4Z,7Z,10Z,13Z,16Z,19Z)-docosa-4,7,10,13,16,19-hexaenamido)-2-(nicotinamido)hexanoic acid **(III-3);**
N-(2-(4Z,7Z,10Z,13Z, 16Z,19Z)-docosa-4,7,10,13,16,19-hexaenamido-2-methylpropyl)nicotinamide **(III-4);** N-(4-(5Z,8Z,11Z,14Z,17Z)-icosa-5,8,11,14,17-pentaenamidobutyl)nicotinamide **(III-5);**
N-(1-(5Z,8Z,11Z,14Z,17Z)-icosa-5,8,11,14,17-pentaenamido-2-methylpropan-2-yl)nicotinamide **(III-6);**
N-(2-(4Z,7Z,10Z,13Z,16Z,19Z)-docosa-4,7,10,13,16,19-hexaenamidoethyl)-N-methylnicotinamide **(III-7);**
N-(1-(5Z,8Z,11Z,14Z,17Z)-icosa-5,8,11,14,17-pentaenamido-2-methylpropan-2-yl)nicotinamide **(III-8);**
N-(2-((4Z,7Z, 10Z,13Z,16Z,19Z)-N-methyldocosa-4,7,10,13,16,19-hexaenamido)ethyl)nicotinamide **(III-9);**
(5Z,8Z,11Z,14Z,17Z)-1-(4-nicotinoylpiperazin-1-yl)icosa-5,8,11,14,17-pentaen-1-one **(III-10);**
N-((S)-1-((2-((5Z,8Z,11Z,14Z,17Z)-icosa-5,8,11,14,17-pentaenamido)ethyl)amino)-1-oxopropan-2-yl)nicotinamide **(III-11);**
N-(2-((S)-2-((4Z,7Z,10Z,13Z,16Z,19Z)-docosa-4,7,10,13,16,19-hexaenamido)propanamido)ethyl)nicotinamide **(III-12);**
(E)-methyl 4-(2-(4Z,7Z,10Z,13Z,16Z,19Z)-docosa-4,7,10,13,16,19-hexaenamidoethylamino)-4-oxobut-2-enoate **(IV-1);**
(E)-methyl 4-(4-(4Z,7Z,10Z,13Z,16Z,19Z)-docosa-4,7,10,13,16,19-hexaenoylpiperazin-1-yl)-4-oxobut-2-enoate **(IV-2);**
(E)-methyl 4-(2-((2-(4Z,7Z,10Z,13Z,16Z,19Z)-docosa-4,7,10,13,16,19-hexaenamidoethyl)(methyl)amino)ethylamino)-4-oxobut-2-enoate **(IV-3);**
(E)-methyl 4-(((S)-1-((2-((4Z,7Z,10Z,13Z,16Z,19Z)-docosa-4,7,10,13,16,19-hexaenamido)ethyl)amino)-1-oxopropan-2-yl)amino)-4-oxobut-2-enoate **(IV-4);**
(E)-methyl 4-((2-((S)-2-((4Z,7Z,10Z,13Z, 16Z,19Z)-docosa-4,7,10,13,16,19-hexaenamido)propanamido)ethyl)amino)-4-oxobut-2-enoate **(IV-5);**
(4Z,7Z,10Z,13Z,16Z,19Z)-1-[4-((5Z,8Z,11Z,14Z,17Z)-icosa-5,8,11,14,17-pentaenoyl)-piperazin-1-yl]-docosa-4,7,10,13,16,19-hexaen-1-one **(VI-1);**
(2S,3R)-2-((4Z,7Z,10Z,13Z, 16Z,19Z)-docosa-4,7,10,13,16,19-hexaenoylamino)-3-[(S)-2-((4Z,7Z,10Z,13Z, 16Z,19Z)-docosa-4,7,10,13,16,19-hexaenoylamino)-propionyloxy]-butyric acid methyl ester **(VI-2);**
(S)-2-((4Z,7Z, 10Z,13Z, 16Z,19Z)-docosa-4,7,10,13,16,19-hexaenoylamino)-5-((5Z,8Z,11Z,14Z,17Z)-icosa-5,8,11,14,17-pentaenoylamino)-pentanoic acid **(VI-3);**
(4Z,7Z, 10Z,13Z,16Z,19Z)-docosa-4,7,10,13,16,19-hexaenoic acid [2-((R)-5-[1,2]dithiolan-3-yl-pentanoylamino)-ethyl]-amide **(VI-4);**
(4Z,7Z, 10Z,13Z, 16Z,19Z)-docosa-4,7,10,13,16,19-hexaenoic acid [2-((5Z,8Z,11Z,14Z,17Z)-icosa-5,8,11,14,17-pentaenoylamino)-ethyl]-amide **(VI-5);**
(4Z,7Z,10Z,13Z, 16Z,19Z)-docosa-4,7,10,13,16,19-hexaenoic acid [2-((4Z,7Z,10Z,13Z, 16Z,19Z)-docosa-4,7,10,13,16,19-hexaenoylamino)-ethyl]-amide **(VI-6);**
(4Z,7Z,10Z,13Z,16Z,19Z)-docosa-4,7,10,13,16,19-hexaenoic acid {2-[2-((5Z,8Z,11Z,14Z,17Z)-icosa-5,8,11,14,17-pentaenoylamino)-ethyldisulfanyl]-ethyl}-amide **(VI-7);**
(4Z,7Z, 10Z,13Z, 16Z, 19Z)-N-(2-(2-(4-isobutylphenyl)propanamido)ethyl)docosa-4,7,10,13,16,19-hexaenamide **(VIIa-1);**
(2S)-2-((4Z,7Z,10Z,13Z,16Z,19Z)-docosa-4,7,10,13,16,19-hexaenamido)-6-(2-(4-isobutylphenyl)propanamido)hexanoic acid **(VIIa-2);**
(2S)-6-((4Z,7Z,10Z,13Z, 16Z,19Z)-docosa-4,7,10,13,16,19-hexaenamido)-2-(2-(4-isobutylphenyl)propanamido)hexanoic acid **(VIIa-3);**
(4Z,7Z,10Z,13Z, 16Z, 19Z)-N-(2-((2-(2-(4-isobutylphenyl)propanamido)ethyl)(methyl)amino)ethyl)docosa-4,7,10,13,16,19-hexaenamide **(VIIa-4);**
(4Z,7Z,10Z,13Z, 16Z, 19Z)-N-(2-((2S)-2-(2-(4-isobutylphenyl)propanamido)propanamido)ethyl)docosa-4,7,10,13,16,19-hexaenamide **(VIIa-5);** and
(4Z,7Z,10Z,13Z, 16Z,19Z)-N-((2S)-1-((2-(2-(4-isobutylphenyl)propanamido)ethyl)amino)-1-oxopropan-2-yl)docosa-4,7,10,13,16,19-hexaenamide **(VIIa-6).**

### EXAMPLES

### Example 1: Sprague Dawley Rat Brain Homogenate Promoted Cleavage of a Linked Bioactive.

The purpose of this assay was to measure the ability of Sprague Dawley rat brain homogenate to hydrolyze a linked bioactive in the presence of fatty acid amide hydrolase inhibitor URB597 (obtained from Cayman Chemical, cat # 10046. Brains were harvested from male Sprague Dawley rats and were homogenized 1:4 (grams:mL) in 20 mM 4-(2-hydroxyethyl)-1-piperazineethanesulfonic acid, pH 7.0. Total protein in the rat brain homogenate was determined by a Bradford protein assay.

Linked bioactive was dissolved in methanol at 50 mM. Hydrolysis reactions were conducted with 500 µM of compound in a reaction buffer of 50 mM Hepes, 1 mM EDTA, 0.1 % Triton X100, pH 8.0, and 1 mg/mL total protein from rat brain homogenate. Reactions were incubated at 37°C. The total reaction volume was 500 µL. Reactions were quenched by transferring 30 µL of reaction into 200 µL of acetonitrile containing 100 ng/mL of internal standard compound, after 0 hours, 1 hour, 2 hours, and 3 hours. Reactions were run in the absence and presence of 5 µM of the FAAH inhibitor URB597

Generally, levels of parent linked bioactive and hydrolysis products were measured using an Agilent 1260 HPLC and an Agilent 6410 triple quadrapole mass spectrometer with an electrospray source and using multiple reaction monitoring and relevant mass/charge transitions. Gradients of methanol and water were chosen with respect to specific parent linked bioactives and relative hydrolysis products. Injection volumes and flow rates were determined for each specific parent linked bioactive and relative hydrolysis products.

Specifically, for the linked bioactive, 20 µL of sample was injected into the HPLC with the mobile phase flow rate set to 0.9 mL/minute. The following gradient of methanol and water was used:

| Time | % Methanol | % Water |
|---|---|---|
| 0.01 | 70 | 30 |
| 0.5 | 70 | 30 |
| 1 | 95 | 5 |
| 4 | 95 | 5 |
| 4.01 | 70 | 30 |
| 4.7 | 70 | 30 |

The gas temperature on the mass spectrometer was set to 350°C. The capillary voltage was set to 4000. The following mass/charge transitions were used for the detection of Metabolite 1 (enzymatic hydrolysis product).

| Compound Name | Precursor Ion | Product Ion | Fragmentor Voltage | Collision Energy Voltage |
|---|---|---|---|---|
| Internal Standard | 515.4 | 205.1 | 135 | 30 |
| Metabolite 1 | 179.1 | 93.1 | 102 | 21 |

Metabolite 1 (enzymatic hydrolysis product) peaks were integrated using Agilent Masshunter Qualitative Analysis software. The integrated values were normalized to an internal standard data generating a mass spectrometer relative ratio value (MS R.R). These values were plotted vs. time using GraphPad Prism.

| **Time (hours)** | **Metabolite 1 Mass Spec R.R. - URB597** | **Metabolite 1 Mass Spec R.R. +URBS97 (5 *µ*M)** |
|---|---|---|
| **0** | **0** | 0 |
| **1** | **0.14** | 0 |
| **2** | **0.28** | 0 |
| **3** | **0.43** | 0 |

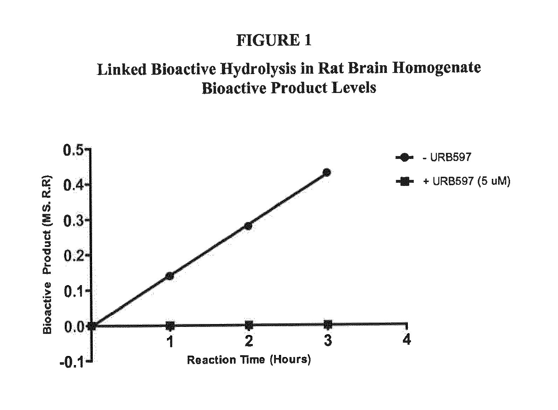

The results of this study, as shown in Figure 1, demonstrate that rat brain homogenate caused hydrolysis of the linked bioactive, which effect was essentially eliminated in the presence of the FAAH inhibitor URB597.

### Example 2: The Therapeutic Effect with Linked Bioactives is Synergistic.

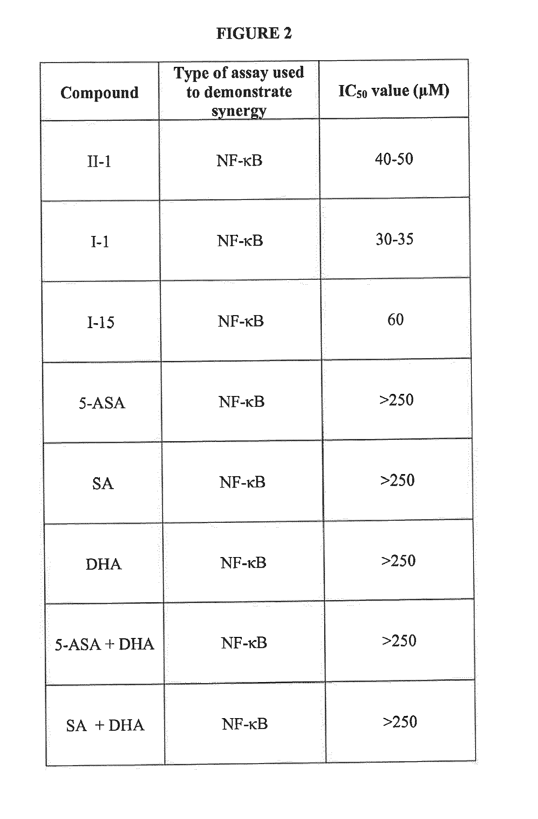

Figures 2-6 exemplify the activity of parent linked bioactives compared to their corresponding hydrolysis products when dosed as separate bioactives.

Selected experiments in Figures 2-6 show the effects of some linked bioactives of the invention on NFκB levels in RAW 264.7 macrophages.

RAW 264.7 cells transfected with an NFκB-driven luciferase reporter were plated in 96 well plates. Cells were treated with Vehicle (0.1% ethanol) or test compounds for 2 hours. As a positive control for inhibition of NFκB signaling, 6 wells were treated with 10 µM dexamethasone. Cells were then challenged with 200 ng/mL LPS for 3 hours in the presence of test compounds. A subset of wells treated with vehicle remained unstimulated with LPS to determine the floor signal of the assay. NFκB driven luciferase activity was developed by addition of BriteLite luciferase kit (PERKIN ELMER®) and measured using a Victor V plate reader. NFκB activity (luciferase activity) for each treatment was normalized to Vehicle wells treated with LPS (% NFκB Response). AlamarBlue was used to monitor cell viability to ensure that inhibition of luciferase signal was not a result of compound cytotoxicity.

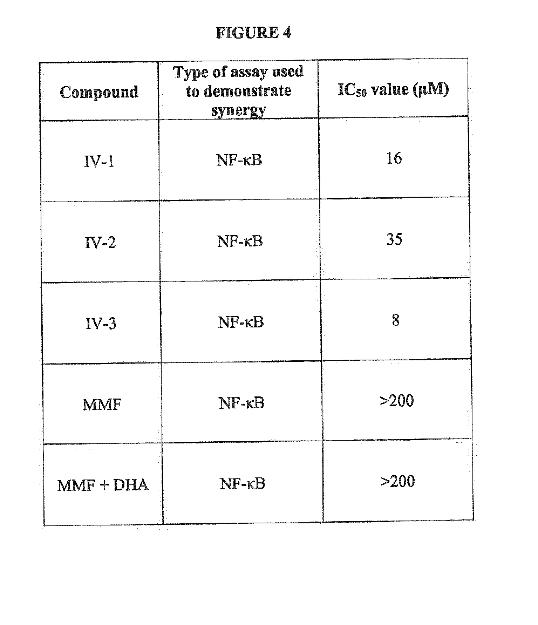

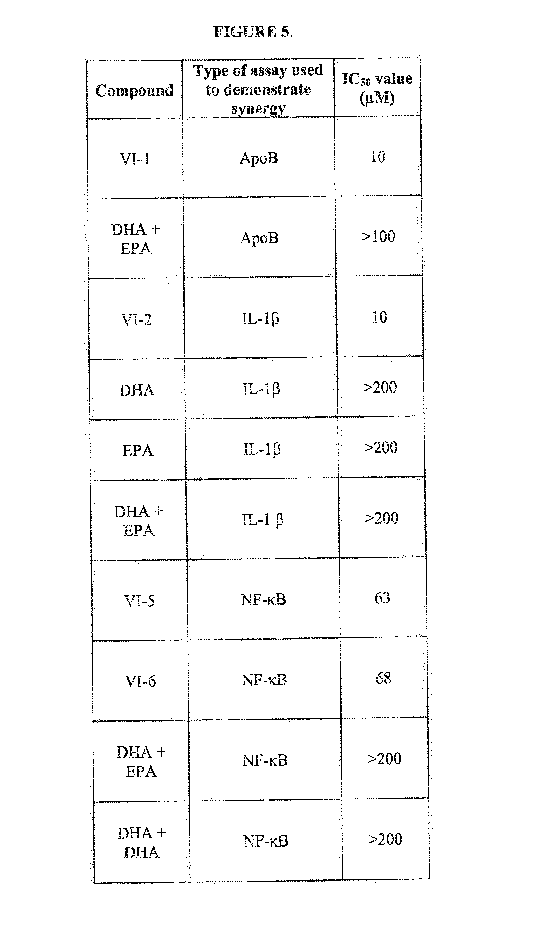

Examples of the synergy demonstrated by the NFκB assay are shown in Figures 2, 4, and 5. In Figure 2, **Compound II-1** is 5-aminosalicylate linked to an omega-3 fatty acid, DHA and demonstrates an IC₅₀ of 40-50 µM in an NFkB assay. However, when 5-aminosalicylate and DHA are used, either separately or together but without linkage, no activity is observed in this assay up to a concentration of 250 µM. Similarly, **Compound 1-1** is salicylic acid linked to DHA and demonstrates an IC₅₀ of 30-35 µM in an NFkB assay. However, when salicylate and DHA are used, either separately or together but without linkage, no activity is observed in this assay up to a concentration of 250 µM. In Figure 4, **Compound IV-1** is mono-methyl fumarate linked to DHA and demonstrates an IC₅₀ of 16 µM in an NFkB assay. However, when mono-methyl fumarate and DHA are used, either separately or together but without linkage, no activity is observed in this assay up to a concentration of 200 µM. In Figure 5, **Compound VI-5** is EPA linked to DHA and demonstrates an IC₅₀ of 63 µM in an NFkB assay. However, when EPA and DHA are used, either separately or together but without linkage, no activity is observed in this assay up to a concentration of 200 µM. **Compound VI-6** is two DHA molecules linked together and demonstrates an IC₅₀ of 68 µM in an NFkB assay. However, when DHA is used, either separately or with a stoichiometry of 2 but without linkage, no activity is observed in this assay up to a concentration of 200 µM.

Selected experiments in Figures 2-6 show the effect of linked bioactives on ApoB secretion in HepG2 cells.

HepG2 cells (ATCC) were seeded at 10,000 cells per well in 96 well plates. After adhering overnight, growth media (10% FBS in DMEM) was removed and cells were serum starved for 24 hours in DMEM containing 0.1% fatty acid free bovine serum albumin (BSA, SIGMA®). Cells were then treated with the compounds. Niacin at 5 mM was used as a positive control. All treatments were performed in triplicate. Simultaneous with compound treatment, ApoB secretion was stimulated with the addition of 0.1 oleate complexed to fatty acid free BSA in a 5:1 molar ratio. Incubation with compounds and oleate was conducted for 24 hours. Media supernatants were removed and ApoB concentrations were measured using ELISA kits (MABTECH AB®). Percent inhibition of ApoB secretion was determined by normalizing data to vehicle treated wells. For a given compound, an IC₅₀ (concentration at which 50% of ApoB secretion is inhibited) can also be determined by using a 4 parameter-fit inhibition curve model (GRAPH PAD PRISM®). In each experiment, cell viability was determined using the ATPlite 1-Step kit (PERKIN ELMER®), such that compound effects due to cytotoxicity could be monitored.

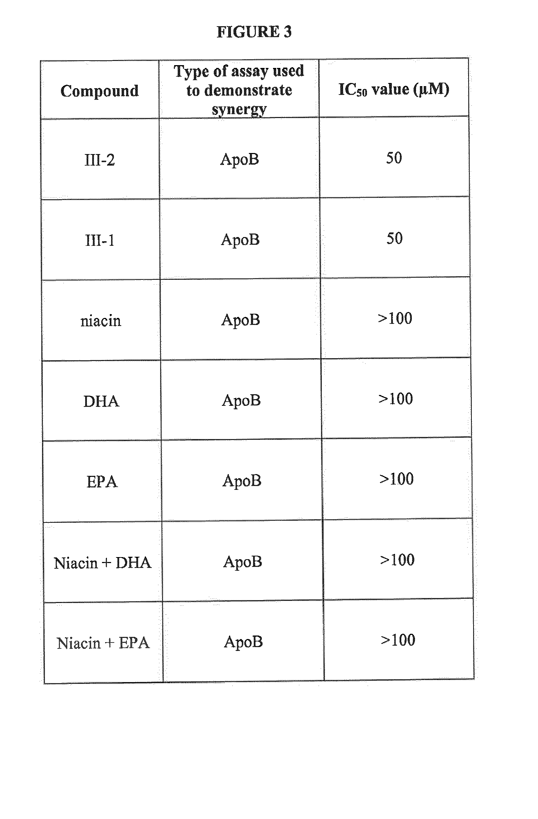

Examples of the synergy demonstrated by the ApoB assay are shown in Figures 3 and 5. In Figure 3, **Compound III-2** is nicotinic acid linked to DHA and demonstrates an IC₅₀ of 50 µM in an ApoB assay. However, when nicotinic acid and DHA are used, either separately or together but without linkage, no activity is observed in this assay up to a concentration of 100 µM. Similarly, **Compound III-1** is nicotinic acid linked to EPA and demonstrates an IC₅₀ of 50 µM in an ApoB assay. However, when nicotinic acid and EPA are used, either separately or together but without linkage, no activity is observed in this assay up to a concentration of 100 µM. In Figure 5, **Compound VI-1** is DHA linked to EPA and demonstrates an IC₅₀ of 10 µM in an ApoB assay. However, when EPA and DHA are used, either separately or together but without linkage, no activity is observed in this assay up to a concentration of 100 µM.

Selected experiments in Figures 2-6 show the effect of linked bioactives on IL-1β.

RAW264.7 macrophages were seeded at a density of 100,000 cells/well in a 96-well plate in DMEM supplemented with 10% FBS and penicillin:streptomycin. 16 hours later, medium was aspirated and replaced with 90µL/well of serum-free DMEM. Linked bioactives were brought up in 100% ethanol to a concentration of 100mM and then diluted 1:100 in 100% FBS for a stock solution consisting of 1mM compound and 1% ethanol. These stock solutions were then diluted 1:10 in FBS supplemented with 1% ethanol to generate a 100 µM of the linked bioactives. 10µL was then added to the RAW246.7 cells to generate final concentrations 10µM of the linked bioactives or 10µM each bioactive, along with vehicle only control. The linked bioactives were allowed to pre-incubate for 2 hours before stimulation of 100 ng/ml LPS (10µL of 1µg/ml LPS was added to each well). Following 3 hours of LPS stimulation, cells were washed once in 1x PBS, aspirated dry, and flash frozen in liquid nitrogen. RNA was then isolated and converted to cDNA using the Cells to cDNA kit (AMBION®) according to the manufacturer's protocol. IL-1β transcript levels were then measured using Taqman primer/probe assay sets (APPLIED BIOSYSTEMS®), normalized to GAPDH using the deltaCt method, and the data expressed relative to vehicle only control.

Examples of the synergy demonstrated by this assay are shown in Figure 5. **Compound VI-2** is EPA linked to DHA and demonstrates an IC₅₀ of 10 µM in an IL-1β assay. However, when EPA and DHA are used, either separately or together but without linkage, no activity is observed in this assay up to a concentration of 200 µM.

Selected experiments in Figures 2-6 show the effect of linked bioactives on LTB₄.

The LTB₄ assay was conducted using a commercial kit (CISBIO, Bedford, MA) in HL-60 cells. HL-60 cells were maintained in IMEM supplemented with 20% serum in 37° C in 5% CO₂. Media was changed 2 times a week and cells kept at a density of 2x10⁵ to 1x10⁶ cells/ml.

Before the experiment cell culture media was changed to 10% serum and HL-60 cells were differentiated for 3 days in 1.3% DMSO. On day 3 cells were concentrated 2 times in the DMSO containing media and 1 ml cell suspension/well plated in 24-well plates. BSA controls or CAT compounds prepared in 1% BSA from an ethanol or DMSO stock solution were added to the wells and the cell were incubated for 21 hours. Next day, cells were transferred to Eppendorf tubes centrifuged at 300xg and rinsed once in DPBS. Cells were re-suspended in 300 µl DPBS containing 2% FBS (assay buffer), and 80 µl/well was plated in 96-well plates (black sides, clear bottom). Some cells were pre-incubated with 10 µM NDGA, a LOX inhibitor. To initiated LTB₄ secretion, cells were stimulated with the calcium ionophore A23187 (5 µM) for 15 min. Plates were then immediately placed on ice and centrifuged at 1500xg for 3 min at 4° C. LTB₄ content in the supernatant was assessed by a homogeneous time resolved fluorescence (HTRF) LTB₄ assay from CISBIO, in a 384-well plate format. LTB₄ standards were diluted in the assay buffer. Cell viability was determined in the 96-well plate by CellTiter-Glo (Promega, Madison, WI).

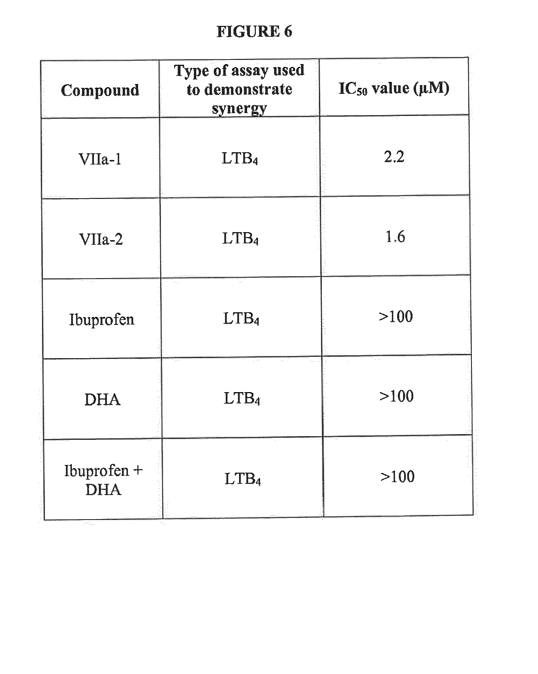

Examples of the synergy demonstrated by this LTB₄ assay are shown in Figure 6. Both compounds **VIIa-1** and **VIIa-2** are ibuprofen linked to DHA and demonstrate an IC₅₀ of 2.2 µM and 1.6 µM respectively in this LTB₄ assay. However, when ibuprofen and DHA are used, either separately or together but without linkage, no activity is observed in this assay up to a concentration of 100 µM.

### Example 3: FAAH and Thiol Reductase Promoted Cleavage of a Linked Bioactive.

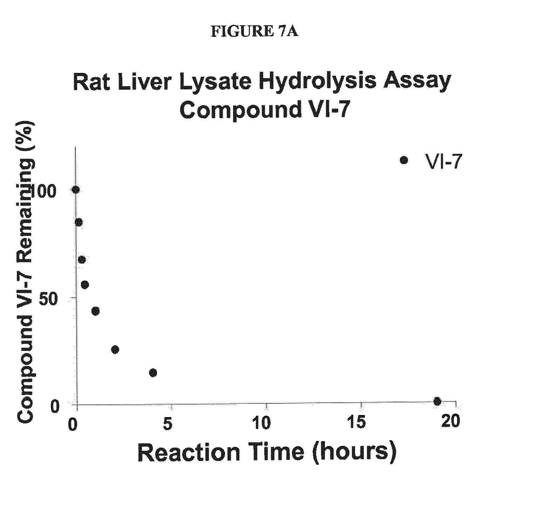

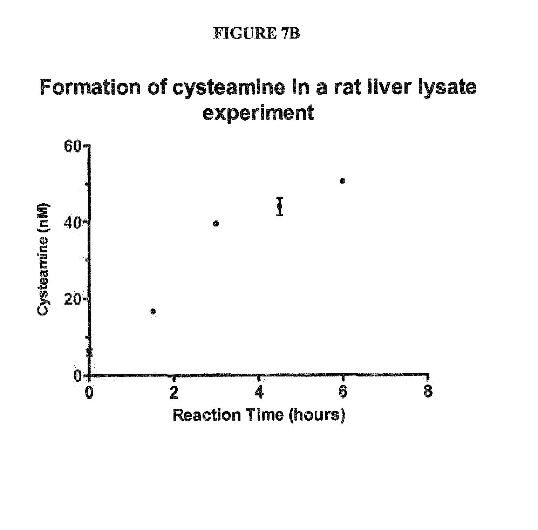

An example that demonstrates the utility of thiol reductase in releasing the linked bioactives is shown in Figure 7A and 7B. The bis-fatty acid linked bioactive employed in this example was (4Z,7Z,10Z,13Z,16Z,19Z)-docosa-4,7,10,13,16,19-hexaenoic acid {2-[2-((5Z,8Z,11Z,14Z,17Z)-icosa-5,8,11,14,17-pentaenoylamino)-ethyldisulfanyl]-ethyl}-amide (VI-7). As discussed below, once inside cells, the disulfide bond in compound VI-7 could be reduced to the corresponding thiol via the action of lysosomal thiol reductase to eventually release cysteamine. Cysteamine, in turn, can be used to treat nephropathic cystinosis, an orphan disease characterized by an excessive accumulation of crystalline cystine inside cells.

The rat liver lysate hydrolysis experiment was performed in Eppendorf tubes, by adding the necessary amount of water for the assay first. A 10x buffer solution with final concentrations of HEPES and EDTA of 50 mM and 1 mM respectively was prepared from 500 mM HEPES and 10 mM EDTA in water. Rat liver lysate was removed from -80°C storage, thawed and centrifuged at 4000g for 5 min. A volume of the supernatant was transferred to the Eppendorf tube yielding a final concentration in the assay of 3 mg/mL rat liver lysate. The tube was gently inverted to mix the components. The reactions are then place in a 37°C incubator for 20 min allowing the reaction mixture to reach 37°C before the reaction was initiated.

To initiate the hydrolysis reaction, the samples were removed from the incubator and the compounds of the invention were added, as a DMSO solution, prepared in a manner to allow for <1% final DMSO concentration and 5 µM final compound concentration. The contents were mixed and then for time point = 0, immediately transferred a volume of the reaction into acetonitrile crash buffer for analysis on the LC/MS/MS in the linear range of the analyte of interest. This process was then repeated for additional time points that were needed (see corresponding Figures 7A and 7B for the different time points that were used)

This rat liver lysate hydrolysis assay was used to assess the hydrolysis of bis-fatty acid linked bioactives into individual components. As an illustrative example, the bis-fatty acid cystamine linked bioactive **VI-7** was assessed in this rat liver lysate assay. For compound **VI-7,** the individual components are DHA, EPA, (4Z,7Z,10Z,13Z,16Z,19Z)-N-(2-mercaptoethyl)docosa-4,7,10,13,16,19-hexaenamide, (5Z,8Z,11Z,14Z,17Z)-N-(2-mercaptoethyl)icosa-5,8,11,14,17-pentaenamide, cystamine and cysteamine. Inside cells, the disulfide bond of **VI-7** can be reduced to the active thiol derivatives (4Z,7Z,10Z,13Z,16Z,19Z)-N-(2-mercaptoethyl)docosa-4,7,10,13,16,19-hexaenamide and (5Z,8Z,11Z,14Z,17Z)-N-(2-mercaptoethyl)icosa-5,8,11,14,17-pentaenamide via the action of lysosomal thiol reductase. Cysteamine would be obtained by further hydrolysis of the amide moieties. The intracellular release of cysteamine would benefit patients with nephropathic cystinosis because cysteamine forms mixed disulfide species with the excess cystine present inside cells suffering from nephropathic cystinosis, thereby removing harmful crystalline cystine. As shown in Figure 7A, compound **VI-7** showed a time-dependent hydrolysis in rat liver lysate. Figure 7B further shows the time dependent formation of cysteamine from compound **VI-7** in this rat liver lysate assay in the absence of the FAAH inhibitor PF-3845. Figure 7B also shows that in the presence of the FAAH inhibitor PF-3845, there is essentially no increase in cysteamine since the hydrolysis of the fatty acid portion is inhibited. The amount of cysteamine that was formed could be quantitated by LC-MS/MS by carrying out an initial derivatization with Ellman's reagent. A saturated solution of Ellmans reagent (10mM) was prepared first by dissolving 39.6mg of reagent (5,5' dithio-bis(2-nitrobenzoic acid)) in 10ml of HEPES buffer (0.01M, pH7). 50 µl of the tissue lysate reaction mixture was transferred to a 1.5 mL eppendorf tube and derivatization of cysteamine was performed with 50 µl 10mM Ellman's reagent (5,5' dithio-bis(2-nitrobenzoic acid). Samples were incubated at room temperature for 10 minutes, vortex for 1 minute followed by protein precipitation by addition of 300 µl of acetonitrile containing IS and 0.2% butylated hydroxyanisole (BHA). Supernatant was transferred to a clean LC vial and 10 µl of the sample was injected on LC-MS/MS for the measurement of cysteamine. The concentration of cysteamine was measured by LC-MS/MS. Derivatized samples (10µl) were injected into the column (Luna, HILIC, 150X4.60mm, 3µ Phenomenex). Cysteamine-Ellmans complex was eluted from the column by a stepwise gradient of 10%mobile phase B at 0 min, 10% B at 2.5 min, 50% B at 2.7 min and 10% B at 3 min. Mobile phase A contained Acetonitrile/Water/Ammonium acetate (20mM) with 0.1% Formic acid and mobile phase B contained Acetonitrile/Ammonium acetate (20mM) with 0.1% formic acid. The column eluate was directly injected into a Agilent triple quad, which was maintained in electrospray positive mode. The retention times for cysteamine-Ellmans adduct was 1.76 min. Derivatized cysteamine was monitored via the transition m/z 274.8-229.9 with a fragmentor of 62 and collision energy of 10eV. The column was maintained at 25°C.

### Example 4: Monoacylglycerol Lipase Promoted Cleavage of a Linked Bioactive.

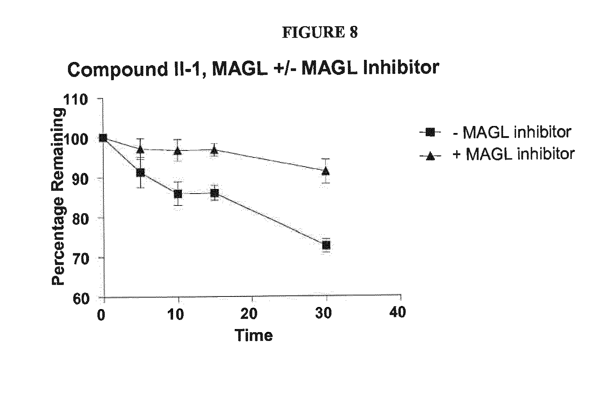

Hydrolysis experiment involving Monoacylglycerol lipase (MAGL) with and without a MAGL inhibitor. A commercially available Monoacylglycerol (MAGL) kit from Cayman (Cat # 705192) was used for the hydrolysis experiment. The hydrolysis was carried out in Eppendorf tubes by adding together 300 µL of a 10 mM Tris-HCl buffer (pH 7.2, 1 mM EDTA), 20 µL of DMSO, and 20 µL of the human recombinant MAG-lipase. Compound **II-1** was added as the last component, 20 µL of a 900 µM solution in DMSO in order to make a 5 µM final concentration. The Eppendorf tubes were gently inverted to mix the components and then placed in a 37°C incubator. A 25 µL sample was withdrawn at each of the following time points: 0, 5, 10, 15, and 30 min. Each sample was analyzed for the remaining amount of compound **II-1** by LC/MS/MS methods using the appropriate acetonitrile crash and internal standard. In a separate experiment, the hydrolysis was then repeated in the presence of a MAGL inhibitor (compound JZL184, supplied with the commercially available MAGL kit). Eppendorf tubes were charged with 300 µL of a 10 mM Tris-HCl buffer (pH 7.2, 1 mM EDTA), 20 µL of a 900 µM solution of JZL184 in DMSO in order to make a 5 µM final concentration, and 20 µL of the human recombinant MAG-lipase. Compound **II-1** was added as the last component, 20 µL of a 900 µM solution in DMSO in order to make a 5 µM final concentration. The Eppendorf tubes were gently inverted to mix the components and then placed in a 37°C incubator. A 25 µL sample was withdrawn at the same time points indicated above and analyzed by LC/MS/MS for the remaining amount of compound **II-1.** As shown in Figure 8, a time-dependent hydrolysis of compound **II-1** was observed using human recombinant MAG-lipase. This rate of hydrolysis of compound **II-1** was reduced significantly in the presence of the MAGL inhibitor JZL184.

### Example 5: Fatty Acid Amide Hydrolases Promoted Cleavage of a Linked Bioactive in COS-7 cells that have been Overexpressed with either FAAH-1 or FAAH-2.

Hydrolysis experiments using COS-7 cells overexpressed with either FAAH-1 or FAAH-2. In order to obtain COS-7 cells that have been overexpressed with either FAAH-1 or FAAH-2, the following protocols were employed: 1.75 x 10⁶ COS-7 cells/plate were seeded onto 10 cm dishes. The next day, cells were transfected, in duplicate, with 10 µg Vector control, flag-FAAH-1 or flag-FAAH-2 plasmids using Lipofectamine 2000 (Invitrogen) according to the manufacturer's protocol. 24 hours post transfection, cells were washed 1 x in cold PBS, aspirated dry, and then scraped off the plate in the presence of 0.6 mL reaction buffer (50 mM HEPES, pH 9.0; 1 mM EDTA). Cells were then homogenated using a VWR VDI 12 Adaptable Homogenizer, on ice for 1 minute, and the sample was then clarified by centrifugation at 6,000 g for 5 minutes. The supernatant of this homogenated fraction was then used for FAAH hydrolysis assays. In order to assess the amount of FAAH protein present in the homogenate fraction, the pellet was then successively lysed with EBC, then RIPA, and then 10% SDS, and the supernatant fraction for the respective buffer lysis condition was analyzed along with the homogenate fraction by western blot, probing with anti-FLAG antibody (Cell Signaling # 2368). Gel densitometry was performed and the %FAAH extracted in each successive lysis conditions was determined according to standard procedures.

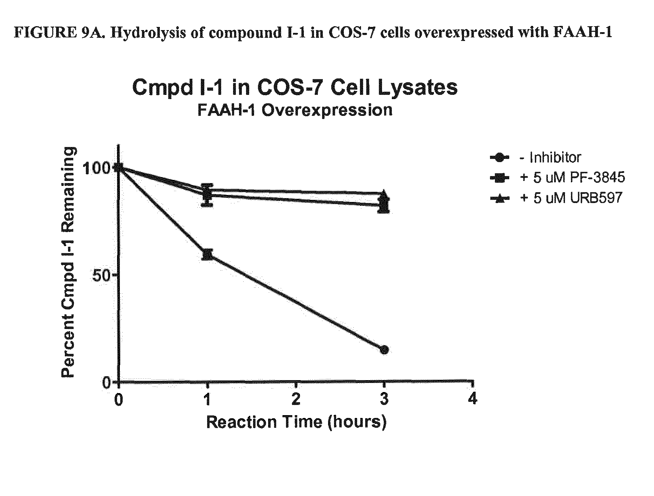

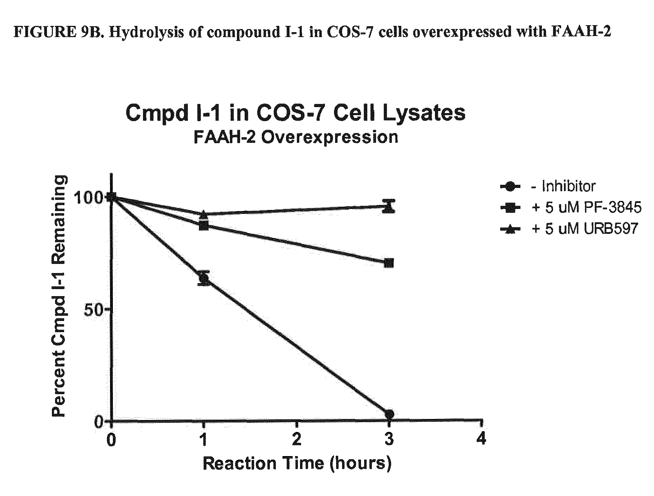

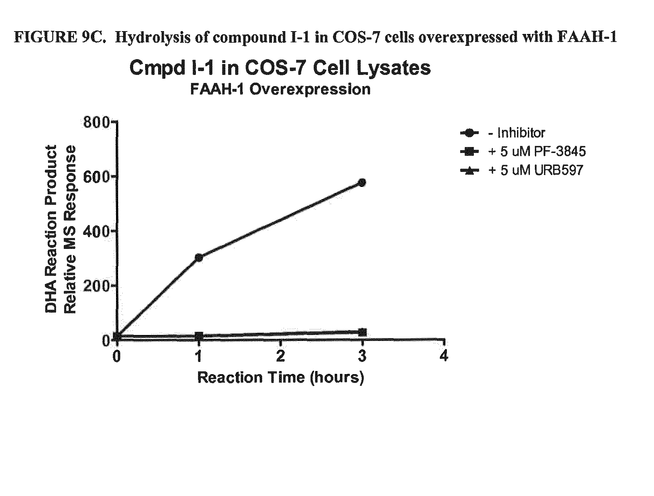

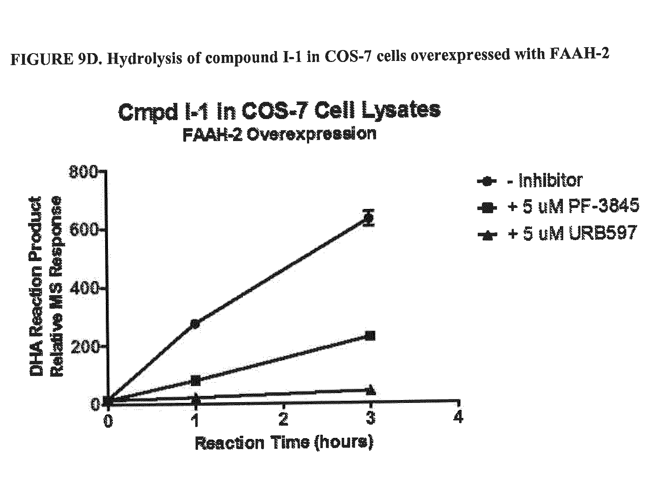

The following hydrolysis experiments were performed using COS-7 cells containing overexpressed FAAH-1 or FAAH-2. Two inhibitors were used: the FAAH-1 specific inhibitor PF-3845 and the FAAH-1/FAAH-2 inhibitor URB597. Compound 1-1 was used and either the disappearance of compound **I-1** was observed or the appearance of DHA in the COS-7 cell lysates was observed. For COS-7 cells containing overexpressed FAAH-1, the protein concentration was 1.03 mg/mL. For COS-7 cells containing overexpressed FAAH-2, the protein concentration was 1.25 mg/mL. Briefly, the hydrolysis experiments were carried out in Eppendorf tubes using procedures similar to the one described above. Briefly, the hydrolysis experiments were carried out in Eppendorf tubes. The hydrolysis was carried out at 37 °C using 50 mM of Hepes buffer (pH 9.0), 1 mM EDTA, compound 1-1 to make a final concentration of 5 µM, and with either PF-3845 or URB597 to make a final concentration of 5 µM. At time = 0, 1, and 3 h, the protein was precipitated with acetonitrile. The samples were centrifuged and the supernatant analyzed by LC/MS/MS for either compound **I-1** or DHA, using the appropriate internal standard. Figure 9A summarizes the hydrolysis of compound **I-1** in COS-7 cells that were overexpressed with FAAH-1. In the absence of any inhibitor, significant hydrolysis of compound **I-1** was observed during the duration of the experiment. This rate of hydrolysis could be significantly reduced by the addition of 5 µM either PF-3845 or URB597 since both compounds are known as FAAH-1 inhibitors. Figure 9B summarizes the hydrolysis of compound 1-1 in COS-7 cells overexpressed FAAH-2. In the presence of the FAAH-1/FAAH-2 inhibitor URB597, this hydrolysis process was significantly reduced. The FAAH-1 inhibitor PF-3845 was not as effective as URB597. Figure 9C summarizes the same hydrolysis of compound 1-1 in COS-7 cells that were overexpressed with FAAH-1; however, in this experiment, the appearance of DHA was monitored instead of the disappearance of **I-1.** As seen in Figure 9C, there was a time dependent formation of DHA; and this rate of formation was significantly inhibited with either PF-3845 or URB597. Figure 9D summarizes the same experiment using COS-7 cells that were overexpressed with FAAH-2. Here, the FAAH-1/FAAH-2 inhibitor URB597 was more effective in reducing the formation of DHA during the duration of the experiment.

### Example 6: N-acylethanolamine-hydrolyzing Acid Amidase Promoted Cleavage of a Linked Bioactive.

Hydrolysis experiment using recombinant NAAA, with and without NAAA inhibitor. Briefly, the reaction is prepared in Eppendorf tubes, adding the calculated amount of water for the assay first. Then, using a 10x buffer comprised of 500 mM Hepes and 10 mM EDTA in water, a volume of this 10X buffer is added to the eppendorf tube allowing for a 10x dilution of the 10x buffer and final concentrations of HEPES and EDTA of 50 mM and 1 mM respectively. DTT and NPO-40 are added to reaction resulting in concentrations of 1 mM and 0.1 % respectively. NAAA enzyme (prepared according to procedures reported in Ueda et al, Progress in Lipid Research 2010, 49, p. 299-315) is removed from -80°C storage, and is thawed on ice. A volume of enzyme is transferred to the Eppendorf yielding a final concentration in the assay of 10 nM. The tube is then gently inverted to mix components. Following mixing, a NAAA inhibitor is added to the reaction mixture to form a final concentration of 5 µM, 1% DMSO max (this set of samples is referred to as + NAAA inhibitor). A volume of DMSO equal to the volume of NAAA inhibitor added to the +NAAA inhibitor samples is added to the - NAAA inhibitor to serve as a vehicle control. The following NAAA inhibitor can be used for this type of hydrolysis experiment: (S)-N-(2-oxooxetan-3-yl)-3-phenylpropanamide (as reported in Solorzano et al, J. Med. Chem. 2010, 53, p. 5770-5781). All reactions are then placed in a 37 °C incubator for 20 minutes allowing the reaction mixture to reach 37 °C before the reaction is initiated. To initiate the hydrolysis, all samples are removed from the incubator and the desired compound, 5 µM final concentration, is added to all tubes. After mixing and then for time point = 0, a volume of the reaction is immediately transferred into a crash buffer. The volume transferred and the volume of crash used will depend on the mass spec sensitivity of the specific analyte being tested. After crashing, the sample is centrifuged at 14,800g for 5 minutes. A portion of the supernant (100 µL) is transferred to a spring loaded HPLC insert and placed into an HPLC vial. The sample is then analyzed by LC/MS/MS. This procedure is then repeated for any additional time points.

### Example 7: Aryl Formamidase Promoted Cleavage of a Linked Bioactive.

Hydrolysis experiment using aryl formamidase. Briefly, the reaction is prepared in Eppendorf tubes, adding the calculated amount of water for the assay first. Then, using a 10x buffer comprised of 500 mM Hepes and 10 mM EDTA in water, a volume of this 10X buffer is added to the Eppendorf tube allowing for a 10x dilution of the 10x buffer and final concentrations of HEPES and EDTA of 50 mM and 1 mM respectively. Aryl formamidase enzyme (Brown et al, Canadian J. Microbiology 1986, 32, p. 465-72) is removed from -80°C storage, and is thawed on ice. A volume of enzyme is transferred to the Eppendorf yielding a final concentration in the assay of 10 nM. The tube is then gently inverted to mix components. All reactions are then placed in a 37 °C incubator for 20 minutes allowing the reaction mixture to reach 37 °C before the reaction is initiated. To initiate the hydrolysis, the reaction tubes are removed from the incubator and the desired fatty acid linked bioactive is added to make a final concentration of 5 µM. The tubes are gently mixed and then for time point = 0, a volume of the reaction is immediately transferred into crash buffer. The volume transferred and the volume of crash used will depend on the mass spec sensitivity of the specific analyte being tested. After crashing, the sample is centrifuged at 14,800g for 5 minutes. A portion of the supernatant (100 µL) is transferred to a spring loaded HPLC insert and placed into an HPLC vial. The sample is then analyzed by LC/MS/MS. This procedure is then repeated for any additional time points.

### METHODS OF MAKING

Compounds **I-1, I-15,** and **II-1** were prepared according to the procedures outlined in WO 2010006085 and US 20100184730. Compounds **III-1** and **III-2** were prepared according to the procedures outlined in WO 2011028689. Compounds **IV-1, IV-2,** and **IV-3** were prepared according to the procedures outlined in WO 2011085211. Compounds **VI-1, VI-2, VI-5, VI-6** and **VI-7** were prepared according to the procedures outlined in WO 2011106688. Compounds **VIIa-1** and **VIIa-2** were prepared according to the procedures outlined in WO 2011028689.

Preparation of (4Z,7Z,10Z,13Z,16Z,19Z)-N-((2S)-1-((2-(2-(4-isobutylphenyl)propanamido)ethyl)amino)-1-oxopropan-2-yl)docosa-4,7,10,13,16,19-hexaenamide (VIIa-6)

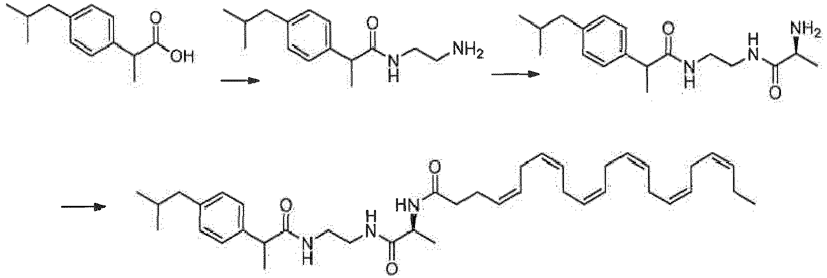

The HCl salt of N-(2-aminoethyl)-2-(4-isobutylphenyl)propanamide was prepared according to the procedures outlined in WO 2011109681. This material (1.62 g, 5.71 mmol) was taken up in 30 mL of CH₂Cl₂ along with L-Boc-Alanine (1.0 g, 5.71 mmol), EDC (1.20 g, 6.28 mmol), HOBT (848 mg, 6.28 mmol) and Et₃N (2.4 mL). The resulting reaction mixture was stirred at room temperature for 18 h. It was then washed with saturated NH₄Cl, brine, dried (Na₂SO₄) and concentrated under reduced pressure. The resulting residue was purified by silica gel chromatography (95% CH₂Cl₂, 5% MeOH) to afford 520 mg of tert-butyl ((2S)-1-((2-(2-(4-isobutylphenyl)propanamido)ethyl)amino)-1-oxopropan-2-yl)carbamate (75% yield). MS (ES) calcd for C₂₃H₃₇N₃O₄: 419.28; found 420 (M + H).

tert-butyl ((2S)-1-((2-(2-(4-isobutylphenyl)propanamido)ethyl)amino)-1-oxopropan-2-yl)carbamate (250 mg , 0.597 mmol) was taken up in 5 mL of a 4 N HCl in dioxane and allowed to stand at room temperature for 1 h. The reaction mixture was then concentrated under reduced pressure to afford the HCl salt of (2S)-2-amino-N-(2-(2-(4-isobutylphenyl)propanamido)ethyl)propanamide. This material was then taken up in 10 mL of CH₂Cl₂ along with DHA (195 mg, 0.597 mmol), EDC (126 mg, 0.657 mmol), HOBT (90 mg, 0.657 mmol) and DIEA (312 µL). The resulting reaction mixture was stirred at room temperature for 2 h. It was then washed with saturated aqueous NH₄Cl, brine, dried (Na₂SO₄) and concentrated under reduced pressure. The resulting residue was purified by silica gel chromatography (95% CH₂Cl₂, 5% MeOH) to afford 120 mg of (4Z,7Z,10Z,13Z,16Z,19Z)-N-((2S)-1-((2-(2-(4-isobutylphenyl)propanamido)ethyl)amino)-1-oxopropan-2-yl)docosa-4,7,10,13,16,19-hexaenamide (32% yield). MS (ES) calcd for C₄₀H₅₉N₃O₃: 629.46; found: 630 (M + H).

Preparation of N-(2-((S)-2-((4Z,7Z,10Z,13Z,16Z,19Z)-docosa-4,7,10,13,16,19-hexaenamido)propanamido)ethyl)-2-hydroxybenzamide (1-18)

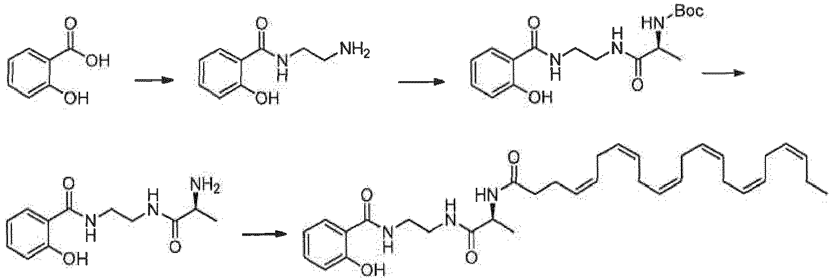

The HCl salt of N-(2-aminoethyl)-2-hydroxybenzamide was prepared according to the procedures outlined in WO 2010006085 and US 20100184730. This compound was subjected to the same reaction conditions described above using L-Boc-Alanine and DHA to obtain N-(2-((S)-2-((4Z,7Z,10Z,13Z,16Z,19Z)-docosa-4,7,10,13,16,19-hexaenamido)propanamido)ethyl)-2-hydroxybenzamide. MS (ES) calcd for C₃₄H₄₇N₃O₄: 561.36; found: 562 (M + H).

Preparation of N-(2-((S)-2-((4Z,7Z,10Z,13Z,16Z,19Z)-docosa-4,7,10,13,16,19-hexaenamido)propanamido)ethyl)nicotinamide (III-12)

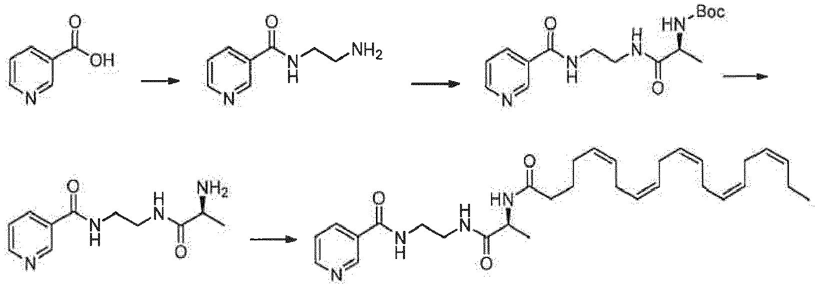

The HCl salt of N-(2-aminoethyl)nicotinamide was prepared according to the procedures outlined in WO 2011028689. This compound was subjected to the same reaction conditions described above using L-Boc-Alanine and EPA to obtain N-(2-((S)-2-((4Z,7Z,10Z,13Z,16Z,19Z)-docosa-4,7,10,13,16,19-hexaenamido)propanamido)ethyl)nicotinamide. MS (ES) calcd for C₃₁H₄₄N₄O₃: 520.34; found: 521 (M + H).

Preparation of (E)-methyl 4-((2-((S)-2-((4Z,7Z,10Z,13Z,16Z,19Z)-docosa-4,7,10,13,16,19-hexaenamido)propanamido)ethyl)amino)-4-oxobut-2-enoate (IV-5)

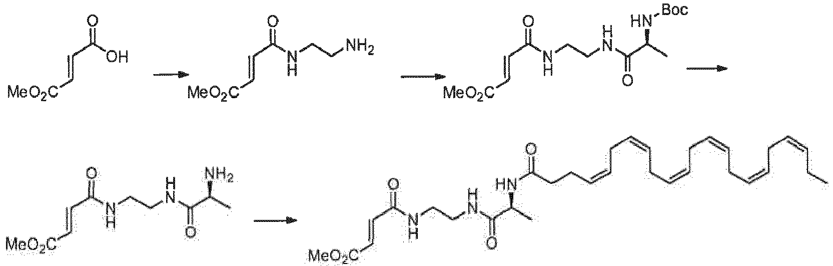

The HCl salt of (E)-methyl 4-((2-aminoethyl)amino)-4-oxobut-2-enoate was prepared according to the procedures outlined in WO 2011106688. This compound was subjected to the same reaction conditions described above using L-Boc-Alanine and DHA to obtain (E)-methyl 4-((2-((S)-2-((4Z,7Z,10Z,13Z,16Z,19Z)-docosa-4,7,10,13,16,19-hexaenamido)propanamido)ethyl)amino)-4-oxobut-2-enoate. MS (ES) calcd for C₃₂H₄₇N₃O₅: 553.35; found: 554 (M + H).

One skilled in the art will readily appreciate that the present invention is well adapted to carry out the objects and obtain the ends and advantages mentioned, as well as those inherent, herein. The present examples along with the methods, procedures, treatments, molecules, and specific compounds described herein and other uses will occur to those skilled in the art that is encompassed within the spirit of the invention as defined by the scope of the claims.

### EQUIVALENTS

Those skilled in the art will recognize, or be able to ascertain, using no more than routine experimentation, numerous equivalents to the specific embodiments described specifically herein. Such equivalents are intended to be encompassed in the scope of the following claims.

### INCORPORATION BY REFERENCE

The entire disclosure of each of the patent documents and scientific publications disclosed hereinabove is expressly incorporated herein by reference for all purposes.

## Claims

1. A method of designing a linked bioactive that is activated by an intracellular enzyme upregulated in cells in a disease state, wherein the enzyme is a fatty acid amide hydrolase (FAAH), the method comprising:
(a) selecting
(i) a first bioactive, (ii) a second bioactive that can be the same or different from the first bioactive, and (iii) a linkage comprising a linker group for linking the first and second bioactives, wherein the linker group is a functional substrate for the FAAH enzyme; and
(b) linking the first and second bioactives with the linkage to produce the linked bioactive, wherein the linked bioactive, upon cleavage by the FAAH enzyme, displays a synergistic effect between the two bioactives that is not observed when the bioactives are administered alone or as non-linked bioactives.

2. The method of claim 1, wherein the FAAH enzyme is FAAH-1.

3. The method of claim 1, wherein the FAAH enzyme is FAAH-2.
